# EUROPEAN PATENT SPECIFICATION

(11) **EP 1 443 961 B1**
(45) Date of publication and mention of the grant of the patent: **06.05.2009**
(21) Application number: 02773840.0
(22) Date of filing: 22.10.2002
(51) Int. Cl.: A61K 39/395, C12P 21/08, C07K 16/28, C07K 16/32, C07K 16/42

(54) **GLYCOPROTEIN COMPOSITIONS**
GLYCOPROTEIN-ZUSAMMENSETZUNGEN
COMPOSITIONS DE GLYCOPROTEINE

(30) Priority: 25.10.2001 US 337642 P; 09.01.2002 US 347694 P
(43) Date of publication of application: 11.08.2004
(73) Proprietor: Genentech, Inc., South San Francisco CA 94080-4990 (US)
(72) Inventor: PRESTA, Leonard, G., San Francisco, CA 94109 (US)
(74) Representative: Walton, Seán Malcolm
(86) International application number: PCT/US2002/033739
(87) International publication number: WO 2003/035835

(56) References cited:
- MIMURA Y ET AL: "The influence of glycosylation on the thermal stability and effector function expression of human IgG1-Fc: properties of a series of truncated glycoforms." MOLECULAR IMMUNOLOGY. 2000 AUG-SEP, vol. 37, no. 12-13, August 2000 (2000-08), pages 697-706, XP002346573 ISSN: 0161-5890
- OHYAMA C ET AL: "Molecular cloning and expression of GDP-D-mannose-4,6-dehydratase, a key enzyme for fucose metabolism defective in Lec13 cells" JOURNAL OF BIOLOGICAL CHEMISTRY, AMERICAN SOCIETY OF BIOLOGICAL CHEMISTS, BALTIMORE, MD, US, vol. 273, no. 23, 5 June 1998 (1998-06-05), pages 14582-14587, XP002328593 ISSN: 0021-9258
- SHIELDS R L ET AL: "High resolution mapping of the binding site on human IgG1 for FcgammaRI, FcgammaRII, FcgammaRIII, and FcRn and design of IgG1 variants with improved binding to the FcgammaR" JOURNAL OF BIOLOGICAL CHEMISTRY, AMERICAN SOCIETY OF BIOLOGICAL CHEMISTS, BALTIMORE, MD, US, vol. 276, no. 9, 2 March 2001 (2001-03-02), pages 6591-6604, XP002271092 ISSN: 0021-9258
- KOJIMA N. ET AL.: 'Characterization of mouse ST8Sia II (STX) as a neural cell adhesion molecule-specific polysialic acid synthase' J. BIOL. CHEM. vol. 271, no. 32, 09 August 1996, pages 19457 - 19463, XP002195629
- DAVIES J. ET AL.: 'Expression of GnTIII in a recombinant anti-CD20 CHO production cell line: expression of antibodies with altered gycoforms leads to an increase in ADCC through higher affinity of FcgammaRIII' BIOTECHNOL. BIOENG. vol. 74, no. 4, August 2001, pages 288 - 294, XP002964541
- SHIELDS R.L. ET AL.: 'Lack of fucose on human IgG1 N-linked oligosaccharide improves binding to human FcgammaRIII and antibody-dependent cellular toxicity' J. BIOL. CHEM. vol. 277, no. 30, 26 July 2002, pages 26733 - 26740, XP002964542

## Description

### BACKGROUND OF THE INVENTION

### Field of the Invention

The present invention concerns compositions comprising glycoproteins with modified glycosylation patterns. More particularly the invention concerns compositions comprising a glycoprotein having a Fc region, wherein about 80-100 % of the glycoprotein in the composition comprises a mature core carbohydrate structure which lacks fucose, attached to the Fc region of the glycoprotein.

### Description of Related Art

### Antibodies

Antibodies are proteins that exhibit binding specificity to a specific antigen. Native antibodies are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains.

The term "variable" refers to the fact that certain portions of the variable domains differ extensively in sequence among antibodies and are responsible for the binding specificity of each particular antibody for its particular antigen. However, the variability is not evenly distributed through the variable domains of antibodies. It is concentrated in three segments called complementarity determining regions (CDRs) both in the light chain and the heavy chain variable domains. The more highly conserved portions of the variable domains are called the framework regions (FRs). The variable domains of native heavy and light chains each comprise four FRs, largely adopting a β-sheet configuration, connected by three CDRs, which form loops connecting, and in some cases forming part of, the β-sheet structure. The CDRs in each chain are held together in close proximity by the FRs and, with the CDRs from the other chain, contribute to the formation of the antigen binding site of antibodies (see Kabat et al., Sequences of Proteins of Immunological Interest, 5th Ed. Public Health Service, National Institutes of Health, Bethesda, MD. (1991)).

The constant domains are not involved directly in binding an antibody to an antigen, but exhibit various effector functions. Depending on the amino acid sequence of the constant region of their heavy chains, antibodies or immunoglobulins can be assigned to different classes. There are five major classes of immunoglobulins: IgA, IgD, IgE, IgG and IgM, and several of these may be further divided into subclasses (isotypes), *e.g*. IgG1, IgG2, IgG3, and IgG4; IgA1 and IgA2. The heavy chain constant regions that correspond to the different classes of immunoglobulins are called α, δ, ε, γ, and µ, respectively. Of the various human immunoglobulin classes, only human IgG1, IgG2, IgG3 and IgM are known to activate complement; and human IgG1 and IgG3 mediate ADCC more effectively than IgG2 and IgG4.

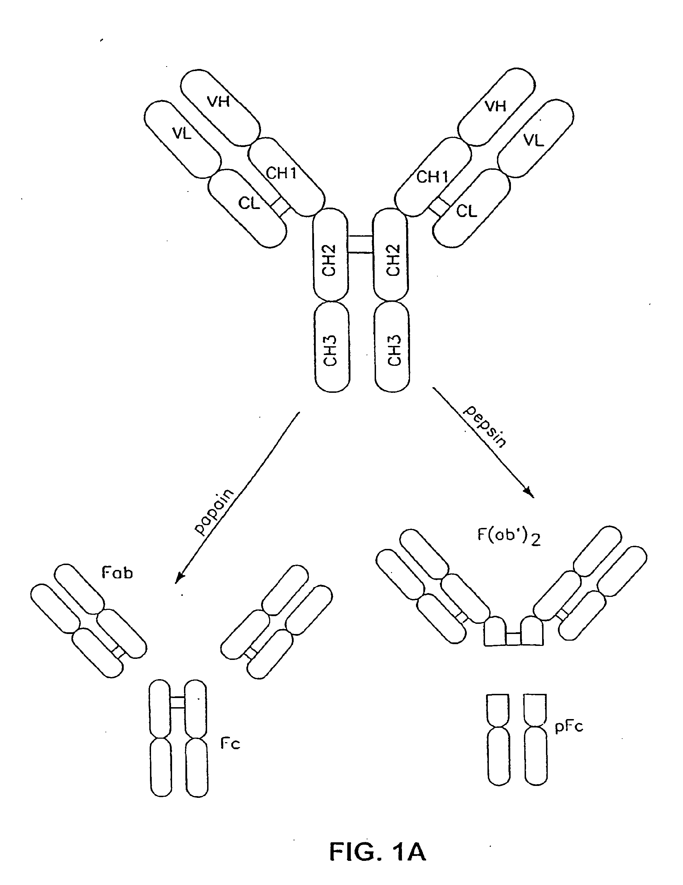

A schematic representation of the native IgG1 structure is shown in Fig. 1A, where the various portions of the native antibody molecule are indicated. Papain digestion of antibodies produces two identical antigen binding fragments, called Fab fragments, each with a single antigen binding site, and a residual "Fc" fragment, whose name reflects its ability to crystallize readily. The crystal structure of the human IgG Fc region has been determined (Deisenhofer, Biochemistry 20:2361-2370 (1981)). In human IgG molecules, the Fc region is generated by papain cleavage N-terminal to Cys 226. The Fc region is central to the effector functions of antibodies.

Other antibody-like molecules have been described. For instance, "immunoadhesins" which combine the binding domain of a heterologous protein such as a receptor, ligand or enzyme, with the effector functions of an Fc region have been reported in the literature. An exemplary such molecule is the tumor necrosis factor receptor-IgG (TNFR-IgG) immunoadhesin described in US Patent No. 5,610,297. Bispecific immunoadhesins and antibody-immunoahesin chimeras have also been described. Stabila, P., Nature Biotech, 16:1357 (1998) describes another Fc region-containing plasma membrane-anchored fusion protein. The fusion protein in this reference combines a type II transmembrane domain that localizes to the plasma membrane, fused to the N-terminus of an Fc region.

Antibodies and immunadhesins are being used as therapeutics in human disease (Glennie et al. Immunol. Today 21:403-410 (2000); King et al. Curr. Opin. Drug Discovery Develop 2:110-117 (1999); Vaswani et al. Ann. Allergy Asthma Immunol. 81:105-119 (1998); and Abraham et al. Sec. Intern. Autumnal Them. Meeting on Sepsis, Deauville, France (1995)). Some of these antibodies and immunoadhesins, *e*.*g*. those which bind to a receptor or ligand and thereby block ligand receptor interaction, may function without utilizing antibody effector mechanisms. Others may need to recruit the immune system to kill the target cell (Clynes et al. Nature Med. 6:443-446 (2000); Clynes et al. PNAS (USA) 95:652-656 (1998); and Anderson et al. Biochem. Soc. Trans. 25:705-708 (1997)).

### Antibody Effector Functions

The effector functions mediated by the antibody Fc region can be divided into two categories: (1) effector functions that operate after the binding of antibody to an antigen (these functions involve the participation of the complement cascade or Fc receptor (FcR)-bearing cells); and (2) effector functions that operate independently of antigen binding (these functions confer persistence in the circulation and the ability to be transferred across cellular barriers by transcytosis). Ward and Ghetie, Therapeutic Immunology 2:77-94 (1995).

While binding of an antibody to the requisite antigen has a neutralizing effect that might prevent the binding of a foreign antigen to its endogenous target (e.g. receptor or ligand), binding alone may not remove the foreign antigen. To be efficient in removing and/or destructing foreign antigens, an antibody should be endowed with both high affinity binding to its antigen, and efficient effector functions.

The interaction of antibodies and antibody-antigen complexes with cells of the immune system effects a variety of responses, including antibody-dependent cell-mediated cytotoxicity (ADCC) and complement dependent cytotoxicity (CDC) (reviewed in Daëron, Annu. Rev. Immunol. 15:203-234 (1997); Ward and Ghetie, Therapeutic Immunol. 2:77-94 (1995); as well as Ravetch and Kinet, Annu. Rev. Immunol. 9:457-492 (1991)).

Several antibody effector functions are mediated by Fc receptors (FcRs), which bind the Fc region of an antibody. FcRs are defined by their specificity for immunoglobulin isotypes; Fc receptors for IgG antibodies are referred to as FcγR, for IgE as FcεR, for IgA as FcαR and so on. Three subclasses of FcγR have been identified: FcγRI (CD64), FcγRII (CD32) and FcγRIII (CD16). Because each FcγR subclass is encoded by two or three genes, and alternative RNA spicing leads to multiple transcripts, a broad diversity in FcγR isoforms exists. The three genes encoding the FcγRI subclass (FcγRIA, FcγRIB and FcγRIC) are clustered in region 1q21.1 of the long arm of chromosome 1; the genes encoding FcγRII isoforms (FcγRIIA, FcγRIIB and FcγRIIC) and the two genes encoding FcγRIII (FcγRIIIA and FcγRIIIB) are all clustered in region 1q22. These different FcR subtypes are expressed on different cell types (reviewed in Ravetch and Kinet, Annu. Rev. Immunol. 9:457-492 (1991)). For example, in humans, FcγRIIIB is found only on neutrophils, whereas FcγRIIIA is found on macrophages, monocytes, natural killer (NK) cells, and a subpopulation of T-cells.

Structurally, the FcγR are all members of the immunoglobulin superfamily, having an IgG-binding α-chain with an extracellular portion comprised of either two (FcγRI and FcγRIII) or three (FcγRI) Ig-like domains. In addition, FcγRI and FcγRIII have accessory protein chains (γ,ζ) associated with the α-chain which function in signal transduction. The receptors are also distinguished by their affinity for IgG. FcγRI exhibits a high affinity for IgG, Kₐ =10⁸-10⁹M⁻¹ (Ravetch et al. Ann. Rev. Immunol. 19:275-290 (2001)) and can bind monomeric IgG. In contrast FcγRII and FcγRIII show a relatively weaker affinity for monomeric IgG Kₐ ≤ 10⁷M⁻¹ (Ravetch et al. Ann. Rev. Immunol. 19:275-290 (2001)), and hence only interact effectively with multimeric immune complexes. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (see review in Daëron, Annu. Rev. Immunol. 15:203-234 (1997)). NK cells carry only FcγRIIIA and binding of antibodies to FcγRIIIA leads to ADCC activity by the NK cells.

Allelic variants of several of the human FcγR have been found in the human population. These allelic variant forms have been shown to exhibit differences in binding of human and murine IgG and a number of association studies have correlated clinical outcomes with the presence of specific allelic forms (reviewed in Lehrnbecher et al. Blood 94(12):4220-4232 (1999)). Several studies have investigated two forms of FcγRIIA, R131 and H131, and their association with clinical outcomes (Hatta et al. Genes and Immunity 1:53-60 (1999); Yap et al. Lupus 8:305-310 (1999); and Lorenz et al. European J. Immunogenetics 22:397-401 (1995)). Two allelic forms of FcγRIIIA, F158 and V158, are only now being investigated (Lehrnbecher *et al., supra*; and Wu et al. J. Clin. Invest. 100(5): 1059-1070 (1997)). The FcγRIIIA(Val158) allotype interacts with human IgG better than the FcγRIIIA(Phe158) allotype (Shields et al. J. Biol. Chem. 276: 6591-6604 (2001); Koene et al. Blood 90:1109-1114 (1997); and Wu et al. J.Clin. Invest. 100: 1059-1070 (1997)).

Another type of Fc receptor is the neonatal Fc receptor (FoRn). FcRn is structurally similar to major histocompatibility complex (MHC) and consists of an α-chain noncovalently bound to β2-mioroglobulin. FcRn has been proposed to regulate homeostasis of IgG in blood as well as possibly control transcytosis across tissues (Ghetie et al. Annu. Rev. Immunol. 18:739-766 (2000)).

The binding site on human and murine antibodies for FcγR have been previously mapped to the so-called "lower hinge region" consisting of residues 233-239 (EU index numbering as in Kabat et al., Sequences of Proteins of Immunological Interest, Sth Ed. Public Health Service, National Institutes of Health, Bethesda, MD. (1991)). Woof et al. Molec. Immunol. 23:319-330 (1986); Duncan et al. Nature 332:563 (1988); Canfield and Morrison, J. Exp. Med. 173:1483-1491 (1991); Chappel et al., Proc. Natl. Acad. Sci USA 88:9036-9040 (1991). Of residues 233-239, P238 and S239 have been cited as possibly being involved in binding.

Other previously cited areas possibly involved in binding to FcγR are: G316-K338 (human IgG) for human FcγRI (by sequence comparison only; no substitution mutants were evaluated) (Woof et al. Molec. Immunol. 23:319-330 (1986)); K274-R301 (human IgG1) for human FcγRIII (based on peptides) (Sarmay et al. Molec. Immunol. 21:43-51 (1984)); Y407-R416 (human IgG) for human FcγRIII (based on peptides) (Gergely et al. Biochem. Soc. Trans. 12:739-743 (1984)); as well as N297 and E318 (murine IgG2b) for murine FcγRII (Lund et al., Molec. Immunol 29:53-59 (1992)). See also Armour et al. Eur. J. Immunol. 29: 2613-2624 (1999).

WO00/42072 (Presta) describes polypeptide variants with improved or diminished binding to FcRs. See, also, Shields et al. J. Biol. Chem. 9(2): 6591-6604 (2001).

C1q and two serine proteases, C1r and C1s, form the complex C1, the first component of the complement dependent cytotoxicity (CDC) pathway. C1q is a hexavalent molecule with a molecular weight of approximately 460,000 and a structure likened to a bouquet of tulips in which six collagenous "stalks" are connected to six globular head regions. Burton and Woof, Advances in Immunol. 51:1-84 (1992). To activate the complement cascade, it is necessary for C1q to bind to at least two molecules of IgG1, IgG2, or IgG3 (the consensus is that IgG4 does not activate complement), but only one molecule of IgM, attached to the antigenic target. Ward and Ghetie, Therapeutic Immunology 2:77-94 (1995) at page 80.

Based upon the results of chemical modifications and crystallographic studies, Burton et al. Nature, 288:338-344 (1980) proposed that the binding site for the complement subcomponent C1q on IgG involves the last two (C-terminal) β-strands of the CH2 domain. Burton later suggested (Molec. Immunol., 22(3):161-206 (1985)) that the region comprising amino acid residues 318 to 337 might be involved in complement fixation.

Duncan and Winter Nature 332:738-40 (1988), using site directed mutagenesis, reported that Glu318, Lys320 and Lys322 form the binding site to C1q. The data of Duncan and Winter were generated by testing the binding of a mouse IgG2b isotype to guinea pig C1q. The role of Glu318, Lys320 and Lys322 residues in the binding of C1q was confirmed by the ability of a short synthetic peptide containing these residues to inhibit complement mediated lysis. Similar results are disclosed in U.S. Patent No. 5,648,260 issued on July 15, 1997, and U.S. Patent No. 5,624,821 issued on April 29, 1997.

The residue Pro331 has been implicated in C1q binding by analysis of the ability of human IgG subclasses to carry out complement mediated cell lysis. Mutation of Ser331 to Pro331 in IgG4 conferred the ability to activate complement (Tao et al., J. Exp. Med., 178:661-667 (1993); Brekke et al., Eur. J. Immunol., 24:2542-47 (1994)).

From the comparison of the data of the Winter group, and the Tao *et al.* and Brekke *et al.* papers, Ward and Ghetie concluded in their review article that there are at least two different regions involved in the binding of C1q: one on the β-strand of the CH2 domain bearing the Glu318, Lys320 and Lys322 residues, and the other on a turn located in close proximity to the same β-strand, and containing a key amino acid residue at position 331.

Other reports suggested that human IgG1 residues Lys235, and Gly237, located in the lower hinge region, play a critical role in complement fixation and activation. Xu et al.,J. Immunol. 150:152A (Abstract) (1993). WO94/29351 published December 22,1994 reports that amino acid residues necessary for C1q and FcR binding of human IgG1 are located in the N-terminal region of the CH2 domain, *i*.*e*. residues 231 to 238.

It has further been proposed that the ability of IgG to bind C1q and activate the complement cascade also depends on the presence, absence or modification of the carbohydrate moiety positioned between the two CH2 domains (which is normally anchored at Asn297). Ward and Ghetie, Therapeutic Immunology 2:77-94 (1995) at page 81.

Polypeptide variants with altered Fc region amino acid sequences and increased or decreased C1q binding capability are described in US patent No. 6,194,551B1 and WO99/51642. See, also, Idusogie et al. J. Immunol. 164: 4178-4184 (2000).

Other methods that improve immune system recruitment include bispecific antibodies, in which one arm of the antibody binds and IgG receptor (Segal et al. J. Immunol. Meth. 248:1-6 (2001); and cytokine-IgG fusion proteins (Penichet et al. J. Immunol. Meth. 248:91-101 (2001)).

### Antibody Glycosylation

Many polypeptides, including antibodies, are subjected to a variety of post-translational modifications involving carbohydrate moieties, such as glycosylation with oligosaccharides. Such glycosylated polypeptides are referred to as "glycoproteins".

There are several factors that can influence glycosylation. The species, tissue and cell type have all been shown to be important in the way that glycosylation occurs. In addition, the extracellular environment, through altered culture conditions such as serum concentration, may have a direct effect on glycosylation. (Lifely et al. Glycobiology 5(8): 813-822 (1995)). Various methods have been proposed to alter the glycosylation pattern achieved in a particular host organism including introducing or overexpressing certain enzymes involved in oligosaccharide production (U.S. Patent No. 5,047,335; U.S..Patent No. 5,510,261). These schemes are not limited to intracellular methods (U.S. Patent No. 5,278,299).

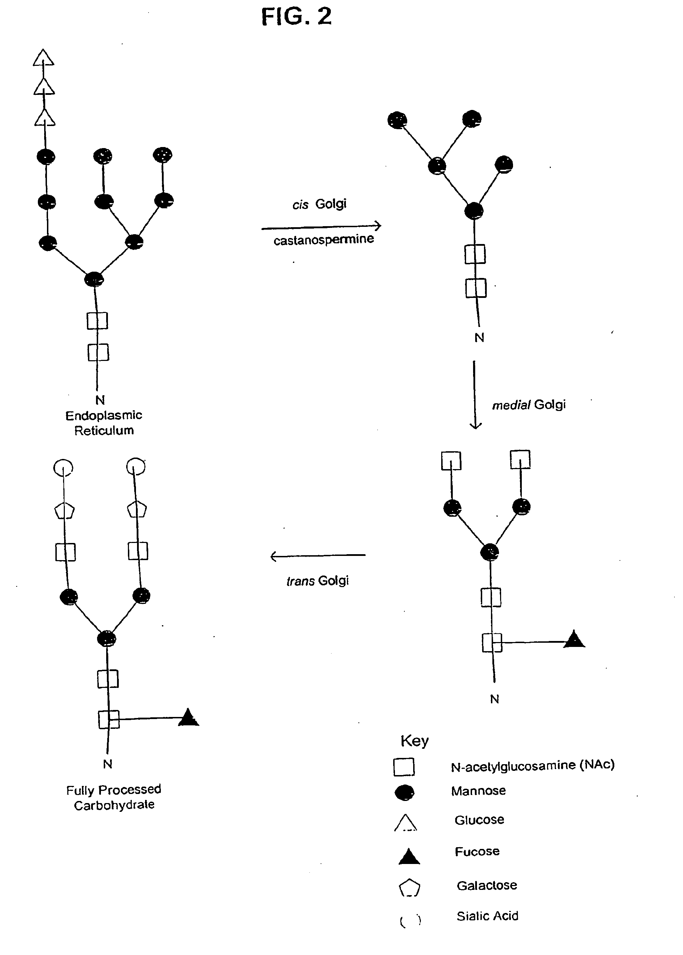

All antibodies contain carbohydrate at conserved positions in the constant regions of the heavy chain. Each antibody isotype has a distinct variety of N-linked carbohydrate structures. Aside from the carbohydrate attached to the heavy chain, up to 30% of human IgGs have a glycosylated Fab region. IgG has a single N-linked biantennary carbohydrate at Asn297 of the CH2 domain. The fully processed carbohydrate structure attached to Asn297 is depicted in Figure 2 herein. For IgG from either serum or produced *ex vivo* in hybridomas or engineered cells, the IgG are heterogeneous with respect to the Asn297 linked carbohydrate. Jefferis et al. Immunol. Rev. 163:59-76 (1998); and Wright et al. Trends Biotech 15:26-32 (1997). For human IgG, the core oligosaccharide normally consists of GlcNAc₂Man₃GlcNAc, with differing numbers of outer residues. Figure 2 herein depicts the processing pathway of oligosaccharide to mature carbohydrate. The early synthesized species Glu₃Man₉GlcNac₂ is transferred to Asn297 in the CH2 domain of the antibody as it emerges from the ribosome. After the three terminal glucoses are trimed as the glycoprotein passes through the endoplasmic reticulum, the glycoprotein moves to the *cis* Golgi where mannose residues are enzymatically removed by α-mannosidases. Processing can stop at this juncture, yielding hyper-mannosylated glycoproteins. Otherwise, processing can continue to yield Man₅GlcNac₂. Action of N-acetylglycosaminyltransferase I in the *medial* Golgi is the committed step in complex oligosaccharide synthesis. In the *medial* and *trans* Golgi, the oligosaccharide undergoes further processing steps in which mannose residues are trimmed and the sugar residues are sequentially added. The newly synthesized glycoprotein then exits the Golgi and is transported to the cell membrane or is secreted.

Variation among individual IgG occurs via attachment of galactose and/or galactose-sialic acid at the two terminal GlcNac or via attachment of a third GlcNAc arm (bisecting GlcNAc). The carbohydrate linked to Asn297 of IgG has been studied. Absence of the carbohydrate affects binding to C1q and FcγR (and consequently affects complement activation and ADCC). Leatherbarrow et al. Molec. Immunol. 22:407-415 (1985); Duncan et al. Nature 332:738-740 (1988); Walker et al. Biochem. J. 259:347-353 (1989); Dorai et al. Hybridoma 10:211-217 (1990); and Horan Hand et al. Cancer Immunol. Immunother. 35:165-174 (1992). While binding to FcRn appears unaffected by lack of carbohydrate (Hobbs et al. Molec. Immunol. 29:949-956 (1992); and Kim et al. Eur. J. Immunol. 24:542-548 (1994)), effect on clearance is uncertain (Dorai et al. Hybridoma 10:211-217 (1990); Horan Hand et al. Cancer Immunol. Immunother. 35:165-174 (1992); Hobbs et al. Molec. Immunol. 29:949-956 (1992); Kim et al. Eur. J. Immunol. 24:542-548 (1994); Wawrzynczak et al. Biochem. Soc. Trans. 17:1061-1062 (1989); and Tao et al. J. Immuno. 143:2595-2601 (1989)). When the carbohydrate is present, the nature of the sugar residues can also influence the IgG effector functions. The presence or absence of terminal galactose residues has been reported to affect function (Wright et al. J. Immunol. 160:3393-3402 (1998)) and appears correlated with rheumatoid arthritis (Parekh et al. Nature 316:452-457 (1985)). Human IgG isolated from sera of patients with multiple myeloma shows extremes in presence/absence of fucose, galactose, and bisecting N-acetylglycosamine (Parekh et al. Nature 316:452-457 (1985)). Raju *et al.* describe variation in glycosylation of IgG from different species (Raju et al. Glycobiology 10(5):477-486 (2000)).

Boyd *et al.* found that removal of terminal sialic acid from CHO-derived CAMPATH-1H through glycopeptidase F digestion did not effect any of the tested IgG activities, whereas removal of the majority of the galactose residues from desialylated CAMPATH-1H was found to reduce (but not abolish) complement lysis activity. Other activities were not affected by degalactosylation. Boyd et al. Molec. Immunol. 32(17/18):1311-1318 (1995). Kumpel et al., Hum. Antibod. Hybridomas, 5(3-4):143-151 (1994) report that galactosylation of human IgG monoclonal antibody affects Fc receptor-mediated functional activity.

Rothman *et al.* tested the ADCC function of monoclonal IgG purified from hybridomas treated with glycosidase inhibitors that acted at different stages in the carbohydrate processing pathway. Rothman et al. Molecular Immunol. 26(12):1113-1123 (1989). Treatment with castanospermine, which inhibits removal of glucose residues from the nascent oligosaccharide (Kaushal et al. Meth. Enzymol. 230:316-329 (1994)), showed enhanced ADCC by NK cells, which express only FcγRIII, but not by other types of effectors cells such as monocytes. Lectin-binding analysis suggested that the castanospermine-treated IgG lacked fucose; however the IgG resulting from castanospermine treatment may have had other carbohydrate structure, such as hyper-mannosylation as well as terminal glucose residues (Kaushal et al. Meth. Enzymol. 230:316-329 (1994); Hashim et al. Immunology 63:383-388 (1988); Hashim et al. Molec. Immunol. 24:1087-1096 (1987)), not routinely found on IgG secreted from non-treated cells or from human serum.

WO 97/30087 describes preparation of glycosylated antibodies where an N-glycosylation site of the Fc domain of the antibody is substituted with a biantennary oligosaccharide.

Umana *et al.* introduced a β(1,4)-*N*-acetylglucosaminyltransferase III (GcTIII) gene that catalyzes the addition of a bisected GlcNAc to the carbohydrate core attached to Asn297 of the antibody into chinese hamster ovary (CHO) cells. The glycoforms produced by the engineered CHO cells were considered to have optimized ADCC. See WO 99/54342 and Umana et al., Nature Biotechnology, 17: 176-180 (1999).

WO98/58964 (Raju et al.) describes antibody compositions wherein substantially all of the N-linked oligosaccharide is a G2 oligosaccharide. G2 refers to a biantennary structure with two terminal Gals and no NeuAcs. WO99/22764 (Raju et al.) refers to antibody compositions which are substantially free of a glycoprotein having an N-linked G1, G0, or G-1 oligosaccharide in its CH2 domain. G1 refers to a biantennary structure having one Gal and no NeuAcs, G0 refers to a biantennary structure wherein no terminal NeuAcs or Gals are present and G-1 refers to the core unit minus one GlcNAc.

WO00/61739 reports that 47% of anti-hIL-5R antibodies expressed by YB2/0 (rat myeloma) cells have α 1-6 fucose-unked sugar chains, compared to 73% of those antibodies expressed by NSO (mouse myeloma) cells. The fucose relative ratio of α-hIL-5R antibodies expressed by various host cells was YB2/0 < CHO/d < NSO.

Routier *et al.* studied the glycosylation pattern of a humanized IgG1 antibody (D1.3) expressed in CHO-DUKX cells. The structures of the N-glycans of the CHO-expressed were biantennary N-glycans with core fucose but lacking bisecting GlcNAc and sialic acid. The structures were heterogeneous with respect to the terminal galaotosylation and were therefore called G₂, G₁ and G₀. Routier et al. Glycoconjugate J. 14:201-207 (1997).

It has been previously reported that O-linked fucose has been found on a number of polypeptides and that the attached fucose is important for proper activity of the polypeptide. See WO98/33924, which describes methods of glycosylating with an O-fucose moiety. Stankova et al. J. Immunol. 135(6):3719-3728 (1985) found that fucose significantly enhances the cytolytic capacity of mixed leukocyte culture (MCL)-induced or preincubated effector cells. Cameron et al. Immunol. Lett. 11:39-44 (1985) found that α-L-fucose appears to play an important role in macrophage-tumor cell interactions.

There is a continuing need in the art to produce glycoproteins, such as antibodies, having improved biological activity.

Mimura et al. (Molecular Immunology 37:697-706 Aug 2000) described enzymatic treatments of a glycosylated Fc fragment of IgG1-Wid isolated from myeloma serum and an anti-MHC class II antibody IgG1 L243 heavy chain, to produce truncated glycoforms. Properties of the truncated glycoforms were studied to examine the influence of glycosylation on the thermal stability and effector function of human IgG1-Fc. The authors reported that truncated oligosaccharides conferred a degree of functional activity and thermodynamic stability to the IgG1-Fc compared with deglycosylated IgG1-Fc.

Kojima et al. (J. Biol. Chem. 271:19457-19463 Aug 1996) reported the characterisation of mouse enzyme STX as a polysialic acid synthase that polysialylates NCAM. The study employed an NCAM fusion with Fc of human IgG1 as an enzyme substrate. When treated with fucosidase, polysialylation of the substrate by STX was reduced, indicating that fucose is required for polysialylation by STX.

Ohyama et al. (J. Biol. Chem. 273:14582-14587 1998) reported cloning of the gene GDP-d-mannose-4,6-dehydratase (GMD), which generates GDP-mannose-4-keto-6-D-deoxymannose from GDP mannose, which is subsequently converted to GDP-L-fucose. The enzyme GMD is defective in Lec13 CHO cells, which produce fucosylation-deficient glycoproteins. Putative roles for fucosylated glycoproteins in leukocyte adhesion and trafficking were discussed.

### SUMMARY OF THE INVENTION

The invention relates to glycoprotein compositions, as set out in the appended claims. Described herein are glycoprotein compositions of a glycoprotein having a Fc region, wherein about 80-100% (and preferably about 90-99%) of the glycoprotein in the composition comprises a mature core carbohydrate structure which lacks fucose, attached to the Fc region of the glycoprotein. Such compositions were demonstrated herein to exhibit a surprising 100-fold improvement in binding to Fc(RIIIA(F158), which is not as effective as Fc(RIIIA(V158) in interacting with human IgG. Thus, the compositions herein are anticipated to be superior to previously described compositions, especially for therapy of human patients who express Fc(RIIIA(F158). Fc(RIIIA (F158) is more common than Fc(RIIIA (V158) in normal, healthy African Americans and Caucasians. See Lehrnbecher et al. Blood 94:4220 (1999).

The present application further demonstrates the synergistic increase in FcγRIII binding and/or ADCC function that results from combining the glycosylation variations herein with amino acid sequence modification(s) in the Fc region of the glycoprotein. In order to generate the Fc region amino acid sequence variant with improved ADCC activity, one will generally engineer an Fc region variant with improved binding affinity for FcγRIII, which is thought to be an important FcR for mediating ADCC. For example, one may introduce an amino acid modification (*e*.*g*. a substitution) into the parent Fe region at any one or more of amino acid positions 256, 290, 298, 312, 326, 330, 333, 334, 360, 378 or 430 to generate such a variant. The variant with improved binding affinity for FcγRIII may further have reduced binding affinity for FcγRII, especially reduced affinity for the inhibiting FcγRIIB receptor. In the preferred embodiments, the Fc region has amino acid substitutions at positions 298, 333 and 334, *e.g*. S298A/E333A/K334A. The Fc region with an altered amino acid sequence further comprises a glycosylation variation which yet further enhances ADCC. For instance, the variant Fc region may have attached thereto a mature core carbohydrate structure which lacks fucose.

Thus, the invention provides a composition comprising a glycoprotein having a Fc region, wherein about 51-100% of the glycoprotein in the composition comprises a mature core carbohydrate structure which lacks fucose, attached to the Fc region of the glycoprotein, and wherein the Fc region comprises an amino acid sequence that differs from a native sequence Fc region. More preferably, about 80-100% of the glycoprotein in the composition comprises a mature core carbohydrate structure which lacks fucose and most preferably about 90-99% of the glycoprotein in the composition lacks fucose attached to the mature core carbohydrate structure.

The glycoprotein may, for example, comprise an antibody or an immunoadhesin. The glycoprotein generally comprises an Fc region, preferably a human Fc region; *e*.*g*., a human IgG1, IgG2, IgG3 or IgG4 Fc region. The glycoprotein displays increased binding to an FcγRIII (such as FcγRIIIA (F158) and/or FcγRIIIA (V158)) and improved ADCC relative to the glycoprotein with fucose attached to its mature core carbohydrate structure.

The invention also provides a pharmaceutical preparation comprising the glycoprotein and, optionally, a pharmaceutically acceptable carrier or diluent. This preparation for potential therapeutic use is sterile and may be lyophilized.

Diagnostic and therapeutic uses for the glycoprotein disclosed herein are contemplated. One diagnostic application is a method for determining the presence of an antigen of interest comprising exposing a sample suspected of containing the antigen to the glycoprotein and determining binding of the glycoprotein to the sample.

One therapeutic application is a method of treating a mammal suffering from or predisposed to a disease or disorder that would benefit from such treatment, comprising administering to the mammal a therapeutically effective amount of the composition herein, especially where the composition is a pharmaceutical preparation.

The invention further provides a host cell comprising nucleic acid encoding a glycoprotein which comprises an Fc region, in accordance with the appended claims. Moreover, the invention provides a method for producing a glycoprotein comprising culturing this host cell so that the nucleic acid is expressed and, optionally, recovering the glycoprotein from the host cell culture (*e*.*g*. from the host cell culture medium).

### BRIEF DESCRIPTION OF THE DRAWINGS

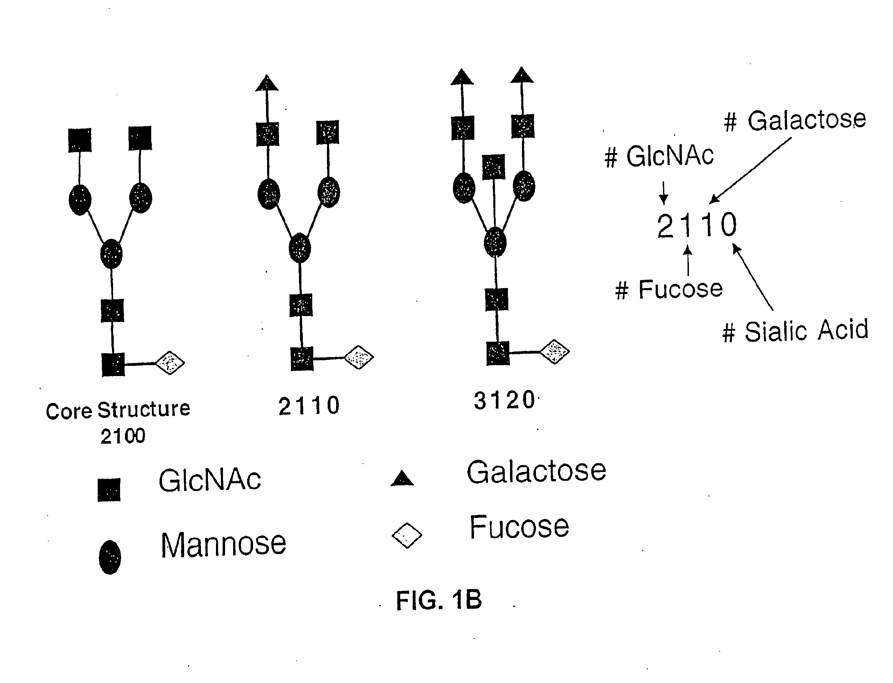

Figure 1A is a schematic representation of a native IgG and enzymatic digestion thereof to generate various antibody fragments. Disulfide bonds are represented by double lines between CH1 and CL domains and the two CH2 domains. V is variable domain; C is constant domain; L stands for light chain and H stands for heavy chain. Figure 1B depicts schematically fully processed or "mature" core carbohydrate structure (2100) attached to Asn297 of serum IgGs, the mature core carbohydrate structure with a single galactose residue (2110) as well as the core carbohydrate structure with two galactose residues and a bisecting GlcNAc (3120). The number of GlcNAc, fucose, galactose and sialic acid residues, respectively, are reflected with the four digit numbering systems shown in this figure.
Figure 2 illustrates the addition of oligosaccharide to Asn297 in the CH2 domain of the IgG, followed by processing thereof in the *cis, medial* and *trans* Golgi to generate the complex biantennary fully processed carbohydrate structure. Castanospermine inhibits removal of glucose and mannose residues from the nascent oligosaccharide.

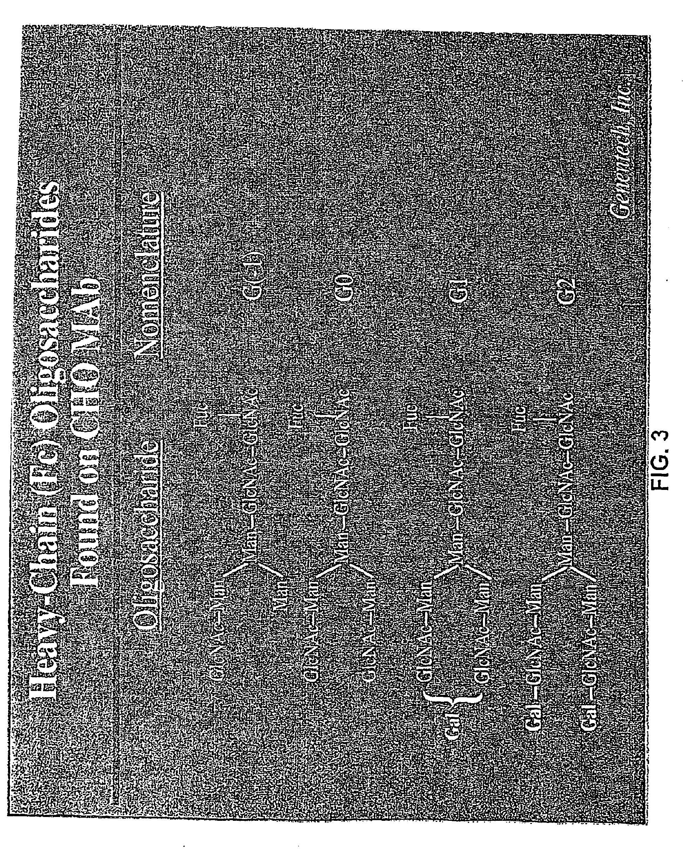

Figure 3 shows heavy chain (Fc) oligosaccharides found on antibodies expressed in CHO cells with normal fucose metabolism.
Throughout the further figure legends and Examples, the following designations are used: "Hu4D5" is an abbreviation for humanized anti-HER2 4D5 antibody, chinese hamster ovary are abbreviated as "CHO", CHO-DP12 cells cultured in 15 cm plates are designated "CHO-P", CHO-DP12 cells cultured in spinner flasks are designated "CHO-S", Human embryonic kidney 293 cells are abbreviated as "HEK293", "Lec 13" represents the CHO cell line with defective fucose metabolism obtained from Pamela Stanley from the Albert Einstein College of Medicine of Yeshiva University, Bronx, New York, Hu4D5 with S298A/E333A/K334A substitutions in the Fc region thereof is called "Hu4D5-AAA", "E27" is the affinity matured/humanized anti-IgE antibody described in U.S. Patent No. 6,172,213, E27 with S298A/E333A/K334A substitutions in the Fc region thereof is designated "E27-AAA", and peripheral blood mononuclear cell antibody-dependent cell-mediated cytotoxicity is abbreviated "PBMC ADCC."
Figure 4 shows binding of Hu4D5 monomers to human FcγRI. The Hu4D5 antibody was expressed in CHO-S, HEK293 cells, CHO-P, or Lec13 CHO cells (two different batches).
Figure 5 shows binding of Hu4D5 dimers to human FcγRIIB. The Hu4D5 antibody was expressed in CHO-S or Lec13 cells (three different batches).
Figure 6 shows binding of Hu4D5 dimers to human FcγRIIA(R131). The Hu4D5 antibody was expressed in CHO-S or Lec13 cells (three different batches).
Figure 7 illustrates binding of Hu4D5 dimers to human FcγRIIA(H131). The Hu4D5 antibody was expressed in CHO-S or Lec13 cells (three different batches).
Figure 8 shows binding of Hu4D5 or Hu4D5-AAA dimers expressed in CHO-S or Lec13 cells (three and two different batches, respectively), to human FcγRIIIA(V158).
Figure 9 reveals binding of Hu4D5 dimers expressed in CHO-S or Lec13 cells (three different batches) or Hu4D5-AAA dimers expressed in Lec13 cells (two different batches) to human FcγRIIIA(F158).
Figure 10 depicts binding of anti-IgE (E27) dimers to human FcγRIIIA(V158). E27 expressed in HEK293 cells, CHO-P cells (two batches) or Lec13 cells (two batches) was tested in this assay.
Figure 11 depicts binding of anti-IgE (E27) dimers to human FcγRIIIA(F158). E27 expressed in HEK293 cells, CHO-P cells (two batches) or Lec13 cells (two batches) was tested in this assay.
Figure 12 illustrates binding of anti-IgE (E27) hexamers and E27-AAA to FcγRIIIA(F158). The antibodies were expressed in CHO-P, Lec13 or HEK293 cells.
Figure 13 illustrates binding of anti-IgE (E27) hexamers and E27-AAA to FcγRIIIA(V158). The antibodies were expressed in CHO-P, Lec13 or HEK293 cells.
Figure 14 depicts binding of Hu4D5 expressed in CHO-P, CHO-S or Lec13 cells to human FcRn.
Figure 15 depicts binding of Hu4D5 and anti-CD20 (RITUXAN®) to human C1q. Hu4D5 was expressed in CHO-P or Lec13 cells (two batches). RITUXAN® was expressed in CHO-P cells.
Figure 16 represents binding of Hu4D5 or RITUXAN® to human C1q. Hu4D5 used in this experiment was expressed in CHO-P, Lec13 (three different batches) or CHO-S cells. RITUXAN® was expressed in in CHO-P cells.
Figure 17 depicts PBMC ADCC of SKBR3 breast tumor cells (E:T 30:1) using a FcγRIII VF donor. Spontaneous ADCC compared to that resulting from Hu4D5 expressed in CHO-S or Lec13 cells is shown.
Figure 18 depicts PBMC ADCC of SKBR3 breast tumor cells (E:T 30:1) using another FcγRIII VF donor. Spontaneous ADCC compared to that resulting from Hu4D5 expressed in CHO-S or Lec13 cells is shown.
Figure 19 depicts PBMC ADCC of SKBR3 breast tumor cells (E:T 30:1) using a FcγRIII FF donor. Spontaneous ADCC compared to that resulting from Hu4D5 expressed in CHO-S or Lec13 cells is shown.
Figure 20 depicts PBMC ADCC of SKBR3 breast tumor cells (E:T 30:1) using another FcγRIII FF donor. Spontaneous ADCC compared to that resulting from Hu4D5 expressed in CHO-S or Lec13 cells is shown.
Figure 21 depicts monocyte ADCC of SKBR3 breast tumor cells (E:T 10:1) using a FcγRIIA RR donor. Spontaneous ADCC compared to that resulting from Hu4D5 expressed in CHO-S or Lec13 cells (two different batches) is shown.
Figure 22 depicts monocyte ADCC of SKBR3 breast tumor cells (E:T 10:1) using a FcγRIIA HH donor. Spontaneous ADCC compared to that resulting from Hu4D5 expressed in CHO-S or Lec13 cells is shown.

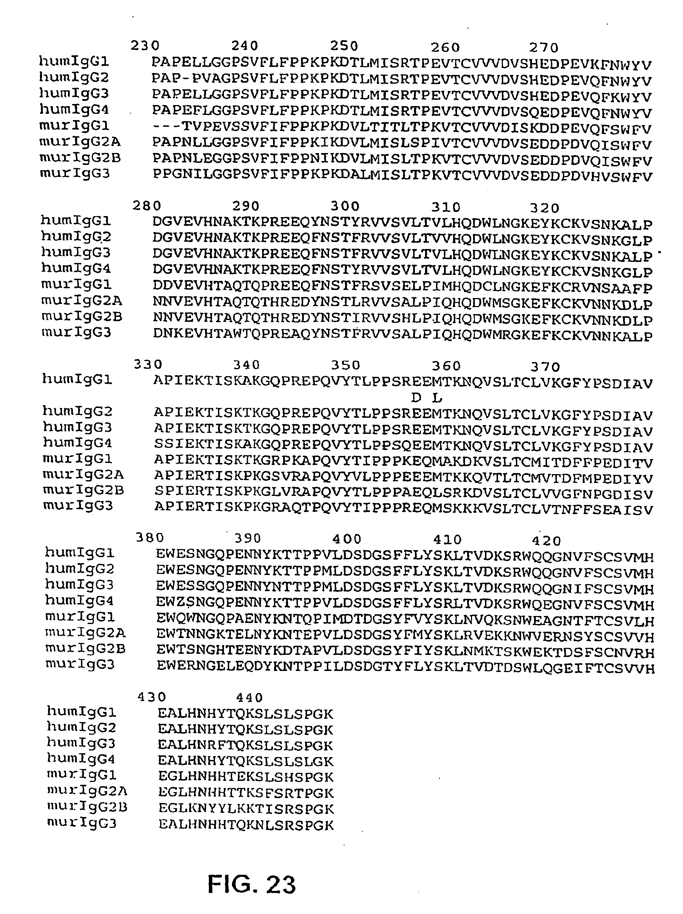

Figure 23 depicts alignments of native sequence IgG Fc regions. Native sequence human IgG Fc region sequences, humIgG1 (non-A and A allotypes) (SEQ ID NOs: 1 and 2, respectively), humIgG2 (SEQ ID NO:3), humIgG3 (SEQ ID NO:4) and humIgG4 (SEQ ID NO:5), are shown. The human IgG1 sequence is the non-A allotype, and differences between this sequence and the A allotype (at positions 356 and 358; EU numbering system) are shown below the human IgG1 sequence. Native sequence murine IgG Fc region sequences, murIgG1 (SEQ ID NO:6), murIgG2A (SEQ ID NO:7), murIgG2B (SEQ ID NO:8) and murIgG3 (SEQ ID NO:9), are also shown.
Figure 24 depicts binding of Hu4D5 and Hu4D5-AAA to CD56 positive natural killer (NK) cells. The products tested were: (1) FITC conjugated anti-human IgG, (2) Hu4D5 from CHO-S, (3) Hu4D5 expressed in Lec 13 cells, and (4) Hu4D5-AAA expressed in Lec 13 cells.
Figure 25 reveals immunofluorescense staining of purified NK cells expressing FcγRIII (F/F) receptors.
Figure 26 provides a comparison of the NK ADDC activity of Hu4D5 from CHO-S, Hu4D5 from Lec13 cells, Hu4D5-AAA from Lec 13 cells, and Hu4D5 from HEK293 cells. The donor was FcγRIII (F/F).
Figure 27 repeats the experiment in Figure 26 with a different FcγRIII (F/F) donor.
Figure 28 depicts binding of anti-HER2 Hu4D5 monomers to CHO cell line stable-transfected with human FcγRIIIA α-chain and γ-chain (representative plot for one assay). Hu4D5 CHO-S, open circles; Hu4D5 Lec13-D, open squares; Hu4D5 Lec13-E, open diamonds; Hu4D5 Lec13-F, open triangles; Hu4D5 HEK293-AAA, filled circles; Hu4D5 Lec13-AAA-B, filled squares; Hu4D5 Lec13-AAA-C, filled diamonds.

### Detailed Description of the Preferred Embodiments

### I. Definitions

Throughout the present specification and claims, the numbering of the residues in an immunoglobulin heavy chain is that of the EU index as in Kabat et al., Sequences of Proteins of Immunological Interest, 5th Ed. Public Health Service, National Institutes of Health, Bethesda, MD (1991), expressly incorporated herein by reference. The "EU index as in Kabat" refers to the residue numbering of the human IgG1 EU antibody.

The carbohydrate moieties of the present invention will be described with reference to commonly used nomenclature for the description of oligosaccharides. A review of carbohydrate chemistry which uses this nomenclature is found in Hubbard et al. Ann. Rev. Biochem. 50:555-583 (1981). This nomenclature includes, for instance, Man, which represents mannose; GlcNAc, which represents 2-N-acetylglucosamine; Gal which represents galactose; Fuc for fucose; and Glc, which represents glucose. Sialic acids are described by the shorthand notation NeuNAc, for 5-N-acetylneuraminic acid, and NeuNGc for 5-glycolylneuraminic.

The term "glycosylation" means the attachment of oligosaccharides (carbohydrates containing two or more simple sugars linked together *e*.*g*. from two to about twelve simple sugars linked together) to a glycoprotein. The oligosaccharide side chains are typically linked to the backbone of the glycoprotein through either N- or O-linkages. The oligosaccharides of the present invention occur generally are attached to a CH2 domain of an Fc region as N-linked oligosaccharides.

"N-linked glycosylation" refers to the attachment of the carbohydrate moiety to an asparagine residue in a glycoprotein chain. The skilled artisan will recognize that, for example, each of murine IgG1, IgG2a, IgG2b and IgG3 as well as human IgG1, IgG2, IgG3, IgG4, IgA and IgD CH2 domains have a single site for N-linked glycosylation at amino acid residue 297 (Kabat et al. Sequences of Proteins of Immunological Interest, 1991).

"Glycoproteins" are polypeptides having one or more oligosaccharide side chains attached thereto.

For the purposes herein, a "mature core carbohydrate structure" refers to a processed core carbohydrate structure attached to an Fc region which generally consists of the following carbohydrate structure GlcNAc(Fucose)-GlcNAc-Man-(Man-GlcNAc)₂ typical of biantennary oligosaccharides represented schematically below:

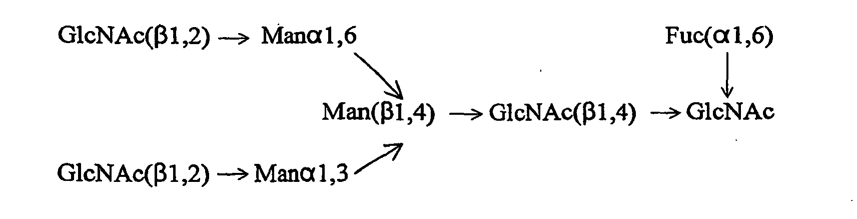

This term specifically includes G-1 forms of the core mature carbohydrate structure lacking a β1,2 GlcNAc residue. Preferably, however, the core carbohydrate structure includes both β1,2 GlcNAc residues. The mature core carbohydrate structure herein generally is not hypermannosylated.

The mature core carbohydrate structure is attached to the Fc region of the glycoprotein, generally via N-linkage to Asn297 of a CH2 domain of the Fc region.

A "bisecting GlcNAc" is a GlcNAc residue attached to the β1,4 mannose of the mature core carbohydrate structure. The bisecting GlcNAc can be enzymatically attached to the mature core carbohydrate structure by a β(1,4)-N-acetylglucosaminyltransferase III enzyme (GnTIII). CHO cells do not normally express GnTIII (Stanley et al. J. Biol. Chem. 261:13370-13378 (1984)), but may be engineered to do so (Umana et al. Nature Biotech. 17:176-180 (1999)).

A glycoprotein that is "essentially free" of one or more selected sugar groups (*e*.*g*. bisecting GlcNAc, one or more galactose residues, or one or more sialic acid residues) is generally produced in a host cell that is defective in the addition of the selected sugar group(s) to the mature core carbohydrate structure, such that about 90-100% of the glycoprotein in a composition will lack the selected sugar group(s) attached to the mature core carbohydrate structure.

A "glycosidase" is an enzyme involved in the biosynthesis of asparagine-linked (N-linked) glycoproteins. A "trimming" enzyme is one which removes oligosaccharide(s), whereas a "transferase" adds oligosaccharide(s). Examples of glycosidases include trimming glucosidases such as glucosidase I and glucosidase II; trimming mannosidases such as rough endoplasmic reticulum mannosidase (rER mannosidase), mannosidase IA, mannosidase IB and mannosidase II; as well as transferases such as glycosyl transferases, *e*.*g*. β(1,4)-N-acetylglucosaminyltransferase III (GnT III), Gal-transferases, sialic-acid-transferases and fuc-transferases.

A "glycosidase inhibitor" refers to a compound or composition which reduces or prevents N-linked oligosaccharide processing by one or more glycosidase(s). Examples include, nojirimycin, 1-deoxynojirimycin (dNM), *N*-Methyl-1-deoxy-nojirimycin (M-dNM), castanospermine, bromoconduritol, 1-deoxymannojirimycin (dMM), australine, MDL, lentiginosine, and Swainsonine (Sw). Glycosidase inhibitors are reviewed in Fuhrmann et al. Biochim. Biophys. Acta 825:95-110 (1985); Kaushal and Elbein, Methods in Enzym. 230:316-329 (1994); and Elbein, A. FASEB 5:3055-3063 (1991).

"Lec13" refers to the lectin-resistant Chinese Hamster Ovary (CHO) mutant cell line which displays a defective fucose metabolism and therefore has a diminished ability to add fucose to complex carbohydrates. That cell line is described in Ripka and Stanley, Somatic Cell & Molec. Gen. 12(1):51-62 (1986); and Ripka et al. Arch. Biochem. Biophys. 249(2):533-545 (1986) and is available from the Albert Einstein College of Medicine of Yeshiva University, Bronx, New York. Lec13 cells are believed lack the transcript for GDP-D-mannose-4,6-dehydratase, a key enzyme for fucose metabolism. Ohyama et al. J. Biol. Chem. 273(23):14582-14587 (1988). GDP-D-mannose-4,6-dehydratase generates GDP-mannose-4-keto-6-D-deoxymannose from GDP-mannose, which is then converted by the FX protein to GDP-L-fucose. Expression of fucosylated oligosaccharides is dependent on the GDP-L-fucose donor substrates and fucosyltransferase(s).

A "fucosyltransferase" is an enzyme that adds one or more fucose(s) to a glycoprotein. Examples include α1,6-fucosyltransferase, FucTI, FucTII, FucTIII, FucTIV, FucTV, FucTVI and FucTVII. Porcine and human α1,6-fucosyltransferases are described in Uozumi et al. J. Biol. Chem. 271:27810-27817 (1996), and Yanagidani et al. J. Biochem. 121:626-632 (1997), respectively.

A "sialyltransferase" is an enzyme that adds one or more sialic acid residue(s) to a glycoprotein. An α2,3 sialytransferase can add sialic acid residue(s) to galactose residue(s) attached to a mature core carbohydrate structure.

A "galactotransferase" is an enzyme that adds one or more galactose residue(s) to a glycoprotein. A β1,4-galactosyltransferase can add galactose residue(s) to the mature core carbohydrate structure.

The term "Fc region-containing glycoprotein" refers to a glycoprotein,' such as an antibody or immunoadhesin, which comprises an Fc region.

The term "Fc region" is used to define a C-terminal region of an immunoglobulin heavy chain, *e*.*g*., as shown in Figure 1A. The "Fc region" may be a native sequence Fc region or a variant Fc region. Although the boundaries of the Fc region of an immunoglobulin heavy chain might vary, the human IgG heavy chain Fc region is usually defined to stretch from as amino acid residue at position Cys226, or from Pro230, to the carboxyl-terminus thereof. The Fc region of an immunoglobulin generally comprises two constant domains, CH2 and CH3, as shown, for example, in Fig. 1A.

A "functional Fc region" possesses an "effector function" of a native sequence Fc region. Exemplary "effector functions" include C1q binding; complement dependent cytotoxicity; Fc receptor binding; antibody-dependent cell-mediated cytotoxicity (ADCC); phagocytosis; down regulation of cell surface receptors (*e*.*g*. B cell receptor; BCR), etc. Such effector functions generally require the Fc region to be combined with a binding domain (*e*.*g*. an antibody variable domain) and can be assessed using various assays as herein disclosed, for example.

A "native sequence Fc region" comprises an amino acid sequence identical to the amino acid sequence of a Fc region found in nature. Native sequence human Fc regions are shown in Fig. 23 and include a native sequence human IgG1 Fc region (non-A and A allotypes); native sequence human IgG2 Fc region; native sequence human IgG3 Fc region; and native sequence human IgG4 Fc region as well as naturally occurring variants thereof. Native sequence murine Fc regions are also shown in Fig. 23. Other examples of native sequence Fc regions include native sequence human IgA Fc region and native sequence human IgD Fc region.

A "variant Fc region" comprises an amino acid sequence which differs from that of a native sequence Fc region by virtue of at least one "amino acid modification" as herein defined. Preferably, the variant Fc region has at least one amino acid substitution compared to a native sequence Fc region or to the Fc region of a parent polypeptide, *e*.*g*. from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith.

"Homology" is defined as the percentage of residues in the amino acid sequence variant that are identical after aligning the sequences and introducing gaps, if necessary, to achieve the maximum percent homology. Methods and computer programs for the alignment are well known in the art. One such computer program is "Align 2," authored by Genentech, Inc., which was filed with user documentation in the United States Copyright Office, Washington, DC 20559, on December 10, 1991.

The terms "Fc receptor" or "FcR" are used to describe a receptor that binds to the Fc region of an antibody. The preferred FcR is a native sequence human FcR Moreover, a preferred FcR is one which binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see review M. in Daëron, Annu. Rev. Immunol. 15:203-234 (1997)). Fc receptors herein include the two known, naturally occuring allotypes, FcγRII(H131) and FcγRII(R131), of human FcγRII which are determined by the amino acid at position 131 (Clark et al. J. Immunol. 143: 1731-1734 (1989)), and the naturally occuring allotypes of human FcγRIIIA. Human FcγRIIIA has naturally occuring allotypes at position 48 (Leu, His or Arg) and at position 158 (Val or Phe). The FcγRIIIA(V158) allotype interacts with human IgG better than the FcγRIIIA(F158) allotype (Shields et al. J. Biol. Chem. 276: 6591-6604 (2001); Koene et al. Blood 90:1109-1114 (1997); and Wu et al. J. Clin. Invest. 100: 1059-1070 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

"Antibody-dependent cell-mediated cytotoxicity" and "ADCC" refer to a cell-mediated reaction in which nonspecific cytotoxic cells that express FcRs (*e*.*g*. Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source thereof, *e*.*g*. from blood or PBMCs.

"Hinge region" is generally defined as stretching from Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions.

The "lower hinge region" of an Fc region is normally defined as the stretch of residues immediately C-terminal to the hinge region, *i*.*e*. residues 233 to 239 of the Fc region.

The "CH2 domain" of the present invention is used herein to describe a CH2 domain having an attachment site for at least one N-linked oligosaccharide, generally at Asn297. It is characteristic of the glycoprotein of the present invention that it contain or be modified to contain at least a CH2 domain having an N-linked oligosaccharide of a human IgG CH2 domain. The CH2 domain is preferably the CHγ2 domain ofhuman IgG1. A human IgG CH2 domain usually extends from about amino acid 231 to about amino acid 340 of the Fc region, using the EU index for numbering of residues in an immunoglobulin heavy chain.

The "CH3 domain" comprises the stretch of residues C-terminal to a CH2 domain in an Fc region (*i*.*e*. from about amino acid residue 341 to about amino acid residue 447 of an IgG).

The terms "amino acids" and "amino acid" refer to all naturally occurring alpha amino acids in both their D and L stereoisomeric forms, and their analogs and derivatives. An analog is defined as a substitution of an atom in the amino acid with a different atom that usually has similar properties. A derivative is defined as an amino acid that has another molecule or atom attached to it. Derivatives would include, for example, acetylation of an amino group, amination of a carboxyl group, or oxidation of the sulfur residues of two cysteine molecules to form cysteine.

As used herein, "polypeptide " refers generally to peptides and proteins having more than about ten amino acids. The polypeptides may be homologous to a host cell in which they are expressed, or preferably, may be exogenous, meaning that they are heterologous, *i*.*e*., foreign, to the host cell being utilized, such as a chimeric, humanized or human antibody produced by a CHO cell.

The term "antibody" is used in the broadest sense and specifically covers monoclonal antibodies (including full length monoclonal antibodies), polyclonal antibodies, multispecific antibodies (*e*.*g*., bispecific antibodies), and antibody fragments so long as they exhibit the desired biological activity.

"Antibody fragments," as defined for the purpose of the present invention, comprise a portion of an intact antibody, generally including the antigen binding or variable region of the intact antibody or the Fc region of an antibody. Examples of antibody fragments include linear antibodies; single-chain antibody molecules; and multispecific antibodies formed from antibody fragments.

The term "monoclonal antibody" as used herein refers to an antibody obtained from a population of substantially homogeneous antibodies, *i*.*e*., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations that typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature 256:495 (1975), or may be made by recombinant DNA methods (see, *e*.*g*., U.S. Patent No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (*e*.*g*., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et al. Nature Biotechnology 14:309-314 (1996): Sheets et al. PNAS (USA) 95:6157-6162 (1998)); Hoogenboom and Winter, J. Mol. Biol., 227:381 (1991); Marks et al., J. Mol. Biol., 222:581 (1991)). Human antibodies can also be made by introducing human immunoglobulin loci into transgenic animals, *e*.*g*., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huszar, Intern. Rev. Immunol. 13:65-93 (1995). Alternatively, the human antibody may be prepared via immortalization of human B lymphocytes producing an antibody directed against a target antigen (such B lymphocytes may be recovered from an individual or may have been immunized *in vitro*)*.* See, *e*.*g*., Cole et al., Monoclonal Antibodies and Cancer Therapy, Alan R. Liss, p. 77 (1985); Boerner et al., J. Immunol., 147 (1):86-95 (1991); and US Pat No. 5,750,373.

The term "hypervariable region" when used herein refers to the amino acid residues of an antibody which are responsible for antigen-binding. The hypervariable region comprises amino acid residues from a "complementarity determining region" or "CDR" (*i*.*e*. residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (*i*.*e*. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)). "Framework" or "FR" residues are those variable domain residues other than the hypervariable region residues as herein defined.

As used herein, the term "immunoadhesin" designates antibody-like molecules which combine the "binding domain" of a heterologous "adhesin" protein (*e*.*g*. a receptor, ligand or enzyme) with an immunoglobulin constant domain. Structurally, the immunoadhesins comprise a fusion of the adhesin amino acid sequence with the desired binding specificity which is other than the antigen recognition and binding site (antigen combining site) of an antibody (*i*.*e*. is "heterologous") and an immunoglobulin constant domain sequence. '

The term "ligand binding domain" as used herein refers to any native cell-surface receptor or any region or derivative thereof retaining at least a qualitative ligand binding ability of a corresponding native receptor. In a specific embodiment, the receptor is from a cell-surface polypeptide having an extracellular domain that is homologous to a member of the immunoglobulin supergenefamily. Other receptors, which are not members of the immunoglobulin supergenefamily but are nonetheless specifically covered by this definition, are receptors for cytokines, and in particular receptors with tyrosine kinase activity (receptor tyrosine kinases), members of the hematopoietin and nerve growth factor receptor superfamilies, and cell adhesion molecules, *e*.*g*. (E-, L- and P-) selectins.

The term "receptor binding domain" is used to designate any native ligand for a receptor, including cell adhesion molecules, or any region or derivative of such native ligand retaining at least a qualitative receptor binding ability of a corresponding native ligand. This definition, among others, specifically includes binding sequences from ligands for the above-mentioned receptors.

An "antibody-immunoadhesin chimera" comprises a molecule that combines at least one binding domain of an antibody (as herein defined) with at least one immunoadhesin (as defined in this application). Exemplary antibody-immunoadhesin chimeras are the bispecific CD4-IgG chimeras described in Berg et al., PNAS (USA) 88:4723-4727 (1991) and Chamow et al., J. Immunol. 153:4268 (1994).

The term "preparation" as used herein is used to define a composition or glycoprotein which has been identified and separated and/or recovered as a component of its environment. Contaminant components of its environment are materials which would interfere with diagnostic or therapeutic uses for the composition or glycoprotein such as unwanted or unintended glycoforms, and may include enzymes, hormones, and other proteinaceous or nonproteinaceous solutes. The preparation of the invention is substantially free of these contaminants. In preferred embodiments, the glycoprotein preparation will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain.

For the purposes herein, a "pharmaceutical preparation" is one which is adapted and suitable for administration to a mammal, especially a human. Thus, the composition can be used to treat a disease or disorder in the mammal. Moreover, the glycoprotein which is the active ingredient in the composition has been subjected to one or more purification or isolation steps, such that contaminant(s) that might interfere with its therapeutic use have been separated therefrom. Generally, the pharmaceutical preparation comprises the therapeutic glycoprotein and a pharmaceutically acceptable carrier or diluent, examples of which are described hereinbelow. The preparation is usually sterile, and may be lyophilized.

For the purposes herein, a "parent glycoprotein" is a glycoprotein having the same amino acid sequence and mature core carbohydrate structure as a glycoprotein variant of the present invention, except that fucose is attached to the mature core carbohydrate structure. For instance, in a composition comprising the parent glycoprotein about 50-100% or about 70-100% of the parent glycoprotein comprises a mature core carbohydrate structure having fucose attached thereto.

The glycoprotein variant which binds an FcR with "better affinity" than a parent glycoprotein, is one which binds any one or more of the above identified FcRs with substantially better binding affinity than the parent glycoprotein, when the amounts of glycoprotein variant and parent polypeptide in the binding assay are essentially the same. For example, the glycoprotein variant with improved FcR binding affinity may display from about 5 fold to about 1000 fold, *e*.*g*. from about 10 fold to about 500 fold improvement in FcR binding affinity compared to the parent glycoprotein, where FcR binding affinity is determined, for example, as disclosed in the Examples herein.

The glycoprotein variant which "mediates antibody-dependent cell-mediated cytotoxicity (ADCC) in the presence of human effector cells more effectively" than a parent polypeptide is one which *in vitro* or *in vivo* is substantially more effective at mediating ADCC, when the amounts of glycoprotein variant and parent glycoprotein used in the assay are essentially the same. Generally, such glycoprotein variants will be identified using the *in vitro* ADCC assay as herein disclosed, but other assays or methods for determining ADCC activity, *e*.*g*. in an animal model etc, are contemplated. The preferred glycoprotein variant is from about 1.5 fold to about 100 fold, *e*.*g*. from about two fold to about fifty fold, more effective at mediating ADCC than the parent, *e*.*g*. in the *in vitro* assay disclosed herein.

An "amino acid modification" refers to a change in the amino acid sequence of a predetermined amino acid sequence. Exemplary modifications include an amino acid substitution, insertion and/or deletion. The preferred amino acid modification herein is a substitution.

An "amino acid modification at" a specified position, *e*.*g*. of the Fc region, refers to the substitution or deletion of the specified residue, or the insertion of at least one amino acid residue adjacent the specified residue. By insertion "adjacent" a specified residue is meant insertion within one to two residues thereof. The insertion may be N-terminal or C-terminal to the specified residue.

An "amino acid substitution" refers to the replacement of at least one existing amino acid residue in a predetermined amino acid sequence with another different "replacement" amino acid residue. The replacement residue or residues may be "naturally occurring amino acid residues" (*i*.*e*. encoded by the genetic code) and selected from the group consisting of: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val). Preferably, the replacement residue is not cysteine. Substitution with one or more non-naturally occurring amino acid residues is also encompassed by the definition of an amino acid substitution herein. A "non-naturally occurring amino acid residue" refers to a residue, other than those naturally occurring amino acid residues listed above, which is able to covalently bind adjacent amino acid residues(s) in a polypeptide chain. Examples of non-naturally occurring amino acid residues include norleucine, ornithine, norvaline, homoserine and other amino acid residue analogues such as those described in Ellman et al. Meth. Enzym. 202:301-336 (1991). To generate such non-naturally occurring amino acid residues, the procedures of Noren et al. Science 244:182 (1989) and Ellman *et al., supra*, can be used. Briefly, these procedures involve chemically activating a suppressor tRNA with a non-naturally occurring amino acid residue followed by *in vitro* transcription and translation of the RNA.

An "amino acid insertion" refers to the incorporation of at least one amino acid into a predetermined amino acid sequence. While the insertion will usually consist of the insertion of one or two amino acid residues, the present application contemplates larger "peptide insertions", *e*.*g*. insertion of about three to about five or even up to about ten amino acid residues. The inserted residue(s) may be naturally occurring or non-naturally occurring as disclosed above.

An "amino acid deletion" refers to the removal of at least one amino acid residue from a predetermined amino acid sequence.

"C1q" is a polypeptide that includes a binding site for the Fc region of an immunoglobulin. C1q together with two serine proteases, C1r and C1s, forms the complex C1, the first component of the complement dependent cytotoxicity (CDC) pathway. Human C1q can be purchased commercially from, *e*.*g*. Quidel, San Diego, CA.

"Treatment" refers to both therapeutic treatment and prophylactic or preventative measures. Those in need of treatment include those already with the disorder as well as those in which the disorder is to be prevented.

A "disorder" or "disease" herein is any condition that would benefit from treatment with the glycoprotein. This includes chronic and acute disorders or diseases including those pathological conditions which predispose the mammal to the disorder in question. In one embodiment, the disorder is cancer, an autoimmune disease, an inflammatory disorder, infection or other condition such as goiter where removal of unwanted tissue or cells is desired. The prefered disease or disorder to be treated herein is cancer or an autoimmune disease.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma and various types of head and neck cancer.

A "B-cell malignancy" herein includes non-Hodgkin's lymphoma (NHL), including low grade/follicular NHL, small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, and Waldenstrom's Macroglobulinemia; leukemia, including acute lymphoblastic leukemia (ALL), chronic lymphocytic leukemia (CLL), Hairy cell leukemia and chronic myeloblastic leukemia; and other hematologic malignancies. Such malignancies may be treated with antibodies directed against B-cell surface markers, such as CD20.

A "hormone independent" cancer is one in which proliferation thereof is not dependent on the presence of a hormone which binds to a receptor expressed by cells in the cancer. Such cancers do not undergo clinical regression upon administration of pharmacological or surgical strategies that reduce the hormone concentration in or near the tumor. Examples of hormone independent cancers include androgen independent prostate cancer, estrogen independent breast cancer, endometrial cancer and ovarian cancer. Such cancers may begin as hormone dependent tumors and progress from a hormone-sensitive stage to a hormone-refractory tumor following anti-hormonal therapy.

An "autoimmune disease" herein is a non-malignant disease or disorder arising from and directed against an individual's own tissues. Examples of autoimmune diseases or disorders include, but are not limited to, inflammatory responses such as inflammatory skin diseases including psoriasis and dermatitis (*e*.*g*. atopic dermatitis); systemic scleroderma and sclerosis; responses associated with inflammatory bowel disease (such as Crohn's disease and ulcerative colitis); respiratory distress syndrome (including adult respiratory distress syndrome; ARDS); dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE); diabetes mellitus (*e*.*g*. Type I diabetes mellitus or insulin dependent diabetes mellitis); multiple sclerosis; Reynaud's syndrome; autoimmune thyroiditis; allergic encephalomyelitis; Sjorgen's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia (including, but not limited to cryoglobinemia or Coombs positive anemia) ; myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia etc.

An "inflammatory disorder" refers to pathological states resulting in inflammation, typically caused by neutrophil chemotaxis. Examples of such disorders include inflammatory skin diseases including psoriasis and atopic dermatitis; systemic scleroderma and sclerosis; responses associated with inflammatory bowel disease (such as Crohn's disease and ulcerative colitis); ischemic reperfusion disorders including surgical tissue reperfusion injury, myocardial ischemic conditions such as myocardial infarction, cardiac arrest, reperfusion after cardiac surgery and constriction after percutaneous transluminal coronary angioplasty, stroke, and abdominal aortic aneurysms; cerebral edema secondary to stroke; cranial trauma; hypovolemic shock; asphyxia; adult respiratory distress syndrome; acute lung injury; Behcet's Disease; dermatomyositis; polymyositis; multiple sclerosis; dermatitis; meningitis; encephalitis; uveitis; osteoarthritis; lupus nephritis; autoimmune diseases such as rheumatoid arthritis, Sjorgen's syndrome, vasculitis; diseases involving leukocyte diapedesis; central nervous system (CNS) inflammatory disorder, multiple organ injury syndrome secondary to septicaemia or trauma; alcoholic hepatitis; bacterial pneumonia; antigen-antibody complex mediated diseases including glomerulonephritis; sepsis; sarcoidosis; immunopathologic responses to tissue/organ transplantation; inflammations of the lung, including pleurisy, alveolitis, vasculitis, pneumonia, chronic bronchitis, bronchiectasis, diffuse panbronchiolitis, hypersensitivity pneumonitis, idiopathic pulmonary fibrosis (IPF), and cystic fibrosis; etc. The preferred indications include acute lung injury, adult respiratory distress syndrome, ischemic reperfusion (including surgical tissue reperfusion injury, myocardial ischemia, and acute myocardial infarction), hypovolemic shock, asthma, bacterial pneumonia and inflammatory bowel disease such as ulcerative colitis. Autoimmune diseases may overlap with inflammatory disorders, and vice versa.

By "blocking an immune response" to a foreign antigen is meant reducing or preventing at least one immune-mediated response resulting from exposure to a foreign antigen. For example, one may dampen a humoral response to the foreign antigen, *i*.*e*., by preventing or reducing the production of antibodies directed against the antigen in the mammal. Alternatively, or additionally, one may suppress idiotype; "pacify" the removal of cells coated with alloantibody; and/or affect alloantigen presentation through depletion of antigen-presenting cells.

By "foreign antigen" is meant a molecule or molecules which is/are not endogenous or native to a mammal which is exposed to it. The foreign antigen may elicit an immune response, *e*.*g*. a humoral and/or T cell mediated response in the mammal. Generally, the foreign antigen will provoke the production of antibodies thereagainst. Examples of foreign antigens contemplated herein include immunogenic therapeutic agents, *e*.*g*. proteins such as antibodies, particularly antibodies comprising non-human amino acid residues (*e*.*g*. rodent, chimeric/humanized, and primatized antibodies); toxins (optionally conjugated to a targeting molecule such as an antibody, wherein the targeting molecule may also be immunogenic); gene therapy viral vectors, such as retroviruses and adenoviruses; grafts; infectious agents (*e*.*g*. bacteria and virus); alloantigens (*i*.*e*. an antigen that occurs in some, but not in other members of the same species) such as differences in blood types, human lymphocyte antigens (HLA), platelet antigens, antigens expressed on transplanted organs, blood components, pregnancy (Rh), and hemophilic factors (*e*.*g*. Factor VIII and Factor IX).

A "tumor-associated antigen" for the purposes herein is an antigen characterized by higher expression on tumor cells compared to normal cells. Specific examples include ErbB receptors, B-cell surface markers, ganglioside GD2, GD3 and GM2 (Ragupathi G., Cancer Immunol. Immunother. 43:152 (1996)); CD52 (Ginaldi et al., Leukemia Research 22:185 (1998)); prostate stem cell antigen (PSCA); and MAGE (Kirkin et al., APMIS 106:665 (1998)).

An "angiogenic factor" herein is a molecule which stimulates angiogenesis. Examples include vascular endothelial growth factor (VEGF), basic or acidic fibroblast growth factor (FGF), and platelet-derived endothelial cell growth factor (PD-ECGF).

An "ErbB receptor" is a receptor protein tyrosine kinase which belongs to the ErbB receptor family and includes EGFR, ErbB2, ErbB3 and ErbB4 receptors and other members of this family to be identified in the future. The ErbB receptor will generally comprise an extracellular domain, which may bind an ErbB ligand; a lipophilic transmembrane domain; a conserved intracellular tyrosine kinase domain; and a carboxyl-terminal signaling domain harboring several tyrosine residues which can be phosphorylated.

The terms "ErbB1", "epidermal growth factor receptor" and "EGFR" are used interchangeably herein and refer to EGFR as disclosed, for example, in Carpenter et al. Ann. Rev. Biochem. 56:881-914 (1987), including naturally occurring mutant forms thereof (*e*.*g*. a deletion mutant EGFR as in Humphrey et al. PNAS (USA) 87:4207-4211 (1990)). *erb*B1 refers to the gene encoding the EGFR protein product.

The expressions "ErbB2" and "HER2" are used interchangeably herein and refer to human HER2 protein described, for example, in Semba et al., PNAS (USA) 82:6497-6501 (1985) and Yamamoto et al. Nature 319:230-234 (1986) (Genebank accession number X03363).

Examples of antibodies that bind HER2 include 4D5, 7C2, 7F3 and 2C4, as well as humanized variants thereof, including huMAb4D5-1, huMAb4D5-2, huMAb4D5-3, huMAb4D5-4, huMAb4D5-5, huMAb4D5-6, huMAb4D5-7 and huMAb4D5-8 as described in Table 3 of U.S. Patent 5,821,337 expressly incorporated herein by reference; and humanized 2C4 mutant nos. 560, 561, 562, 568, 569, 570, 571, 574, or 56869 as described in WO01/00245. 7C2 and 7F3 and humanized variants thereof are described in WO98/17797. The preferred antibodies are those comprising the heavy and light variable regions of huMAb4D5-8, or humanized 2C4 mutant 574.

"Trastuzumab" (HERCEPTIN®) is a recombinant DNA-derived humanized antibody that binds with high affinity in a cell-based assay (Kd = 5 nM) to the extracellular domain of HER2. The antibody is an IgG1 antibody that comprises the heavy and light chain variable regions of the variant huMAb4D5-8 as described in Table 3 of U.S. Patent 5,821,337. The antibody is produced by CHO-DP12 cells.

"ErbB3" and "HER3" refer to the receptor polypeptide as disclosed, for example, in US Pat. Nos. 5,183,884 and 5,480,968 as well as Kraus et al. PNAS (USA) 86:9193-9197 (1989).

The terms "ErbB4" and "HER4" herein refer to the receptor polypeptide as disclosed, for example, in EP Pat Appln No 599,274; Plowman et al., Proc. Natl. Acad. Sci. USA, 90:1746-1750 (1993); and Plowman et al., Nature, 366:473-475 (1993), including isoforms thereof, *e.g.,* as disclosed in WO99/19488, published April 22, 1999.

A "B cell surface marker" herein is an antigen expressed on the surface of a B cell which can be targeted with an antibody which binds thereto. Exemplary B cell surface markers include the CD10, CD19, CD20, CD21, CD22, CD23, CD24, CD40, CD37, CD53, CD72, CD73, CD74, CDw75, CDw76, CD77, CDw78, CD79a, CD79b, CD80, CD81, CD82, CD83, CDw84, CD85 and CD86 leukocyte surface markers. The B cell surface marker of particular interest is preferentially expressed on B cells compared to other non-B cell tissues of a mammal and may be expressed on both precursor B cells and mature B cells. In one embodiment, the marker is one, like CD20 or CD 19, which is found on B cells throughout differentiation of the lineage from the stem cell stage up to a point just prior to terminal differentiation into plasma cells. The preferred B cell surface markers herein are CD19, CD20, CD22 and CD40.

The "CD20" antigen is a ∼35 kDa, non-glycosylated phosphoprotein found on the surface of greater than 90% of B cells from peripheral blood or lymphoid organs. CD20 is expressed during early pre-B cell development and remains until plasma cell differentiation. CD20 is present on both normal B cells as well as malignant B cells. Other names for CD20 in the literature include "B-lymphocyte-restricted antigen" and "Bp35". The CD20 antigen is described in Clark et al. PNAS (USA) 82:1766 (1985), for example.

Examples of antibodies which bind the CD20 antigen include: "C2B8" which is now called "Rituximab" ("RITUXAN®") (US Patent No. 5,736,137, expressly incorporated herein by reference); the yttrium-[90]-labeled 2B8 murine antibody designated "Y2B8" (US Patent No. 5,736,137, expressly incorporated herein by reference); murine IgG2a "B1" optionally labeled with ¹³¹I to generate the "¹³¹I-B1" antibody (BEXXAR^{™}) (US Patent No. 5,595,721, expressly incorporated herein by reference); murine monoclonal antibody "1F5" (Press et al. Blood 69(2):584-591 (1987)); "chimeric 2H7" antibody (US Patent No. 5,677,180, expressly incorporated herein by reference); and monoclonal antibodies L27, G28-2, 93-1B3, B-C1 or NUB2 available from the International Leukocyte Typing Workshop (Valentine et al., In: Leukocyte Typing III (McMichael, Ed., p. 440, Oxford University Press (1987)).

The terms "Rituximab" or "RITUXAN®" herein refer to the genetically engineered chimeric murine/human monoclonal antibody directed against the CD20 antigen and designated "C2B8" in US Patent No. 5,736,137, expressly incorporated herein by reference. The antibody is an IgG₁ kappa immunoglobulin containing murine light and heavy chain variable region sequences and human constant region sequences. Rituximab has a binding affinity for the CD20 antigen of approximately 8.0nM. Rituximab is produced by CHO DG44 cells.

The term "mammal" includes any animals classified as mammals, including humans, cows, horses, dogs and cats. In a preferred embodiment of the invention, the mammal is a human.

A "growth inhibitory agent" when used herein refers to a compound or composition which inhibits growth of a cell (*e*.*g*. a cancer cell) either *in vitro* or *in vivo*. Thus, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13.

Examples of "growth inhibitory" antibodies are those which bind to an antigen and inhibit the growth of cells expressing that antigen. Preferred growth inhibitory anti-HER2 antibodies inhibit growth of SK-BR-3 breast tumor cells in cell culture by greater than 20%, and preferably greater than 50% (*e*.*g*. from about 50% to about 100%) at an antibody concentration of about 0.5 to 30 µg/ml, where the growth inhibition is determined six days after exposure of the SK-BR-3 cells to the antibody (see U.S. Patent No. 5,677,171 issued October 14, 1997). The preferred growth inhibitory antibody is huMAb4D5-8.

An antibody which "induces cell death" is one which causes a viable cell to become nonviable. The cell here is one which expresses the antigen to which the antibody binds. Cell death *in vitro* may be determined in the absence of complement and immune effector cells to distinguish cell death induced by antibody-dependent cell-mediated cytotoxicity (ADCC) or complement dependent cytotoxicity (CDC). Thus, the assay for cell death may be performed using heat inactivated serum (*i*.*e*. in the absence of complement) and in the absence of immune effector cells. To determine whether the antibody is able to induce cell death, loss of membrane integrity as evaluated by uptake of propidium iodide (PI), trypan blue (see Moore et al. Cytotechnology 17:1-11 (1995)) or 7AAD can be assessed relative to untreated cells. Preferred cell death-inducing antibodies are those which induce PI uptake in the PI uptake assay in BT474 cells.

An antibody which "induces apoptosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies). The cell expresses the antigen to which the antibody binds. Preferably the cell is a tumor cell. Various methods are available for evaluating the cellular events associated with apoptosis. For example, phosphatidyl serine (PS) translocation can be measured by annexin binding; DNA fragmentation can be evaluated through DNA laddering; and nuclear/chromatin condensation along with DNA fragmentation can be evaluated by any increase in hypodiploid cells. Preferably, the antibody which induces apoptosis is one which results in about 2 to 50 fold, preferably about 5 to 50 fold, and most preferably about 10 to 50 fold, induction of annexin binding relative to untreated cell in an annexin binding assay using BT474 cells.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (*i*.*e*., slow to some extent and preferably stop) cancer cell infiltration into peripheral organs; inhibit (*i*.*e*., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can, for example, be measured by assessing the time to tumor progression (TTP) and/or determining the response rate (RR).

An "antigen-expressing cancer" is one comprising cells which have sufficient levels of antigen at the surface of cells thereof, such that an anti-antigen antibody can bind thereto and have a therapeutic effect with respect to the cancer.

A cancer "characterized by excessive activation" of an receptor is one in which the extent of receptor activation in cancer cells significantly exceeds the level of activation of that receptor in non-cancerous cells of the same tissue type. Such excessive activation may result from overexpression of the receptor and/or greater than normal levels of a ligand available for activating the receptor in the cancer cells. Such excessive activation may cause and/or be caused by the malignant state of a cancer cell. In some embodiments, the cancer will be subjected to a diagnostic or prognostic assay to determine whether amplification and/or overexpression of a receptor is occurring which results in such excessive activation of the receptor. Alternatively, or additionally, the cancer may be subjected to a diagnostic or prognostic assay to determine whether amplification and/or overexpression a ligand is occurring in the cancer which attributes to excessive activation of the receptor. In a subset of such cancers, excessive activation of the receptor may result from an autocrine stimulatory pathway.

A cancer which "overexpresses" a receptor is one which has significantly higher levels of a receptor, such as HER2, at the cell surface thereof, compared to a noncancerous cell of the same tissue type. Such overexpression may be caused by gene amplification or by increased transcription or translation. Receptor overexpression may be determined in a diagnostic or prognostic assay by evaluating increased levels of the receptor protein present on the surface of a cell (*e*.*g*. via an immunohistochemistry assay; IHC). Alternatively, or additionally, one may measure levels of receptor-encoding nucleic acid in the cell, *e*.*g*. via fluorescent *in situ* hybridization (FISH; see WO98/45479 published October, 1998), southern blotting, or polymerase chain reaction (PCR) techniques, such as real time quantitative PCR (RT-PCR). One may also study receptor overexpression by measuring shed antigen (*e*.*g*., extracellular domain) in a biological fluid such as serum (see, *e*.*g*., U.S. Patent No. 4,933,294 issued June 12, 1990; WO91/05264 published April 18, 1991; U.S. Patent 5,401,638 issued March 28,1995; and Sias et al. J. Immunol. Methods 132: 73-80 (1990)). Aside from the above assays, various *in vivo* assays are available to the skilled practitioner. For example, one may expose cells within the body of the patient to an antibody which is optionally labeled with a detectable label, *e*.*g*. a radioactive isotope, and binding of the antibody to cells in the patient can be evaluated, *e*.*g*. by external scanning for radioactivity or by analyzing a biopsy taken from a patient previously exposed to the antibody.

A cancer which "overexpresses" a ligand is one which produces significantly higher levels of that ligand compared to a noncancerous cell of the same tissue type. Such overexpression may be caused by gene amplification or by increased transcription or translation. Overexpression of the ligand may be determined diagnostically by evaluating levels of the ligand (or nucleic acid encoding it) in the patient, *e*.*g*. in a tumor biopsy or by various diagnostic assays such as the IHC, FISH, southern blotting, PCR or *in vivo* assays described above.

The term "cytotoxic agent" as used herein refers to a substance that inhibits or prevents the function of cells and/or causes destruction of cells. The term is intended to include radioactive isotopes (*e*.*g*. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of cancer. Examples of chemotherapeutic agents include alkylating agents such as thiotepa and cyclosphosphamide (CYTOXAN^{™}); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carninomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxanes, *e.g*. paclitaxel (TAXOL^{®}, Bristol-Myers Squibb Oncology, Princeton, NJ) and docetaxel (TAXOTERE®, Rhône-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylomithine (DMFO); retinoic acid; esperamicins; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

As used herein, the term "EGFR-targeted drug" refers to a therapeutic agent that binds to EGFR and, optionally, inhibits EGFR activation. Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn et al.) and variants thereof, such as chimerized 225 (C225) and reshaped human 225 (H225) (see, WO 96/40210, Imclone Systems Inc.); antibodies that bind type II mutant EGFR (US Patent No. 5,212,290); humanized and chimeric antibodies that bind EGFR as described in US Patent No. 5,891,996; and human antibodies that bind EGFR (see WO98/50433, Abgenix). The anti-EGFR antibody may be conjugated with a cytotoxic agent, thus generating an immunoconjugate (see, *e.g*., EP659,439A2, Merck Patent GmbH). Examples of small molecules that bind to EGFR include ZD1839 (IRESSA^{®} )(Astra Zeneca), CP-358774 or OSI-774 (TARCEVA^{™}) (Genentech) and AG1478.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-10, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "prodrug" as used in this application refers to a precursor or derivative form of a pharmaceutically active substance that is less cytotoxic to tumor cells compared to the parent drug and is capable of being enzymatically activated or converted into the more active parent form. See, *e.g.,* Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

A "liposome" is a small vesicle composed of various types of lipids, phospholipids and/or surfactant which is useful for delivery of a drug (such as the glycoprotein compositions disclosed herein and, optionally, a chemotherapeutic agent) to a mammal. The components of the liposome are commonly arranged in a bilayer formation, similar to the lipid arrangement of biological membranes.

The term "package insert" is used to refer to instructions customarily included in commercial packages of therapeutic products, that contain information about the indications, usage, dosage, administration, contraindications and/or warnings concerning the use of such therapeutic products.

An "isolated" nucleic acid molecule is a nucleic acid molecule that is identified and separated from at least one contaminant nucleic acid molecule with which it is ordinarily associated in the natural source of the polypeptide nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the polypeptide where, for example, the nucleic acid molecule is in a chromosomal location different from that of natural cells.

The expression "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

### II. Modes for Carrying Out the Invention

The invention herein relates to a method for making a substantially homogeneous preparation of an Fc region-containing glycoprotein, wherein about 80-100% of the glycoprotein in the composition comprises a mature core carbohydrate lacking fucose, attached to the Fc region of the glycoprotein. In the preferred embodiments herein, the protein is an antibody or immunoadhesin. The glycoproteins can be prepared, for example, by (a) use of an engineered or mutant host cell that is deficient in fucose metabolism such that it has a reduced ability (or is unable to) fucosylate proteins expressed therein; (b) culturing cells under conditions which prevent or reduce fucosylation; (c) post-translational removal of fucose (*e.g*. with a fucosidase enzyme); (d) post-translational addition of the desired carbohydrate, *e.g*. after recombinant expression of a non-glycosylated glycoprotein; (e) purification of the glycoprotein so as to select for product which is not fucosylated. The present invention contemplates combining two or more of these exemplary methods (a)-(e).

Most preferably, nucleic acid encoding the desired glycoprotein is expressed in a host cell that has is has a reduced ability (or is unable to) fucosylate proteins expressed therein. Preferably, the host cell is a dihydrofolate reductase (DHFR) deficient chinese hamster ovary (CHO) cell, *e.g*. a Lec13 CHO cell, or *e.g*., a CHO-K1, DUX-B11, CHO-DP12 or CHO-DG44 CHO host cell which has been modified so that the glycoprotein produced therein is not substantially fucosylated. Thus, the cell may display altered expression or activity for the fucosyltransferase enzyme, or another enzyme or substrate involved in adding fucose to the N-linked oligosaccharide may have diminished activity and/or reduced levels in the host cell.

The core carbohydrate structure is mature, thus, the use of inhibitors, such as castanospermine, which inhibit or interfere with processing of the mature carbohydrate should generally be avoided. According to one preferred embodiment of the invention, about 80-100% of the glycoprotein in the composition recovered from the recombinant host cell producing the glycoprotein will have a core carbohydrate structure which lacks fucose attached to the Fc region of the glycoprotein, hereinafter a "fucose-free glycoprotein composition." By "recovered" here is meant that material obtained directly from the host cell culture without subjecting that material to a purification step which enriches for fucose-free glycoprotein.

However, the present invention does contemplate enriching the amount of fucose-free glycoprotein by various techniques, such as purification using a lectin substrate to remove fucose-containing glycoprotein from the desired composition.

It will be appreciated that the amount of fucose-free glycoprotein from various batches of recombinantly produced glycoprotein may vary. For instance, in the Examples below, the % of total oligosaccharide without fucose attached to the glycoprotein expressed by CHO-Lec13 cells ranged from 88%-95%.

Preferably about 90-99% of the glycoprotein in the composition comprises a mature core carbohydrate structure which lacks fucose attached to the Fc region of the glycoprotein.

Various forms of the carbohydrate structure may exist in the composition. For instance, the carbohydrate attached to the glycoprotein may be represented by the following formula: 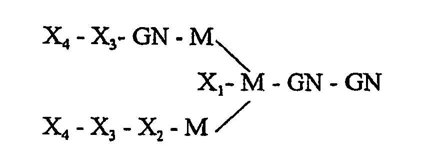 wherein,
M is mannose.
GN is GlcNAc.
X₁ is an optional bisecting GlcNAc residue, with additional monosaccharide(s) optionally attached to the bisecting GlcNAc.
X₂ is a prefered GlcNAc residue.
X₃ is an optional Gal residue, one Gal residue may be attached to each GN arm.
X₄ is an optional terminal sialic acid residues, one or two sialic acid residues may be attached.

The fucose-free glycoprotein compositions herein display improved binding to one or more FcγRIII receptors, compared to a composition of the same glycoprotein, but where most (*e.g*. about 50-100%, or about 70-100%) of the glycoprotein in that composition has fucose attached to the mature core carbohydrate structure (hereinafter a "fucose-containing glycoprotein composition). For instance, the fucose-free glycoprotein compositions herein may display 100-1000 fold improved binding to an FcγRIII, such as FcγRIII(F158), when compared to the fucose-containing glycoprotein composition. In that the F158 allotype is less effective in interacting with human IgG than V158, this is thought to provide a significant advantage from a therapeutic perspective, especially in patients who express FcγRIII(F158). Moreover, the fucose-free glycoprotein compositions herein display better ADCC activity compared to their counterpart fucose-containing glycoprotein compositions, *e.g*. from about 2-20 fold improved ADCC activity.

Aside from the fucose-free mature core carbohydrate structure, additional oligosaccharides may be attached to the core carbohydrate structure. For instance, a bisecting GlcNAc may, or may not be, attached. By the way of example, the host cell may lack the GnTIII enzyme and hence the glycoprotein may be essentially free of bisecting GlcNAc. Alternatively, the glycoprotein may be expressed in host cell (*e.g*. a Y0 host or engineered CHO cell) which adds the bisecting GlcNAc. One or more (generally one or two) galactose residues may also be attached to the core carbohydrate structure. Finally, one or more terminal sialic acid residues (usually one or two) may be attached to core carbohydrate structure, *e.g*. by linkage to galactose residue(s).

The compositions herein are, in the preferred embodiment, prepared and intended for therapeutic use. Hence, the preferred composition is a pharmaceutical preparation comprising the glycoprotein and a pharmaceutically acceptable carrier or diluent such as those exemplified below. Such preparations are usually sterile and may be lyophilized.

In the preferred embodiment of the invention, the glycoprotein is an antibody and exemplary methods for generating antibodies are described in more detail in the following sections. The glycoprotein may, however, be any other glycoprotein comprising an Fc region, *e.g*. an immunoadhesin. Methods for making immunoadhesins are elaborated in more detail hereinbelow.

### A. Variant Fc Region Sequences

In one embodiment of the invention, the glycosylation variant further comprises a variant Fc region with an amino acid sequence which differs from that of a native sequence Fc region. Where the variant Fc region has more than one amino acid substitution, generally, but not necessarily, amino acid substitutions in the same class are combined to achieve the desired result. Various classes of amino acid substitutions are described in the following table.

**TABLE 1**

| **CLASSES OF Fc REGION VARIANTS** | | |
|---|---|---|
| Class | FcR binding property | Position of Fc region substitution(s) |
| 1A | reduced binding to all FcγR | 238, 265, 269, 270, 297*, 327, 329 |
| 1B | reduced binding to both FcγRII and FcγRIII | 239,294,295,303,338,373,376,416, 435 |
| 2 | improved binding to both FcγRII and FcγRIII | 256, 290, 312, 326, 330, 339, 378, 430 |
| 3 | improved binding to FcγRII and no effect on FcγRIII binding | 255, 258, 267, 276, 280, 283, 285, 286, 305, 307, 309, 315, 320, 331, 337, 398 |
| 4 | improved binding to FcγRII and reduced binding to FcγRIII | 268, 272, 301, 322, 340 |
| 5 | reduced binding to FcγRII and no effect on FcγRIII binding | 292, 324, 335, 414, 419, 43 8, 43 9 |
| 6 | reduced binding to FcγRII and improved binding to FcγRIII | 298, 333 |
| 7 | no effect on FcγRII binding and reduced binding to FcγRIII | 248, 249, 252, 254, 278, 289, 293, 296, 338, 382, 388, 389, 434, 437 |
| 8 | no effect on FcγRII binding and improved binding to FcγRIII | 334, 360 |

| | | |
|---|---|---|
| * deglycosylated version | | |

Aside from amino acid substitutions, the present invention contemplates other modifications of the parent Fc region amino acid sequence in order to generate an Fc region variant with altered effector function.

One may, for example, delete one or more amino acid residues of the Fc region in order to reduce binding to an FcR. Generally, one will delete one or more of the Fc region residues identified herein as effecting FcR binding in order to generate such an Fc region variant. Generally, no more than one to about ten Fc region residues will be deleted according to this embodiment of the invention. The Fc region herein comprising one or more amino acid deletions will preferably retain at least about 80%, and preferably at least about 90%, and most preferably at least about 95%, of the parent Fc region or of a native sequence human Fc region.

One may also make amino acid insertion Fc region variants, which variants have altered effector function. For example, one may introduce at least one amino acid residue (*e.g*. one to two amino acid residues and generally no more than ten residues) adjacent to one or more of the Fc region positions identified herein as impacting FcR binding. By "adjacent" is meant within one to two amino acid residues of a Fc region residue identified herein. Such Fc region variants may display enhanced or diminished FcR binding and/or ADCC activity. In order to generate such insertion variants, one may evaluate a co-crystal structure of a polypeptide comprising a binding region of an FcR (*e.g*. the extracellular domain of the FcR of interest) and the Fc region into which the amino acid residue(s) are to be inserted (see, for example, Deisenhofer, Biochemistry 20(9):2361-2370 (1981); and Burmeister et al., Nature 342:379-383, (1994)) in order to rationally design an Fc region variant with, *e.g*., improved FcR binding ability. Such insertion(s) will generally be made in an Fc region loop, but not in the secondary structure (*i.e*. in a β-strand) of the Fc region.

By introducing the appropriate amino acid sequence modifications in a parent Fc region, one can generate a variant Fc region which (a) mediates antibody-dependent cell-mediated cytotoxicity (ADCC) in the presence of human effector cells more effectively and/or (b) binds an Fc gamma receptor (FcγR) with better affinity than the parent polypeptide. Such Fc region variants will generally comprise at least one amino acid modification in the Fc region. Combining amino acid modifications is thought to be particularly desirable. For example, the variant Fc region may include two, three, four, five, etc substitutions therein, *e.g.* of the specific Fc region positions identified herein.

Preferably, the parent polypeptide Fc region is a human Fc region, *e.g.* a native sequence human Fc region human IgG1 (A and non-A allotypes), IgG2, IgG3 or IgG4 Fc region. Such sequences are shown in Fig. 23.

To generate an Fc region with improved ADCC activity, the parent polypeptide preferably has pre-existing ADCC activity, *e.g.*, it comprises a human IgG1 or human IgG3 Fc region. In one embodiment, the variant with improved ADCC mediates ADCC substantially more effectively than an antibody with a native sequence IgG1 or IgG3 Fc region and the antigen-binding region of the variant. Preferably, the variant comprises, or consists essentially of, substitutions of two or three of the residues at positions 298, 333 and 334 of the Fc region. Most preferably, residues at positions 298, 333 and 334 are substituted, (*e.g*. with alanine residues). Moreover, in order to generate the Fc region variant with improved ADCC activity, one will generally engineer an Fc region variant with improved binding affinity for FcγRIII, which is thought to be an important FcR for mediating ADCC. For example, one may introduce an amino acid modification (*e.g*. a substitution) into the parent Fc region at any one or more of amino acid positions 256, 290, 298, 312, 326, 330, 333, 334, 360, 378 or 430 to generate such a variant. The variant with improved binding affinity for FcγRIII may further have reduced binding affinity for FcγRII, especially reduced affinity for the inhibiting FcγRIIB receptor.

The amino acid modification(s) are preferably introduced into the CH2 domain of a Fc region, since the experiments herein indicate that the CH2 domain is important for FcR binding activity. Moreover, unlike the teachings of the above-cited art, the instant application contemplates the introduction of a modification into a part of the Fc region other than in the lower hinge region thereof.

Useful amino acid positions for modification in order to generate a variant IgG Fc region with altered Fc gamma receptor (FcγR) binding affinity or activity include any one or more of amino acid positions 23 8, 239, 248, 249, 252, 254, 255, 256, 258, 265, 267, 268, 269, 270, 272, 276,278,280,283,285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439 of the Fc region. Preferably, the parent Fc region used as the template to generate such variants comprises a human IgG Fc region. Where residue 331 is substituted, the parent Fc region is preferably not human native sequence IgG3, or the variant Fc region comprising a substitution at position 331 preferably displays increased FcR binding, *e.g.* to FcγRII.

To generate an Fc region variant with reduced binding to the FcγR one may introduce an amino acid modification at any one or more of amino acid positions 238, 239, 248, 249, 252, 254, 265, 268, 269, 270, 272, 278, 289, 292, 293, 294, 295, 296, 298, 301, 303, 322, 324, 327, 329, 333, 335, 338, 340, 373, 376,382, 388, 389, 414, 416, 419, 434, 435, 437, 438 or 439 of the Fc region.

Variants which display reduced binding to FcγRI, include those comprising an Fc region amino acid modification at any one or more of amino acid positions 23 8, 265, 269, 270, 327 or 329.

Variants which display reduced binding to FcγRII include those comprising an Fc region amino acid modification at any one or more of amino acid positions 238, 265, 269, 270, 292, 294, 295, 298, 303, 324, 327, 329, 333, 335, 338, 373, 376, 414, 416, 419, 435, 438 or 439.

Fc region variants which display reduced binding to FcγRIII include those comprising an Fc region amino acid modification at any one or more of amino acid positions 238, 239, 248, 249, 252, 254, 265, 268, 269, 270, 272, 278, 289, 293, 294, 295, 296, 301, 303, 322, 327, 329, 338, 340, 373, 376, 382, 388, 389, 416, 434, 435 or 437.

Variants with improved binding to one or more FcγRs may also be made. Such Fc region variants may comprise an amino acid modification at any one or more of amino acid positions 255, 256, 258, 267, 268, 272, 276, 280, 283, 285, 286, 290, 298, 301, 305, 307, 309, 312, 315, 320, 322, 326, 330, 331, 333, 334, 337, 340, 360, 378, 398 or 430 of the Fc region.

For example, the variant with improved FcγR binding activity may display increased binding to FcγRIII, and optionally may further display decreased binding to FcγRII; *e.g.* the variant may comprise an amino acid modification at position 298 and/or 333 of an Fc region.

Variants with increased binding to FcγRII include those comprising an amino acid modification at any one or more of amino acid positions 255, 256, 258, 267, 268, 272, 276, 280, 283, 285, 286, 290, 301, 305, 307, 309, 312, 315, 320, 322, 326, 330, 331, 337, 340, 378, 398 or 430 of an Fc region. Such variants may further display decreased binding to FcγRIII. For example, they may include an Fc region amino acid modification at any one or more of amino acid positions 268, 272, 298, 301, 322 or 340.

While it is preferred to alter binding to a FcγR, Fc region variants with altered binding affinity for the neonatal receptor (FcRn) are also contemplated herein. Fc region variants with improved affinity for FcRn are anticipated to have longer serum half-lives, and such molecules will have useful applications in methods of treating mammals where long half-life of the administered polypeptide is desired, *e.g*., to treat a chronic disease or disorder. Fc region variants with decreased FcRn binding affinity, on the contrary, are expected to have shorter half-lives, and such molecules may, for example, be administered to a mammal where a shortened circulation time may be advantageous, *e.g*. for *in vivo* diagnostic imaging or for polypeptides which have toxic side effects when left circulating in the blood stream for extended periods, etc. Fc region variants with decreased FcRn binding affinity are anticipated to be less likely to cross the placenta, and thus may be utilized in the treatment of diseases or disorders in pregnant women.

Fc region variants with altered binding affinity for FcRn include those comprising an Fc region amino acid modification at any one or more of amino acid positions 238, 252, 253, 254, 255, 256, 265, 272, 286, 288, 303, 305, 307, 309, 311, 312, 317; 340, 356, 360, 362, 376, 378, 380, 382, 386, 388, 400, 413, 415, 424, 433, 434, 435, 436, 439 or 447. Those which display reduced binding to FcRn will generally comprise an Fc region amino acid modification at any one or more of amino acid positions 252, 253, 254, 255, 288, 309, 386, 388, 400, 415, 433, 435, 436, 439 or 447; and those with increased binding to FcRn will usually comprise an Fc region amino acid modification at any one or more of amino acid positions 238, 256, 265, 272, 286, 303, 305, 307, 311, 312, 317, 340, 356, 360, 362, 376, 378, 380, 382, 413, 424 or 434.

The polypeptide variant(s) prepared as described above may be subjected to further modifications, oftentimes depending on the intended use of the polypeptide. Such modifications may involve further alteration of the amino acid sequence (substitution, insertion and/or deletion of amino acid residues), fusion to heterologous polypeptide(s) and/or covalent modifications. Such "further modifications" may be made prior to, simultaneously with, or following, the amino acid modification(s) disclosed above which result in an alteration of Fc receptor binding and/or ADCC activity. In one embodiment, one may combine the Fc region modification herein with Fc region substitutions disclosed in the references cited in the "Related Art" section of this application.

Alternatively or additionally, it may be useful to combine the above amino acid modifications with one or more further amino acid modifications that alter C1q binding and/or complement dependent cytoxicity function of the Fc region.

The starting polypeptide of particular interest herein is usually one that binds to C1q and displays complement dependent cytotoxicity (CDC). The further amino acid substitutions described herein will generally serve to alter the ability of the starting polypeptide to bind to C1q and/or modify its complement dependent cytotoxicity function, *e.g.* to reduce and preferably abolish these effector functions. However, polypeptides comprising substitutions at one or more of the described positions with improved C1q binding and/or complement dependent cytotoxicity (CDC) function are contemplated herein. For example, the starting polypeptide may be unable to bind C1q and/or mediate CDC and may be modified according to the teachings herein such that it acquires these further effector functions. Moreover, polypeptides with pre-existing C1q binding activity, optionally further having the ability to mediate CDC may be modified such that one or both of these activities are enhanced.

To generate an Fc region with altered C1q binding and/or complement dependent cytotoxicity (CDC) function, the amino acid positions to be modified are generally selected from heavy chain positions 270, 322, 326, 327, 329, 331, 333, and 334, where the numbering of the residues in an IgG heavy chain is that of the EU index as in Kabat et al., Sequences of Proteins of Immunological Interest, 5th Ed. Public Health Service, National Institutes of Health, Bethesda, MD (1991). In one embodiment, only one of the eight above-identified positions is altered in order to generate the polypeptide variant region with altered C1q binding and/or complement dependent cytotoxicity (CDC) function. Preferably only residue 270, 329 or 322 is altered if this is the case. Alternatively, two or more of the above-identified positions are modified. If substitutions are to be combined, generally substitutions which enhance human C1q binding (*e.g*. at residue positions 326, 327, 333 and 334) or those which diminish human C1q binding (*e.g*., at residue positions 270, 322, 329 and 331) are combined. In the latter embodiment, all four positions (*i.e*., 270, 322, 329 and 331) may be substituted. Preferably, further substitutions at two, three or all of positions 326, 327, 333 or 334 are combined, optionally with other Fc region substitutions, to generate a polypeptide with improved human C1q binding and preferably improved CDC activity *in vitro* or *in vivo*.

Proline is conserved at position 329 in human IgG's. This residue is preferably replaced with alanine, however substitution with any other amino acid is contemplated, *e.g*., serine, threonine, asparagine, glycine or valine.

Proline is conserved at position 331 in human IgG1, IgG2 and IgG3, but not IgG4 (which has a serine residue at position 331). Residue 331 is preferably replaced by alanine or another amino acid, *e.g.* serine (for IgG regions other than IgG4), glycine or valine.

Lysine 322 is conserved in human IgGs, and this residue is preferably replaced by an alanine residue, but substitution with any other amino acid residue is contemplated, *e.g*. serine, threonine, glycine or valine.

D270 is conserved in human IgGs, and this residue may be replaced by another amino acid residue, *e.g*. alanine, serine, threonine, glycine, valine, or lysine.

K326 is also conserved in human IgGs. This residue may be substituted with another residue including, but not limited to, valine, glutamic acid, alanine, glycine, aspartic acid, methionine or tryptophan, with tryptophan being preferred.

Likewise, E333 is also conserved in human IgGs. E333 is preferably replaced by an amino acid residue with a smaller side chain volume, such as valine, glycine, alanine or serine, with serine being preferred.

K334 is conserved in human IgGs and may be substituted with another residue such as alanine or other residue.

In human IgG1 and IgG3, residue 327 is an alanine. In order to generate a variant with improved C1q binding, this alanine may be substituted with another residue such as glycine. In IgG2 and IgG4, residue 327 is a glycine and this may be replaced by alanine (or another residue) to diminish C1q binding.

As disclosed above, one can design an Fc region with altered effector function, *e.g.*, by modifying C1q binding and/or FcR binding and thereby changing CDC activity and/or ADCC activity. For example, one can generate a variant Fc region with improved C1q binding and improved FcγRIII binding; *e.g.* having both improved ADCC activity and improved CDC activity. Alternatively, where one desires that effector function be reduced or ablated, one may engineer a variant Fc region with reduced CDC activity and/or reduced ADCC activity. In other embodiments, one may increase only one of these activities, and optionally also reduce the other activity, *e.g.* to generate an Fc region variant with improved ADCC activity, but reduced CDC activity *and vice versa.*

With respect to further amino acid sequence alterations, any cysteine residue not involved in maintaining the proper conformation of the polypeptide variant also may be substituted, generally with serine, to improve the oxidative stability of the molecule and prevent aberrant cross linking.

Another type of amino acid substitution serves to alter the glycosylation pattern of the polypeptide. This may be achieved by deleting one or more carbohydrate moieties found in the polypeptide, and/or adding one or more glycosylation sites that are not present in the polypeptide. Glycosylation of polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Addition of glycosylation sites to the polypeptide is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original polypeptide (for O-linked glycosylation sites). An exemplary glycosylation variant has an amino acid substitution of residue Asn 297 of the heavy chain.

Moreover, the class, subclass or allotype of the Fc region may be altered by one or more further amino acid substitutions to generate an Fc region with an amino acid sequence more homologous to a different class, subclass or allotype as desired. For example, a murine Fc region may be altered to generate an amino acid sequence more homologous to a human Fc region; a human non-A allotype IgG1 Fc region may be modified to achieve a human A allotype IgG1 Fc region etc. In one embodiment, the amino modification(s) herein which alter FcR binding and/or ADCC activity are made in the CH2 domain of the Fc region and the CH3 domain is deleted or replaced with another dimerization domain. Preferably, however, the CH3 domain is retained (aside from amino acid modifications therein which alter effector function as herein disclosed).

The glycoprotein prepared as described above may be subjected to further modifications, oftentimes depending on the intended use of the glycoprotein. Such modifications may involve further alteration of the amino acid sequence (substitution, insertion and/or deletion of amino acid residues), fusion to heterologous polypeptide(s) and/or covalent modifications.

Another type of amino acid substitution serves to alter the glycosylation pattern of the glycoprotein. Such glycosylation variations may be in addition to the glycosylation variation with respect to lack of fucose described herein and may be achieved by deleting one or more carbohydrate moieties found in the glycoprotein, and/or adding one or more glycosylation sites that are not present in the glycoprotein. Glycosylation of glycoproteins is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a glycoprotein creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Addition of glycosylation sites to the glycoprotein is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original glycoprotein (for O-linked glycosylation sites).

### B. Biological Activity Screening

The glycoprotein variant may be subjected to one or more assays to evaluate any change in biological activity compared to the starting polypeptide.

Preferably the glycoprotein variant essentially retains the ability to bind antigen compared to the nonvariant polypeptide, *i.e.* the binding capability is no worse than about 20 fold, *e.g*. no worse than about 5 fold of that of the nonvariant polypeptide. The binding capability of the polypeptide variant may be determined using techniques such as fluorescence activated cell sorting (FACS) analysis or radioimmunoprecipitation (RIA), for example.

The ability of the glycoprotein variant to bind an FcR may be evaluated. Where the FcR is a high affinity Fc receptor, such as FcγRI, FcRn, FcγRIIB or FcγRIIIA, binding can be measured by titrating monomeric glycoprotein variant and measuring bound glycoprotein variant using an antibody which specifically binds to the glycoprotein variant in a standard ELISA format (see Examples below). Another FcR binding assay for low affinity FcRs is described in WO00/42072 (Presta) and US Patent No. 6,242,195B1

To assess ADCC activity of the glycoprotein variant, an *in vitro* ADCC assay, may be performed using varying effector:target ratios. Useful "effector cells" for such assays include peripheral blood mononuclear cells (PBMC) and Natural Killer (NK) cells. Alternatively, or additionally, ADCC activity of the glycoprotein variant may be *assessed in vivo, e.g.,* in a animal model such as that disclosed in Clynes et al. PNAS (USA) 95:652-656 (1998).

The ability of the variant to bind C1q and mediate complement dependent cytotoxicity (CDC) may be assessed.

To determine C1q binding, a C1q binding ELISA may be performed. Briefly, assay plates may be coated overnight at 4°C with glycoprotein variant or starting polypeptide (control) in coating buffer. The plates may then be washed and blocked. Following washing, an aliquot of human C1q may be added to each well and incubated for 2 hrs at room temperature. Following a further wash, 100 µl of a sheep anti-complement C1q peroxidase conjugated antibody may be added to each well and incubated for 1 hour at room temperature. The plate may again be washed with wash buffer and 100 µl of substrate buffer containing OPD (O-phenylenediamine dihydrochloride (Sigma)) may be added to each well. The oxidation reaction, observed by the appearance of a yellow color, may be allowed to proceed for 30 minutes and stopped by the addition of 100 µl of 4.5 N H₂SO₄. The absorbance may then read at (492-405) nm.

An exemplary glycoprotein variant is one that displays a "significant reduction in C1q binding" in this assay. This means that about 100µg/ml of the glycoprotein variant displays about 50 fold or more reduction in C1q binding compared to 100µg/ml of a control antibody having a nonmutated IgG1 Fc region. In the most preferred embodiment, the glycoprotein variant "does not bind C1q", *i.e.* 100µg/ml of the glycoprotein variant displays about 100 fold or more reduction in C1q binding compared to 100µg/ml of the control antibody.

Another exemplary variant is one which "has a better binding affinity for human C1q than the parent polypeptide". Such a molecule may display, for example, about two-fold or more, and preferably about five-fold or more, improvement in human C1q binding compared to the parent polypeptide (*e.g*. at the IC₅₀ values for these two molecules). For example, human C1q binding may be about two-fold to about 500-fold, and preferably from about two-fold or from about five-fold to about 1000-fold improved compared to the parent polypeptide.

To assess complement activation, a complement dependent cytotoxicity (CDC) assay may be performed, *e.g.* as described in Gazzano-Santoro et al., J. Immunol. Methods 202:163 (1996). Briefly, various concentrations of the glycoprotein variant and human complement may be diluted with buffer. Cells which express the antigen to which the glycoprotein variant binds may be diluted to a density of ∼1 x 10⁶ cells /ml. Mixtures of glycoprotein variant, diluted human complement and cells expressing the antigen may be added to a flat bottom tissue culture 96 well plate and allowed to incubate for 2 hrs at 37°C and 5% CO₂ to facilitate complement mediated cell lysis. 50 µl of alamar blue (Accumed International) may then be added to each well and incubated overnight at 37°C. The absorbance is measured using a 96-well fluorometer with excitation at 530 nm and emission at 590 nm. The results may be expressed in relative fluorescence units (RFU). The sample concentrations may be computed from a standard curve and the percent activity as compared to nonvariant polypeptide is reported for the glycoprotein variant of interest.

Yet another exemplary variant "does not activate complement". For example, 0.6 µg/ml of the glycoprotein variant displays about 0-10% CDC activity in this assay compared to a 0.6 µg/ml of a control antibody having a nonmutated IgG1 Fc region. Preferably the variant does not appear to have any CDC activity in the above CDC assay.

The glycoprotein may be one which displays enhanced CDC compared to a parent polypeptide, *e.g.,* displaying about two-fold to about 100-fold improvement in CDC activity *in vitro* or *in vivo* (*e.g*. at the IC₅₀ values for each molecule being compared).

Fc region variants with altered binding affinity for the neonatal receptor (FcRn) are also contemplated herein. Fc region variants with improved affinity for FcRn are anticipated to have longer serum half-lives, and such molecules will have useful applications in methods of treating mammals where long half-life of the administered glycoprotein is desired, *e.g.*, to treat a chronic disease or disorder. Fc region variants with decreased FcRn binding affinity, on the contrary, are expected to have shorter half-lives, and such molecules may, for example, be administered to a mammal where a shortened circulation time may be advantageous, *e.g.* for *in vivo* diagnostic imaging or for polypeptides which have toxic side effects when left circulating in the blood stream for extended periods, etc. Fc region variants with decreased FcRn binding affinity are anticipated to be less likely to cross the placenta, and thus may be utilized in the treatment of diseases or disorders in pregnant women.

### C. Antibody Preparation

In the preferred embodiment of the invention, the glycoprotein which is modified according to the teachings herein is an antibody. Techniques for producing antibodies follow:

### (i) Antigen selection and preparation

Where the glycoprotein is an antibody, it is directed against an antigen of interest. Preferably, the antigen is a biologically important glycoprotein and administration of the antibody to a mammal suffering from a disease or disorder can result in a therapeutic benefit in that mammal. However, antibodies directed against nonpolypeptide antigens (such as tumor-associated glycolipid antigens; see US Patent 5,091,178) are also contemplated.

Where the antigen is a polypeptide, it may be a transmembrane molecule (*e.g*. receptor) or ligand such as a growth factor. Exemplary antigens include molecules such as renin; a growth hormone, including human growth hormone and bovine growth hormone; growth hormone releasing factor; parathyroid hormone; thyroid stimulating hormone; lipoproteins; alpha-1-antitrypsin; insulin A-chain; insulin B-chain; proinsulin; follicle stimulating hormone; calcitonin; luteinizing hormone; glucagon; clotting factors such as factor VIIIC, factor IX, tissue factor (TF), and von Willebrands factor; anti-clotting factors such as Protein C; atrial natriuretic factor; lung surfactant; a plasminogen activator, such as urokinase or human urine or tissue-type plasminogen activator (t-PA); bombesin; thrombin; hemopoietic growth factor; tumor necrosis factor-alpha and -beta; enkephalinase; RANTES (regulated on activation normally T-cell expressed and secreted); human macrophage inflammatory protein (MIP-1-alpha); a serum albumin such as human serum albumin; Muellerian-inhibiting substance; relaxin A-chain; relaxin B-chain; prorelaxin; mouse gonadotropin-associated peptide; a microbial protein, such as beta-lactamase; DNase; IgE; a cytotoxic T-lymphocyte associated antigen (CTLA), such as CTLA-4; inhibin; activin; vascular endothelial growth factor (VEGF); receptors for hormones or growth factors; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-β; platelet-derived growth factor (PDGF); fibroblast growth factor such as aFGF and bFGF; epidermal growth factor (EGF); transforming growth factor (TGF) such as TGF-alpha and TGF-beta, including TGF-β1, TGF-β2, TGF-β3, TGF-β4, or TGF-β5; a tumor necrosis factor (TNF) such as TNF-α or TNF-β; insulin-like growth factor-I and -II (IGF-I and IGF-II); des(1-3)-IGF-I (brain IGF-I), insulin-like growth factor binding proteins; CD proteins such as CD3, CD4, CD8, CD19, CD20, CD22 and CD40; erythropoietin; osteoinductive factors; immunotoxins; a bone morphogenetic protein (BMP); an interferon such as interferon-alpha, - beta, and -gamma; colony stimulating factors (CSFs), *e.g*., M-CSF, GM-CSF, and G-CSF; interleukins (ILs), *e.g*., IL-1, IL-2, IL-3, IL-4, IL-5, IL-6, IL-7, IL-8, IL-9 and IL-10; superoxide dismutase; T-cell receptors; surface membrane proteins; decay accelerating factor; viral antigen such as, for example, a portion of the AIDS envelope; transport proteins; homing receptors; addressins; regulatory proteins; integrins such as CD11a, CD11b, CD11c, CD18, an ICAM, VLA-4 and VCAM; a tumor associated antigen such as HER2, HER3 or HER4 receptor; and fragments of any of the above-listed polypeptides.

Exemplary molecular targets for antibodies encompassed by the present invention include CD proteins such as CD3, CD4, CD8, CD19, CD20, CD22, CD34 and CD40; members of the ErbB receptor family such as the EGF receptor, HER2, HER3 or HER4 receptor; prostate stem cell antigen (PSCA); cell adhesion molecules such as LFA-1, Mac1, p150.95, VLA-4, ICAM-1, VCAM, α4/β7 integrin, and αv/β3 integrin including either α or β subunits thereof (*e.g*. anti-CD11a, anti-CD18 or anti-CD11b antibodies); growth factors such as VEGF; tissue factor (TF); a tumor necrosis factor (TNF) such as TNF-α or TNF-β, alpha interferon (α-IFN); an interleukin, such as IL-8; IgE; blood group antigens; flk2/flt3 receptor; obesity (OB) receptor; *mpl* receptor; CTLA-4; protein C etc.

Soluble antigens or fragments thereof, optionally conjugated to other molecules, can be used as immunogens for generating antibodies. For transmembrane molecules, such as receptors, fragments of these (*e.g*. the extracellular domain of a receptor) can be used as the immunogen. Alternatively, cells expressing the transmembrane molecule can be used as the immunogen. Such cells can be derived from a natural source (*e.g*. cancer cell lines) or may be cells which have been transformed by recombinant techniques to express the transmembrane molecule. Other antigens and forms thereof useful for preparing antibodies will be apparent to those in the art.

### (ii) Polyclonal antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, *e.g.*, keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining, *e.g.*, 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### (iii) Monoclonal antibodies

Monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster or macaque monkey, is immunized as hereinabove described to elicit lymphocytes that produce or are capable of producing antibodies that will specifically bind to the protein used for immunization. Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown *in vivo* as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (*e.g*., by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as *E. coli* cells, simian COS cells, Chinese hamster ovary (CHO) cells, or myeloma cells that do not otherwise produce immunoglobulin protein, to obtain the synthesis of monoclonal antibodies in the recombinant host cells. Recombinant production of antibodies will be described in more detail below.

In a further embodiment, antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy- and light-chain constant domains in place of the homologous murine sequences (U.S. Patent No. 4,816,567; Morrison, et al., Proc. Natl Acad. Sci. USA, 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### (iv) Humanized and human antibodies

A humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987)). Another method uses a particular framework derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e*., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the CDR residues are directly and most substantially involved in influencing antigen binding.

Alternatively, it is now possible to produce transgenic animals (*e.g*., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e.g.*, Jakobovits et al., Proc. Natl. Acad. Sci. USA, 90:2551 (1993); Jakobovits et al., Nature, 362:255-258 (1993); Bruggermann et al., Year in Immuno., 7:33 (1993); and Duchosal et al. Nature 355:258 (1992). Human antibodies can also be derived from phage-display libraries (Hoogenboom et al., J. Mol. Biol., 227:381 (1991); Marks et al., J. Mol. Biol., 222:581-597 (1991); Vaughan et al. Nature Biotech 14:309 (1996)).

### (v) Multispecific antibodies

Multispecific antibodies have binding specificities for at least two different antigens. While such molecules normally will only bind two antigens (*i.e*. bispecific antibodies, BsAbs), antibodies with additional specificities such as trispecific antibodies are encompassed by this expression when used herein. Examples of BsAbs include those with one arm directed against a tumor cell antigen and the other arm directed against a cytotoxic trigger molecule such as anti-FcyRI/anti-CD15, anti-p185^{HER2}/FcγRIII (CD16), anti-CD3/anti-malignant B-cell (1D10), anti-CD3/anti-p185^{HER2}, anti-CD3/anti-p97, anti-CD3/anti-renal cell carcinoma, anti-CD3/anti-OVCAR-3, anti-CD3/L-D1 (anti-colon carcinoma), anti-CD3/anti-melanocyte stimulating hormone analog, anti-EGF receptor/anti-CD3, anti-CD3/anti-CAMA1, anti-CD3/anti-CD19, anti-CD3/MoV18, anti-neural cell adhesion molecule (NCAM)/anti-CD3, anti-folate binding protein (FBP)/anti-CD3, anti-pan carcinoma associated antigen (AMOC-31)/anti-CD3; BsAbs with one arm which binds specifically to a tumor antigen and one arm which binds to a toxin such as anti-saporin/anti-Id-1, anti-CD22/anti-saporin, anti-CD7/anti-saporin, anti-CD38/anti-saporin, anti-CEA/anti-ricin A chain, anti-interferon-α (IFN-α)/anti-hybridoma idiotype, anti-CEA/anti-vinca alkaloid; BsAbs for converting enzyme activated prodrugs such as anti-CD30/anti-alkaline phosphatase (which catalyzes conversion of mitomycin phosphate prodrug to mitomycin alcohol); BsAbs which can be used as fibrinolytic agents such as anti-fibrin/anti-tissue plasminogen activator (tPA), anti-fibrin/anti-urokinase-type plasminogen activator (uPA); BsAbs for targeting immune complexes to cell surface receptors such as anti-low density lipoprotein (LDL)/anti-Fc receptor (*e.g*. FcγRI, FcγRII or FcγRIII); BsAbs for use in therapy of infectious diseases such as anti-CD3/anti-herpes simplex virus (HSV), anti-T-cell receptor: CD3 complex/anti-influenza, anti-FcγR/anti-HIV; BsAbs for tumor detection *in vitro* or *in vivo* such as anti-CEA/anti-EOTUBE, anti-CEA/anti-DPTA, anti-p185^{HER2}/anti-hapten; BsAbs as vaccine adjuvants; and BsAbs as diagnostic tools such as anti-rabbit IgG/anti-ferritin, anti-horse radish peroxidase (HRP)/anti-hormone, anti-somatostatin/anti-substance P, anti-HRP/anti-FITC, anti-CEA/anti-β-galactosidase. Examples of trispecific antibodies include anti-CD3/anti-CD4/anti-CD37, anti-CD3/anti-CD5/anti-CD37 and anti-CD3/anti-CD8/anti-CD37. Bispecific antibodies can be prepared as full length antibodies or antibody fragments (*e.g*. F(ab')₂ bispecific antibodies). Bispecific antibodies are reviewed in Segal et al. J. Immunol. Methods 248:1-6 (2001).

Methods for making bispecific antibodies are known in the art. Traditional production of full length bispecific antibodies is based on the coexpression of two immunoglobulin heavy chain-light chain pairs, where the two chains have different specificities (Milstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBD J., 10:3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986). According to another approach described in W096/27011, the interface between a pair of antibody molecules can be engineered to maximize the percentage of heterodimers which are recovered from recombinant cell culture. The preferred interface comprises at least a part of the C_{H}3 domain of an antibody constant domain. In this method, one or more small amino acid side chains from the interface of the first antibody molecule are replaced with larger side chains (*e.g*. tyrosine or tryptophan). Compensatory "cavities" of identical or similar size to the large side chain(s) are created on the interface of the second antibody molecule by replacing large amino acid side chains with smaller ones (*e.g*. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/20373, and EP 03089). Heteroconjugate antibodies may be made using any convenient cross-linking methods. Suitable cross-linking agents are well known in the art, and are disclosed in US Patent No. 4,676,980, along with a number of cross-linking techniques.

Antibodies with more than two valencies are contemplated. For example, trispecific antibodies can be prepared. Tutt et al. J. Immunol. 147: 60 (1991).

### (vi) Multivalent antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (*e.g.* tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody will comprise an Fc region and three or more antigen binding sites amino-terminal to the Fc region. The preferred multivalent antibody herein comprises (or consists of) three to about eight, but preferably four, antigen binding sites. The multivalent antibody comprises at least one polypeptide chain (and preferably two polypeptide chains), wherein the polypeptide chain(s) comprise two or more variable domains. For instance, the polypeptide chain(s) may comprise VD1-(X1)ₙ-VD2-(X2)ₙ-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-vH-CH1-Fc region chain; or VH-CH1-VH-CH1-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain. Multivalent antibodies are described in WO 01/00238 and WO 00/44788.

### (vii) Affinity matured antibodies

The antibody herein may be an affinity matured antibody comprising substitution(s) of one or more hypervariable region residues of a parent antibody (*e.g*. a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (*e.g*. 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (*e.g*. binding affinity). In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and its antigen. Such contact residues and neighboring residues are candidates for substitution according to the techniques elaborated herein. Once such variants are generated, the panel of variants is subjected to screening, and antibodies with superior properties in one or more relevant assays may be selected for further development.

### (viii) Immunoconjugates

The invention also pertains to therapy with immunoconjugates comprising the glycoprotein conjugated to an anti-cancer agent such as a cytotoxic agent or a growth inhibitory agent.

Chemotherapeutic agents useful in the generation of such immunoconjugates have been described above.

Conjugates of an antibody and one or more small molecule toxins, such as a calicheamicin, maytansinoids, a trichothene, and CC1065, and the derivatives of these toxins that have toxin activity, are also contemplated herein.

In one preferred embodiment, the glycoprotein of the invention is conjugated to one or more maytansinoid molecules.

Glycoprotein-maytansinoid conjugates may be prepared by chemically linking the glycoprotein (*e.g*. an antibody) to a maytansinoid molecule without significantly diminishing the biological activity of either the glycoprotein or the maytansinoid molecule. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Patent No. 5,208,020. Preferred maytansinoids are maytansinol and maytansinol analogues modified in the aromatic ring or at other positions of the maytansinol molecule, such as various maytansinol esters. There are many linking groups known in the art for making antibody-maytansinoid conjugates, including, for example, those disclosed in U.S. Patent No. 5,208,020 or EP Patent 0 425 235 B1, and Chari et al. Cancer Research 52: 127-131 (1992). The linking groups include disufide groups, thioether groups, acid labile groups, photolabile groups, peptidase labile groups, or esterase labile groups, as disclosed in the above-identified patents, disulfide and thioether groups being preferred. Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particularly preferred coupling agents include N-succinimidyl-3-(2-pyridyldithio) propionate (SPDP) (Carlsson et al., Biochem. J. 173:723-737 (1978)) and N-succinimidyl-4-(2-pyridylthio)pentanoate (SPP) to provide for a disulfide linkage. The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hyrdoxymethyl, the C-15 position modified with a hydroxyl group, and the C-20 position having a hydroxyl group. In a preferred embodiment, the linkage is formed at the C-3 position of maytansinol or a maytansinol analogue.

Another immunoconjugate of interest comprises the glycoprotein conjugated to one or more calicheamicin molecules. The calicheamicin family of antibiotics are capable of producing double-stranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, γ₁^{I}, α₂^{I}, α₃^{I}, N-acetyl-γ₁^{I}, PSAG and θ^{I}₁ (Hinman et al. Cancer Research 53: 3336-3342 (1993), Lode et al. Cancer Research 58: 2925-2928 (1998) and the aforementioned U.S. patents to American Cyanamid). Another anti-tumor drug that the glycoprotein can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

Other antitumor agents that can be conjugated to the glycoproteins of the invention include BCNU, streptozoicin, vincristine and 5-fluorouracil, the family of agents known collectively LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates an immunoconjugate formed between the glycoprotein and a compound with nucleolytic activity (*e.g*. a ribonuclease or a DNA endonuclease such as a deoxyribonuclease; DNase).

For selective destruction of the tumor, the antibody may comprise a highly radioactive atom. A variety of radioactive isotopes are available for the production of radioconjugated antibodies. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the conjugate is used for diagnosis, it may comprise a radioactive atom for scintigraphic studies, for example tc^{99m} or I¹²³, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as tc^{99m} or I¹²³, .Re¹⁸⁶, Re¹⁸⁸ and In¹¹¹ can be attached via a cysteine residue in the peptide. Yttrium-90 can be attached via a lysine residue. The IODOGEN method (Fraker et al. Biochem. Biophys. Res. Commun. 80: 49-57 (1978) can be used to incorporate iodine-123. "Monoclonal Antibodies in Immunoscintigraphy" (Chatal,CRC Press 1989) describes other methods in detail.

Conjugates of the glycorprotein and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al. Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al. Cancer Research 52: 127-131 (1992); U.S. Patent No. 5,208,020) may be used.

Alternatively, a fusion protein comprising the glycoprotein and cytotoxic agent may be made, *e.g.* by recombinant techniques or peptide synthesis. The length of DNA may comprise respective regions encoding the two portions of the conjugate either adjacent one another or separated by a region encoding a linker peptide which does not destroy the desired properties of the conjugate.

In yet another embodiment, the antibody may be conjugated to a "receptor" (such streptavidin) for utilization in tumor pre-targeting wherein the antibody-receptor conjugate is administered to the patient, followed by removal of unbound conjugate from the circulation using a clearing agent and then administration of a "ligand" (*e.g*. avidin) which is conjugated to a cytotoxic agent (*e.g*. a radionucleotide).

### (ix) Antibody Dependent Enzyme Mediated Prodrug Therapy (ADEPT)

The antibodies of the present invention may also be used in ADEPT by conjugating the antibody to a prodrug-activating enzyme which converts a prodrug (*e.g*. a peptidyl chemotherapeutic agent, see WO81/01145) to an active anti-cancer drug. See, for example, WO 88/07378 and U.S. Patent No. 4,975,278.

The enzyme component of the immunoconjugate useful for ADEPT includes any enzyme capable of acting on a prodrug in such a way so as to covert it into its more active, cytotoxic form.

Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galactosidase and neuraminidase useful for converting glycosylated prodrugs into free drugs; β-lactamase useful for converting drugs derivatized with β-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs (see, *e.g*., Massey, Nature 328: 457-458 (1987)). Antibody-abzyme conjugates can be prepared as described herein for delivery of the abzyme to a tumor cell population.

The enzymes of this invention can be covalently bound to the antibodies by techniques well known in the art such as the use of the heterobifunctional crosslinking reagents discussed above. Alternatively, fusion proteins comprising at least the antigen binding region of an antibody of the invention linked to at least a functionally active portion of an enzyme of the invention can be constructed using recombinant DNA techniques well known in the art (see, *e.g*., Neuberger et al., Nature, 312: 604-608 (1984).

### (x) Other glycoprotein modifications

Other modifications of the glycoprotein are contemplated herein. For example, the glycoprotein may be linked to one of a variety of nonproteinaceous polymers, *e.g.*, polyethylene glycol, polypropylene glycol, polyoxyalkylenes, or copolymers of polyethylene glycol and polypropylene glycol. The antibody also may be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, 16th edition, Oslo, A., Ed., (1980).

The glycoproteins disclosed herein may also be formulated as immunoliposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA, 82:3688 (1985); Hwang et al., Proc. Natl Acad. Sci. USA, 77:4030 (1980); U.S. Pat. Nos. 4,485,045 and 4,544,545; and WO97/38731 published October 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al. J. Biol. Chem. 257: 286-288 (1982) via a disulfide interchange reaction. A chemotherapeutic agent is optionally contained within the liposome. See Gabizon et al. J. National Cancer Inst. 81(19)1484 (1989).

### (xi) Exemplary antibodies

Preferred antibodies within the scope of the present invention include those comprising the amino acid sequences of the following antibodies:
anti-HER2 antibodies including antibodies comprising the heavy and light chain variable regions of huMAb 4D5-8 (Carter et al., Proc. Natl. Acad. Sci. USA, 89:4285-4289 (1992), U.S. Patent No. 5,725,856);
anti-CD20 antibodies such as chimeric anti-CD20 "C2B8" as in US Patent No. 5,736,137 (RITUXAN®), a chimeric or humanized variant of the 2H7 antibody as in US Patent No. 5,721,108, B1 or Tositumomab (BEXXAR®);
anti-IL-8 (St John et al., Chest, 103:932 (1993), and International Publication No. WO 95/23865);
anti-VEGF antibodies including humanized and/or affinity matured anti-VEGF antibodies such as the humanized anti-VEGF antibody huA4.6.1 AVASTIN^{™} (Kim et al., Growth Factors, 7:53-64 (1992), International Publication No. WO 96/30046, and WO 98/45331, published October 15, 1998);
anti-PSCA antibodies (WO01/40309);
anti-CD40 antibodies, including S2C6 and humanized variants thereof (WO00/75348);
anti-CD11a (US Patent No. 5,622,700, WO 98/23761, Steppe et al., Transplant Intl. 4:3-7 (1991), and Hourmant et al., Transplantation 58:377-380 (1994));
anti-IgE (Presta et al., J. Immunol. 151:2623-2632 (1993), and International Publication No. WO 95/19181; US Patent No. 5,714,338, issued February 3, 1998 or US Patent No. 5,091,313, issued February 25,1992, WO 93/04173 published March 4,1993, or WO 99/01556 published January 14, 1999, US Patent No. 5,714,338);
anti-CD18 (US Patent No. 5,622,700, issued April 22, 1997, or as in WO 97/26912, published July 31, 1997);
anti-Apo-2 receptor antibody (WO 98/51793 published November 19, 1998);
anti-TNF-α antibodies including cA2 (REMICADE®), CDP571 and MAK-195 (See, US Patent No. 5,672,347 issued September 30, 1997, Lorenz et al. J. Immunol. 156(4):1646-1653 (1996), and Dhainaut et al. Crit. Care Med. 23(9):1461-1469 (1995));
anti-Tissue Factor (TF) (European Patent No. 0 420 937 B1 granted November 9, 1994);
anti-human α₄-β₇ integrin (WO 98/06248 published February 19, 1998);
anti-EGFR (chimerized or humanized 225 antibody as in WO 96/40210 published December 19, 1996);
anti-CD3 antibodies such as OKT3 (US Patent No. 4,515,893 issued May 7, 1985);
anti-CD25 or anti-tac antibodies such as CHI-621 (SIMULECT®) and (ZENAPAX®) (See US Patent No. 5,693,762 issued December 2, 1997);
anti-CD4 antibodies such as the cM-7412 antibody (Choy et al. Arthritis Rheum 39(1):52-56 (1996));
anti-CD52 antibodies such as CAMPATH-1H (Riechmann et al. Nature 332:323-337 (1988);
anti-Fc receptor antibodies such as the M22 antibody directed against FcγRI as in Graziano et al. J. Immunol. 155(10):4996-5002 (1995);
anti-carcinoembryonic antigen (CEA) antibodies such as hMN-14 (Sharkey et al. Cancer Res. 55(23Suppl): 5935s-5945s (1995);
antibodies directed against breast epithelial cells including huBrE-3, hu-Mc 3 and CHL6 (Ceriani et al. Cancer Res. 55(23): 5852s-5856s (1995); and Richman et al. Cancer Res. 55(23 Supp): 5916s-5920s (1995));
antibodies that bind to colon carcinoma cells such as C242 (Litton et al. Eur J. Immunol. 26(1):1-9 (1996));
anti-CD38 antibodies, e.g. AT 13/5 (Ellis et al. J. Immunzol. 155(2):925-937 (1995));
anti-CD33 antibodies such as Hu M195 (Jurcic et al. Cancer Res 55(23 Suppl):5908s-5910s (1995) and CMA-676 or CDP771;
anti-CD22 antibodies such as LL2 or LymphoCide (Juweid et al. Cancer Res 55(23 Suppl):5899s-5907s (1995);
anti-EpCAM antibodies such as 17-1A (PANOREX®);
anti-GpIIb/IIIa antibodies such as abciximab or c7E3 Fab (REOPRO®);
anti-RSV antibodies such as MEDI-493 (SYNAGIS®);
anti-CMV antibodies such as PROTOVIR®;
anti-HIV antibodies such as PRO542;
anti-hepatitis antibodies such as the anti-Hep B antibody OSTAVIR®;
anti-CA 125 antibody OvaRex;
anti-idiotypic GD3 epitope antibody BEC2;
anti-αvβ3 antibody VITAMIN®;
anti-human renal cell carcinoma antibody such as ch-G250; ING-1;
anti-human 17-1A antibody (3622W94);
anti-human colorectal tumor antibody (A33);
anti-human melanoma antibody R24 directed against GD3 ganglioside;
anti-human squamous-cell carcinoma (SF-25); and
anti-human leukocyte antigen (HLA) antibodies such as Smart ID10 and the anti-HLA DR antibody Oncolym (Lym-1).

While the glycoprotein of interest herein is preferably an antibody, other Fc region-containing glycoproteins which can be modified according to the methods described herein are contemplated. An example of such a molecule is an immunoadhesin.

### D. Immunoadhesin Preparation

The simplest and most straightforward immunoadhesin design combines the binding domain(s) of the adhesin (*e*.*g*. the extracellular domain (ECD) of a receptor) with the Fc region of an immunoglobulin heavy chain. Ordinarily, when preparing the immunoadhesins of the present invention, nucleic acid encoding the binding domain of the adhesin will be fused C-terminally to nucleic acid encoding the N-terminus of an immunoglobulin constant domain sequence, however N-terminal fusions are also possible.

Typically, in such fusions the encoded chimeric polypeptide will retain at least functionally active hinge, C_{H}2 and C_{H}3 domains of the constant region of an immunoglobulin heavy chain. Fusions are also made to the C-terminus of the Fc portion of a constant domain, or immediately N-terminal to the C_{H}1 of the heavy chain or the corresponding region of the light chain. The precise site at which the fusion is made is not critical; particular sites are well known and may be selected in order to optimize the biological activity, secretion, or binding characteristics of the immunoadhesin.

In a preferred embodiment, the adhesin sequence is fused to the N-terminus of the Fc region of immunoglobulin G₁ (IgG₁). It is possible to fuse the entire heavy chain constant region to the adhesin sequence. However, more preferably, a sequence beginning in the hinge region just upstream of the papain cleavage site which defines IgG Fc chemically (*i*.*e*. residue 216, taking the first residue of heavy chain constant region to be 114), or analogous sites of other immunoglobulins is used in the fusion. In a particularly preferred embodiment, the adhesin amino acid sequence is fused to (a) the hinge region and C_{H}2 and C_{H}3 or (b) the C_{H}1, hinge, C_{H}2 and C_{H}3 domains, of an IgG heavy chain.

For bispecific immunoadhesins, the immunoadhesins are assembled as multimers, and particularly as heterodimers or heterotetramers. Generally, these assembled immunoglobulins will have known unit structures. A basic four chain structural unit is the form in which IgG, IgD, and IgE exist. A four chain unit is repeated in the higher molecular weight immunoglobulins; IgM generally exists as a pentamer of four basic units held together by disulfide bonds. IgA globulin, and occasionally IgG globulin, may also exist in multimeric form in serum. In the case of multimer, each of the four units may be the same or different.

Various exemplary assembled immunoadhesins within the scope herein are schematically diagrammed below:
(a) AC_{L}-AC_{L};
(b) AC_{H}-(AC_{H}, AC_{L}-AC_{H}, AC_{L}-V_{H}C_{H}, or V_{L}C_{L}-AC_{H});
(c) AC_{L}-AC_{H}-(AC_{L}-AC_{H}, AC_{L}-V_{H}C_{H}, V_{L}C_{L}-AC_{H}, or V_{L}C_{L}-V_{H}C_{H})
(d) AC_{L}-V_{H}C_{H}-(AC_{H}, or AC_{L}-V_{H}C_{H}, or V_{L}C_{L}-AC_{H});
(e) V_{L}C_{L}-AC_{H}-(AC_{L}-V_{H}C_{H}, or V_{L}C_{L}-AC_{H}); and
(f) (A-Y)ₙ-(V_{L}C_{L}-V_{H}C_{H})2,
wherein each A represents identical or different adhesin amino acid sequences;
V_{L} is an immunoglobulin light chain variable domain;
V_{H} is an immunoglobulin heavy chain variable domain;
C_{L} is an immunoglobulin light chain constant domain;
C_{H} is an immunoglobulin heavy chain constant domain;
n is an integer greater than 1;
Y designates the residue of a covalent cross-linking agent.

In the interests of brevity, the foregoing structures only show key features; they do not indicate joining (J) or other domains of the immunoglobulins, nor are disulfide bonds shown. However, where such domains are required for binding activity, they shall be constructed to be present in the ordinary locations which they occupy in the immunoglobulin molecules.

Alternatively, the adhesin sequences can be inserted between immunoglobulin heavy chain and light chain sequences, such that an immunoglobulin comprising a chimeric heavy chain is obtained. In this embodiment, the adhesin sequences are fused to the 3' end of an immunoglobulin heavy chain in each arm of an immunoglobulin, either between the hinge and the C_{H}2 domain, or between the C_{H}2 and C_{H}3 domains. Similar constructs have been reported by Hoogenboom, et al., Mol. Immunol. 28:1027-1037 (1991).

Although the presence of an immunoglobulin light chain is not required in the immunoadhesins of the present invention, an immunoglobulin light chain might be present either covalently associated to an adhesin-immunoglobulin heavy chain fusion polypeptide, or directly fused to the adhesin. In the former case, DNA encoding an immunoglobulin light chain is typically coexpressed with the DNA encoding the adhesin-immunoglobulin heavy chain fusion protein. Upon secretion, the hybrid heavy chain and the light chain will be covalently associated to provide an immunoglobulin-like structure comprising two disulfide-linked immunoglobulin heavy chain-light chain pairs. Methods suitable for the preparation of such structures are, for example, disclosed in U.S. Patent No. 4,816,567, issued 28 March 1989.

Immunoadhesins are most conveniently constructed by fusing the cDNA sequence encoding the adhesin portion in-frame to an immunoglobulin cDNA sequence. However, fusion to genomic immunoglobulin fragments can also be used (see, *e*.*g*. Aruffo et al., Cell 61:1303-1313 (1990); and Stamenkovic et al., Cell 66:1133-1144 (1991)). The latter type of fusion requires the presence of Ig regulatory sequences for expression. cDNAs encoding IgG heavy-chain constant regions can be isolated based on published sequences from cDNA libraries derived from spleen or peripheral blood lymphocytes, by hybridization or by polymerase chain reaction (PCR) techniques. The cDNAs encoding the "adhesin" and the immunoglobulin parts of the immunoadhesin are inserted in tandem into a plasmid vector that directs efficient expression in the chosen host cells.

### E. Vectors, Host Cells and Recombinant Methods

The invention also provides isolated nucleic acid encoding a glycoprotein as disclosed herein, vectors and host cells comprising the nucleic acid, and recombinant techniques for the production of the glycoprotein.

For recombinant production of the glycoprotein, the nucleic acid encoding it is isolated and inserted into a replicable vector for further cloning (amplification of the DNA) or for expression. DNA encoding the glycoprotein is readily isolated and sequenced using conventional procedures (*e*.*g*., by using oligonucleotide probes that are capable of binding specifically to genes encoding the glycoprotein). Many vectors are available. The vector components generally include, but are not limited to, one or more of the following: a signal sequence, an origin of replication, one or more marker genes, an enhancer element, a promoter, and a transcription termination sequence.

### (i) Signal sequence component

The glycoprotein of this invention may be produced recombinantly not only directly, but also as a fusion polypeptide with a heterologous polypeptide, which is preferably a signal sequence or other polypeptide having a specific cleavage site at the N-terminus of the mature protein or polypeptide. The heterologous signal sequence selected preferably is one that is recognized and processed (*i*.*e*., cleaved by a signal peptidase) by the host cell. For prokaryotic host cells that do not recognize and process the native polypeptide signal sequence, the signal sequence is substituted by a prokaryotic signal sequence selected, for example, from the group of the alkaline phosphatase, penicillinase, lpp, or heat-stable enterotoxin II leaders. For yeast secretion the native signal sequence may be substituted by, *e*.*g*., the yeast invertase leader, α factor leader (including *Saccharomyces* and *Kluyveromyces* α-factor leaders), or acid phosphatase leader, the *C. albicans* glucoamylase leader, or the signal described in WO 90/13646. In mammalian cell expression, mammalian signal sequences as well as viral secretory leaders, for example, the herpes simplex gD signal, are available.

The DNA for such precursor region is ligated in reading frame to DNA encoding the polypeptide.

### (ii) Origin of replication component

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

### (iii) Selection gene component

Expression and cloning vectors may contain a selection gene, also termed a selectable marker. Typical selection genes encode proteins that (a) confer resistance to antibiotics or other toxins, *e*.*g*., ampicillin, neomycin, methotrexate, or tetracycline, (b) complement auxotrophic deficiencies, or (c) supply critical nutrients not available from complex media, *e*.*g*., the gene encoding D-alanine racemase for *Bacilli*.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the polypeptide nucleic acid, such as DHFR, thymidine kinase, metallothionein-I and -II, preferably primate metallothionein genes, adenosine deaminase, ornithine decarboxylase, *etc*.

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity.

Alternatively, host cells (particularly wild-type hosts that contain endogenous DHFR) transformed or co-transformed with DNA sequences encoding polypeptide, wild-type DHFR protein, and another selectable marker such as aminoglycoside 3'-phosphotransferase (APH) can be selected by cell growth in medium containing a selection agent for the selectable marker such as an aminoglycosidic antibiotic, *e*.*g*., kanamycin, neomycin, or G418. See U.S. Patent No. 4,965,199.

A suitable selection gene for use in yeast is the *trp*1 gene present in the yeast plasmid YRp7 (Stinchcomb et al., Nature, 282:39 (1979)). The *trp*1 gene provides a selection marker for a mutant strain of yeast lacking the ability to grow in tryptophan, for example, ATCC No. 44076 or PEP4-1. Jones, Genetics, 85:12 (1977). The presence of the *trp*1 lesion in the yeast host cell genome then provides an effective environment for detecting transformation by growth in the absence of tryptophan. Similarly, *Leu*2-deficient yeast strains (ATCC 20,622 or 38,626) are complemented by known plasmids bearing the *Leu*2 gene.

In addition, vectors derived from the 1.6 µm circular plasmid pKD1 can be used for transformation of *Kluyveromyces* yeasts. Alternatively, an expression system for large-scale production of recombinant calf chymosin was reported for *K. lactis*. Van den Berg, Bio/Technology, 8:135 (1990). Stable multi-copy expression vectors for secretion of mature recombinant human serum albumin by industrial strains of *Kluyveromyces* have also been disclosed. Fleer et al., Bio/Technology, 9:968-975 (1991).

### (iv) Promoter component

Expression and cloning vectors usually contain a promoter that is recognized by the host organism and is operably linked to the polypeptide nucleic acid. Promoters suitable for use with prokaryotic hosts include the *phoA* promoter, β-lactamase and lactose promoter systems, alkaline phosphatase, a tryptophan (trp) promoter system, and hybrid promoters such as the tac promoter. However, other known bacterial promoters are suitable. Promoters for use in bacterial systems also will contain a Shine-Dalgarno (S.D.) sequence operably linked to the DNA encoding the polypeptide.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Polypeptide transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, *e*.*g*., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Patent No. 4,419,446. A modification of this system is described in U.S. Patent No. 4,601,978. See also Reyes et al., Nature 297:598-601 (1982) on expression of human β-interferon cDNA in mouse cells under the control of a thymidine kinase promoter from herpes simplex virus. Alternatively, the rous sarcoma virus long terminal repeat can be used as the promoter.

### (v) Enhancer element component

Transcription of a DNA encoding the polypeptide of this invention by higher eukaryotes is often increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) on enhancing elements for activation of eukaryotic promoters. The enhancer may be spliced into the vector at a position 5' or 3' to the polypeptide-encoding sequence, but is preferably located at a site 5' from the promoter.

### (vi) Transcription termination component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding the polypeptide. One useful transcription termination component is the bovine growth hormone polyadenylation region. See WO94/11026 and the expression vector disclosed therein.

### (vii) Selection and transformation of host cells

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as *Escherichia, e.g., E. coli, Enterobacter, Erwinia, Klebsiella, Proteus, Salmonella, e.g., Salmonella typhimurium, Serratia, e.g., Serratia marcescans*, and *Shigella*, as well as *Bacilli* such as *B. subtilis* and *B. licheniformis* (*e.g., B. licheniformis* 41P disclosed in DD 266,710 published 12 April 1989), *Pseudomonas* such as *P. aeruginosa*, and *Streptomyces*. One preferred *E. coli* cloning host is *E. coli* 294 (ATCC 31,446), although other strains such as *E. coli* B, *E. coli* X1776 (ATCC 31,537), and *E. coli* W3110 (ATCC 27,325) are suitable. These examples are illustrative rather than limiting.

In addition to prokaryotes, eukaryotic microbes such as filamentous fungi or yeast are suitable cloning or expression hosts for polypeptide-encoding vectors. *Saccharomyces cerevisiae*, or common baker's yeast, is the most commonly used among lower eukaryotic host microorganisms. However, a number of other genera, species, and strains are commonly available and useful herein, such as *Schizosaccharomyces pombe; Kluyveromyces* hosts such as, *e*.*g*., *K. lactis, K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans*, and *K. marxianus; yarrowia* (EP 402,226); *Pichia pastoris* (EP 183,070); *Candida; Trichoderma reesia* (EP 244,234); *Neurospora crassa; Schwanniomyces* such as *Schwanniomyces occidentalis*; and filamentous fungi such as, *e.g., Neurospora, Penicillium, Tolypocladium*, and *Aspergillus* hosts such as *A. nidulans* and *A. niger*.

Suitable host cells for the expression of glycosylated polypeptide are derived from multicellular organisms. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bonzbyx mori* have been identified. A variety of viral strains for transfection are publicly available, *e*.*g*., the L-1 variant of *Autographa californica* NPV and the Bm-5 strain of *Bombyx mori* NPV, and such viruses may be used as the virus herein according to the present invention, particularly for transfection of *Spodoptera frugiperda* cells.

Plant cell cultures of cotton, corn, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; mouse myeloma cells, such as NSO (*e*.*g*. RCB0213, Bebbington et al., Rio/Technology 10:169 (1992)) and SP2/0 cells (*e*.*g*. SP2/0-Ag14 cells, ATCC CRL 1581); rat myeloma cells, such as YB2/0 cells (*e*.*g*. YB2/3HL.P2.G11.16Ag.20 cells, ATCC CRL 1662); and a human hepatoma line (Hep G2). Dihydrofolate reductase (DHFR) deficient CHO cells are a preferred cell line for practicing the invention, with CHO-K1, DUX-B11, CHO-DP12, CHO-DG44 (Urlaub et al., Somatic Cell and Molecular Genetics 12:555 (1986)), and Lec 13 being exemplary CHO host cell lines. DUX-B11 cells have been transfected with a pSVEHIGNeo carrying the cDNA for preproinsulin, thus generating the clone CHO-DP12. In the case of CHO-K1 (ATCC CRL 61), DUX-B11 (Simonsen et al. PNAS(USA) 80:2495-2499 (1983)), DG44 or CHO-DP12 host cells, these may be altered such that they are deficient in their ability to fucosylate proteins expressed therein.

The invention is also applicable to hybridoma cells. The term "hybridoma" refers to a hybrid cell line produced by the fusion of an immortal cell line of immunologic origin and an antibody producing cell. The term encompasses progeny of heterohybrid myeloma fusions, which are the result of a fusion with human cells and a murine myeloma cell line subsequently fused with a plasma cell, commonly known as a trioma cell line. Furthermore, the term is meant to include any immortalized hybrid cell line that produces antibodies such as, for example, quadromas (See, *e*.*g*., Milstein et al., Nature, 537:3053 (1983)). The hybrid cell lines can be of any species, including human and mouse.

In a most preferred embodiment the mammalian cell is a non-hybridoma mammalian cell, which has been transformed with exogenous isolated nucleic acid encoding the polypeptide of interest. By "exogenous nucleic acid" or "heterologous nucleic acid" is meant a nucleic acid sequence that is foreign to the cell, or homologous to the cell but in a position within the host cell nucleic acid in which the nucleic acid is ordinarily not found.

### (viii) Culturing the host cells

Host cells are transformed with the above-described expression or cloning vectors for polypeptide production and cultured in conventional nutrient media modified as appropriate for inducing promoters, selecting transformants, or amplifying the genes encoding the desired sequences.

The host cells used to produce the polypeptide of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enzym. 58:44 (1979), Baines et al., Anal. Biochem.102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN^{™} drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

All culture medium typically provides at least one component from one or more of the following categories:
1) an energy source, usually in the form of a carbohydrate such as glucose;
2) all essential amino acids, and usually the basic set of twenty amino acids plus cystine;
3) vitamins and/or other organic compounds required at low concentrations;
4) free fatty acids; and
5) trace elements, where trace elements are defined as inorganic compounds or naturally occurring elements that are typically required at very low concentrations, usually in the micromolar range.

The culture medium is preferably free of serum, e.g. less than about 5%, preferably less than 1%, more preferably 0 to 0.1 % serum, and other animal-derived proteins. However, they can be used if desired. In a preferred embodiment of the invention the cell culture medium comprises excess amino acids. The amino acids that are provided in excess may, for example, be selected from Asn, Asp, Gly, Ile, Leu, Lys, Met, Ser, Thr, Trp, Tyr and Val. Preferably, Asn, Asp, Lys, Met, Ser and Trp are provided in excess. For example, amino acids, vitamins, trace elements and other media components at one or two times the ranges specified in European Patent EP 307,247 or U.S. Patent No 6,180,401 may be used. These two documents are incorporated by reference herein.

For the culture of the mammalian cells expressing the desired protein and capable of adding the desired carbohydrates at specific positions, numerous culture conditions can be used paying particular attention to the host cell being cultured. Suitable culture conditions for mammalian cells are well known in the art (Cleveland et al., J. Immunol. Methods 56:221-234 (1983)) or can be easily determined by the skilled artisan (see, for example, Animal Cell Culture: A Practical Approach 2nd Ed., Rickwood, D. and Hames, B.D., eds. Oxford University Press, New York (1992)), and vary according to the particular host cell selected.

### (ix) Glycoprotein purification

When using recombinant techniques, the glycoprotein can be produced intracellularly, in the periplasmic space, or directly secreted into the medium. If the glycoprotein is produced intracellularly, as a first step, the particulate debris, either host cells or lysed fragments, is removed, for example, by centrifugation or ultrafiltration. Carter et al., Bio/Technology 10:163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the glycoprotein is secreted into the medium, supernatants from such expression systems are generally first concentrated using a commercially available protein concentration filter, for example, an Amicon or Millipore Pellicon ultrafiltration unit A protease inhibitor such as PMSF may be included in any of the foregoing steps to inhibit proteolysis and antibiotics may be included to prevent the growth of adventitious contaminants.

The glycoprotein composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc region that is present in the glycoprotein. Protein A can be used to purify glycoproteins that are based on human γ1, γ2, or y4 heavy chains (Lindmark et al., J. Immunol. Meth. 62:1-13 (1983)). Protein G is recommended for all mouse isotypes and for human γ3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the glycoprotein comprises a C_{H}3 domain, the Bakerbond ABX™ resin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE^{™} chromatography on an anion or cation exchange resin (such as a polyaspartic acid column), chromatofocusing, SDS-PAGE, and ammonium sulfate precipitation are also available depending on the glycoprotein to be recovered.

In one embodiment, the glycoprotein may be purified using adsorption onto a lectin substrate (*e*.*g*. a lectin affinity column) to remove fucose-containing glycoprotein from the preparation and thereby enrich for fucose-free glycoprotein.

### F. Analysis of the Glycoprotein

The complex carbohydrate portion of the glycoprotein produced by the processes of the present invention may be readily analyzed to determine that the glycosylation reaction described above is complete. The oligosaccharides are analyzed by conventional techniques of carbohydrate analysis. Thus, for example, techniques such as lectin blotting, well-known in the art, reveal proportions of terminal mannose or other sugars such as galactose.

Preferably, carbohydrates are analyzed by MALD1-TOF mass spectral analysis as in Example 1 below and Shields et al., J. Biol. Chem. 9(2):6591-6604 (2001).

Several methods are known in the art for glycosylation analysis and are useful in the context of the present invention. Such methods provide information regarding the identity and the composition of the oligosaccharide attached to the peptide. Methods for carbohydrate analysis useful in the present invention include but are not limited to lectin chromatography; HPAEC-PAD, which uses high pH anion exchange chromatography to separate oligosaccharides based on charge; NMR; Mass spectrometry; HPLC; GPC; monosaccharide compositional analysis; sequential enzymatic digestion.

Additionally, methods for releasing oligosaccharides are known. These methods include
1) enzymatic, *e*.*g*. using fucosidase such as α-L-fucosidase to remove fucose;
2) elimination using harsh alkaline environment to release mainly 0-linked structures; and
3) chemical methods using anhydrous hydrazine to release both N-and O-linked oligosaccharides.

Neutral and amino-sugars can be determined by high performance anion-exchange chromatography combined with pulsed amperometric detection (HPAE-PAD Carbohydrate System, Dionex Corp.). For instance, sugars can be released by hydrolysis in 20% (v/v) trifluoroacetic acid at 100 C for 6 h. Hydrolysates are then dried by lyophilization or with a

Speed-Vac (Savant Instruments). Residues are then dissolved in 1% sodium acetate trihydrate solution and analyzed on a HPLC-AS6 column as described by Anumula et al. Anal. Biochem. 195:269-280 (1991).

Sialic acid can be determined separately by the direct colorimetric method of Yao et al. Anal Biochem. 179:332-335 (1989)) in triplicate samples. In a preferred embodiment the thiobarbaturic acid (TBA) of Warren, L. J. Biol Chem 238:(8) (1959) is used.

Alternatively, immunoblot carbohydrate analysis may be performed. According to this procedure protein-bound carbohydrates are detected using a commercial glycan detection system (Boehringer) which is based on the oxidative immunoblot procedure described by Haselbeck and Hosel (Haselbeck et al. Glycoconjugate J., 7:63 (1990)).

Methods of analysis include those described for the analysis of antibody associated oligosaccharides and described in, for example Wormald et al., Biochem. 36:1370-1380 (1997); Sheeley et al. Anal. Biochem. 247: 102-110 (1997) and Cant et al., Cytotechnology 15:223-228 (1994) as well as the references cited therein.

### G. Pharmaceutical Formulations

Therapeutic formulations of the glycoprotein can be prepared by mixing the glycoprotein having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e*.*g*., Zn-protein complexes); and/or non-ionic surfactants such as TWEEN^{™}, PLURONICS^{™} or polyethylene glycol (PEG).

The formulation herein may also contain more than one active compound as necessary for the particular indication being treated, preferably those with complementary activities that do not adversely affect each other. For instance, the formulation may further comprise another antibody or a chemotherapeutic agent. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the glycoprotein, which matrices are in the form of shaped articles, *e*.*g*., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers ofL-glutamic acid and γ ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

The pharmaceutical composition may be lyphilized. Lyophilized antibody formulations are described in US Patent No. 6,267,958. Stable aqueous antibody formulations are described in US Patent No. 6,171,586B1.

### H. Non-Therapeutic Uses for the Glycoprotein

The glycoprotein of the invention may be used as an affinity purification agent. In this process, the glycoprotein is immobilized on a solid phase such a Sephadex resin or filter paper, using methods well known in the art. The immobilized glycoprotein is contacted with a sample containing the antigen to be purified, and thereafter the support is washed with a suitable solvent that will remove substantially all the material in the sample except the antigen to be purified, which is bound to the immobilized glycoprotein. Finally, the support is washed with another suitable solvent, such as glycine buffer, pH 5.0, that will release the antigen from the glycoprotein.

The glycoprotein may also be useful in diagnostic assays, *e*.*g*., for detecting expression of an antigen of interest in specific cells, tissues, or serum.

For diagnostic applications, the glycoprotein typically will be labeled with a detectable moiety. Numerous labels are available which can be generally grouped into the following categories:
(a) Radioisotopes, such as ³⁵S, ¹⁴C, ¹²⁵I, ³H, and ¹³¹I. The glycoprotein can be labeled with the radioisotope using the techniques described in Current Protocols in Immunology, Volumes 1 and 2, Coligen et al., Ed. Wiley-Interscience, New York, New York, Pubs. (1991) for example and radioactivity can be measured using scintillation counting.
(b) Fluorescent labels such as rare earth chelates (europium chelates) or fluorescein and its derivatives, rhodamine and its derivatives, dansyl, Lissamine, phycoerythrin and Texas Red are available. The fluorescent labels can be conjugated to the glycoprotein using the techniques disclosed in *Current Protocols in Immunology, supra*, for example. Fluorescence can be quantified using a fluorimeter.
(c) Various enzyme-substrate labels are available and U.S. Patent No. 4,275,149 provides a review of some of these. The enzyme generally catalyzes a chemical alteration of the chromogenic substrate that can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (*e*.*g*., firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (*e*.*g*., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al., Methods for the Preparation of Enzyme-Antibody Conjugates for use in Enzyme Immunoassay, in Methods in Enzym. (ed J. Langone & H. Van Vunakis), Academic press, New York, 73:147-166 (1981).

Examples of enzyme-substrate combinations include, for example:
(i) Horseradish peroxidase (HRPO) with hydrogen peroxidase as a substrate, wherein the hydrogen peroxidase oxidizes a dye precursor (*e*.*g*., orthophenylene diamine (OPD) or 3,3',5,5'-tetramethyl benzidine hydrochloride (TMB));
(ii) alkaline phosphatase (AP) with para-Nitrophenyl phosphate as chromogenic substrate; and
(iii) β-D-galactosidase (β-D-Gal) with a chromogenic substrate (*e*.*g*., p-nitrophenyl-β-D-galactosidase) or fluorogenic substrate 4-methylumbelliferyl-β-D-galactosidase.

Numerous other enzyme-substrate combinations are available to those skilled in the art. For a general review of these, see U.S. Patent Nos. 4,275,149 and 4,318,980.

Sometimes, the label is indirectly conjugated with the glycoprotein. The skilled artisan will be aware of various techniques for achieving this. For example, the glycoprotein can be conjugated with biotin and any of the three broad categories of labels mentioned above can be conjugated with avidin, or *vice versa*. Biotin binds selectively to avidin and thus, the label can be conjugated with the glycoprotein in this indirect manner. Alternatively, to achieve indirect conjugation of the label with the glycoprotein, the glycoprotein is conjugated with a small hapten (*e*.*g*., digoxin) and one of the different types of labels mentioned above is conjugated with an anti-hapten polypeptide (*e*.*g*., anti-digoxin antibody). Thus, indirect conjugation of the label with the glycoprotein can be achieved.

In another embodiment of the invention, the glycoprotein need not be labeled, and the presence thereof can be detected using a labeled antibody which binds to the glycoprotein.

The glycoprotein of the present invention may be employed in any known assay method, such as competitive binding assays, direct and indirect sandwich assays, and immunoprecipitation assays. Zola, Monoclonal Antibodies: A Manual of Techniques, pp.147-158 (CRC Press, Inc. 1987).

The glycoprotein may also be used for *in vivo* diagnostic assays. Generally, the glycoprotein is labeled with a radionuclide (such as ¹¹¹In, ⁹⁹Tc, ¹⁴C, ¹³¹I, ¹²⁵I, ³H, ³²p or ³⁵S) so that the antigen or cells expressing it can be localized using immunoscintiography.

### I. In Vivo Uses for the Glycoprotein

It is contemplated that the glycoprotein of the present invention may be used to treat a mammal *e*.*g*. a patient suffering from, or predisposed to, a disease or disorder who could benefit from administration of the glycoprotein. The conditions which can be treated with the glycoprotein are many and include cancer (*e*.*g*. where the glycoprotein binds a tumor associated antigen, a B-cell surface antigen such as CD20, an ErbB receptor such as the HER2 receptor, an angiogenic factor such as vascular endothelial growth factor (VEGF)); allergic conditions such as asthma (with an anti-IgE antibody); and LFA-1-mediated disorders (*e*.*g*. where the glycoprotein is an anti-LFA-1 or anti-ICAM-1 antibody) etc. In the case of the antibody which binds a B-cell surface marker such as CD20, the prefered indications are a B-cell malignancy (*e*.*g*. non-Hodgkin's lymphoma), an autoimmune disease, or for blocking an immune response to a foreign antigen (see WO01/03734).

Where the antibody binds an ErbB receptor, the disorder preferably is ErbB-expressing cancer, e.g. a benign or malignant tumor characterized by overexpression of the ErbB receptor. Such cancers include, but are not limited to, breast cancer, squamous cell cancer, small-cell lung cancer, non-small cell lung cancer, gastrointestinal cancer, pancreatic cancer, glioblastoma, cervical cancer, ovarian cancer, bladder cancer, hepatoma, colon cancer, colorectal cancer, endometrial carcinoma, salivary gland carcinoma, kidney cancer, liver cancer, prostate cancer, vulval cancer, thyroid cancer, hepatic carcinoma and various types of head and neck cancer.

According to the teachings herein, one may prepare a glycoprotein with a variant Fc region which has improved ADCC activity. Such molecules will find applications in the treatment of different disorders.

For example, the glycoprotein with improved ADCC activity may be employed in the treatment of diseases or disorders where destruction or elimination of tissue or foreign microorganisms is desired. For example, the glycoprotein may be used to treat cancer; autoimmune diseases, inflammatory disorders; infections (*e*.*g*. bacterial, viral, fungal or yeast infections); and other conditions (such as goiter) where removal of tissue is desired, etc.

The glycoprotein is administered by any suitable means, including parenteral, subcutaneous, intraperitoneal, intrapulmonary, and intranasal, and, if desired for local immunosuppressive treatment, intralesional administration. Parenteral infusions include intramuscular, intravenous, intraarterial, intraperitoneal, or subcutaneous administration. In addition, the glycoprotein is suitably administered by pulse infusion, particularly with declining doses of the glycoprotein. Preferably the dosing is given by injections, most preferably intravenous or subcutaneous injections, depending in part on whether the administration is brief or chronic.

For the prevention or treatment of disease, the appropriate dosage of glycoprotein will depend on the type of disease to be treated, the severity and course of the disease, whether the glycoprotein is administered for preventive or therapeutic purposes, previous therapy, the patient's clinical history and response to the glycoprotein, and the discretion of the attending physician. The glycoprotein is suitably administered to the patient at one time or over a series of treatments.

Depending on the type and severity of the disease, about 1 µg/kg to 15 mg/kg (*e*.*g*., 0.1-20mg/kg) of glycoprotein is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays. The Examples herein demonstrate that lower doses of the glycoprotein (*e*.*g*. fucose-free antibody) may be administered, compared to the fucose-containing glycoprotein.

The glycoprotein composition should be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the glycoprotein to be administered will be governed by such considerations, and is the minimum amount necessary to prevent, ameliorate, or treat a disease or disorder. The glycoprotein need not be, but is optionally formulated with one or more agents currently used to prevent or treat the disorder in question. The effective amount of such other agents depends on the amount of glycoprotein present in the formulation, the type of disorder or treatment, and other factors discussed above. These are generally used in the same dosages and with administration routes as used hereinbefore or about from 1 to 99% of the heretofore employed dosages.

Therapeutic antibody compositions generally are placed into a container having a sterile access port, for example, an intravenous solution bag or vial having a stopper pierceable by a hypodermic injection needle.

A cancer patient to be treated with an antibody as an antagonist as disclosed herein may also receive radiation therapy. Alternatively, or in addition, a chemotherapeutic agent may be administered to the patient. Preparation and dosing schedules for such chemotherapeutic agents may be used according to manufacturers' instructions or as determined empirically by the skilled practitioner. Preparation and dosing schedules for such chemotherapy are also describe in Chemotherapy Service Ed., M.C. Perry, William & Wilkins, Baltimore, MD (1992). The chemotherapeutic agent may precede, or follow administration of the antagonist or may be given simultaneously therewith. For cancer indications, it may be desirable to also administer additional antibodies against tumor associated antigens or against angiogenic factors, such as antibodies which bind to HER2 or vascular endothelial growth factor (VEGF). Alternatively, or in addition, one or more cytokines may be co-administered to the patient.

Glycoprotein compositions may be provided in an article of manufacture and kit containing materials useful for the treatment of cancer, for example. The article of manufacture comprises a container with a label. Suitable containers include, for example, bottles, vials, and test tubes. The containers may be formed from a variety of materials such as glass or plastic. The container holds a composition comprising the glycoprotein preparations described herein. The active agent in the composition is the particular glycoprotein. The label on the container indicates that the composition is used for the treatment or prevention of a particular disease or disorder, and may also indicate directions for *in vivo*, such as those described above.

The kit comprises the container described above and a second container comprising a buffer. It may further include other materials desirable from a commercial and user standpoint, including other buffers, diluents, filters, needles, syringes, and package inserts with instructions for use.

The invention will be more fully understood by reference to the following examples. They should not, however, be construed as limiting the scope of this invention.

### EXAMPLES

In order to evaluate the role of fucosylated oligosaccharide in IgG function, the Lec13 cell line (Ripka et al. Arch. Biochem. Biophys. 249:533-545 (1986)) was utilized to express human IgG1. This CHO cell line is deficient in its ability to add fucose, but provided IgG with oligosaccharide which was otherwise similar to that found in normal CHO cell lines and from human serum. The resultant IgG products were used to evaluate the effect of fucosylated carbohydrate on antibody effector functions, including binding to human FcγR, human C1q, human FcRn, and ADCC using human effector cells.

### EXAMPLE 1

### Binding to Human FcR

*cDNA Constructs for Stable Cell Lines:* The heavy and light chains of the humanized anti-HER2 antibody Hu4D5 (Carter et al., Proc. Natl. Acad. Sci. USA, 89:4285 (1992)), the humanized, affinity matured anti-IgE antibody E27 (U.S. Patent No. 6,172,213), and the chimeric anti-CD20 antibody C2B8 (U.S. Patent No. 5,736,137) were subcloned into a previously described mammalian cell expression vector (Lucas et al. Nucl. Acid Res. 24, 1774-1779 (1996)). Puromycin is used as a selective marker in DHFR(+) cells, such as the Lec13 cells, and the DHFR site was retained for methotrexate amplification of the stable cell line.

*Transfection and Culturing ofLecl3 and Wild-type CHO Cells:* The CHO cell line Pro-Lec13.6a (Lec13), was obtained from Professor Pamela Stanley of Albert Einstein College of Medicine of Yeshiva University. Parental lines are Pro- (proline auxotroph) and Gat- (glycine, adenosine, thymidine auxotroph). The CHO-DP12 cell line used for wild-type antibodies is a derivative of the CHO-K1 cell line (ATCC #CCL-61), which is dihydrofolate reductase deficient, and has a reduced requirement for insulin. Cell lines were transfected with cDNA using the Superfect method (Qiagen, Valencia, CA). Selection of the Lec13 cells expressing transfected antibodies was performed using puromycin dihydrochloride (Calbiochem, San Diego, CA) at 10 µg/ml in growth medium containing: MEM Alpha Medium with L-glutamine, ribonucleosides and deoxyribonucleosides (GIBCO-BRL, Gaithersburg, MD), supplemented with 10% inactivated FBS (GIBCO), 10 mM HEPES, and 1X penicillin/streptomycin (GIBCO). The CHO cells were similarly selected in growth medium containing Ham's F12 without GHT: Low Glucose DMEM without Glycine with NaHCO3 supplemented with 5% FBS (GIBCO), 10 mM HEPES, 2 mM L-glutamine, 1X GHT(glycine, hypoxanthine,thymidine), and 1X penicillin/streptomycin. Colonies formed within two to three weeks and were pooled for expansion and protein expression. The cell pools were seeded initially at 3 x 10⁶ cells/10 cm plate for small batch protein expression. The cells were converted to serum-free media once they grew to 90-95% confluency and after 3-5 days cell supernatants were collected and tested in an Fc IgG- and intact IgG-ELISA to estimate protein expression levels. Lec13 and CHO cells were seeded at approximately 8 x 10⁶ cells/15 cm plate one day prior to converting to PS24 production medium, supplemented with 10 mg/L recombinant human insulin and 1 mg/L trace elements.

*Protein Expression:* Lec13 cells and CHO cells remained in serum-free production medium for 3-5 days. Supernatants were collected and clarified by centrifugation in 150 ml conical tubes to remove cells and debris. The protease inhibitors PMSF and aprotinin (Sigma, St. Louis, MO) were added and the supernatants were concentrated 5-fold on stirred cells using MWCO30 filters (Amicon, Beverly, MA) prior to immediate purification using protein G chromatography (Amersham Pharmacia Biotech, Piscataway, NJ)). All proteins were buffer exchanged into phosphate-buffered saline (PBS) using Centripriep-30 concentrators (Amicon) and analyzed by SDS-polyacrylamide gel electrophoresis. Protein concentrations were determined using A280 and verified using amino acid composition analysis. On average, the Lecl3 cells generated 10 µg mAb per 15 cm plate; expression in control CHO cells for all antibodies was 4-5 times higher than in the Lec13 cells. Antibodies generated from CHO-DP12 grown on plates will be denoted as CHO-P.

CHO-DP12 cells were also grown in spinner flasks. Cells were seeded at 6 x 10⁵ cells/ml and grown at 37°C for two days. On the third day, the temperature was shifted to 33°C and the cells allowed to grow until viability dropped to 70% due to the pH dropping to ∼6.5. Antibodies derived from CHO-DP12 cells grown in spinner flasks will be denoted as CHO-S.

*Matrix-Assisted Laser Desorption*/*Ionization Time-of-flight (MALDI-TOF) Mass Spectral Analysis of Asparagine-Linked Oligosaccharides:* N-linked oligosaccharides were released from recombinant glycoproteins using the procedure of Papac et al., Glycobiology 8, 445-454 (1998). Briefly, the wells of a 96 well PVDF-lined microtitre plate (Millipore, Bedford, MA) were conditioned with 100 µl methanol that was drawn through the PDVF membranes by applying vacuum to the Millipore Multiscreen vacuum manifold. The conditioned PVDF membranes were washed with 3 X 250 µl water. Between all wash steps the wells were drained completely by applying gentle vacuum to the manifold. The membranes were washed with reduction and carboxymethylation buffer (RCM) consisting of 6 M guanidine hydrochloride, 360 mM Tris, 2 mM EDTA, pH 8.6. Glycoprotein samples (50 µg) were applied to individual wells, again drawn through the PVDF membranes by gentle vacuum and the wells were washed with 2 X 50 µl of RCM buffer. The immobilized samples were reduced by adding 50 µl of a 0.1 M dithiothreitol (DTT) solution to each well and incubating the microtitre plate at 37°C for 1 hr. DTT was removed by vacuum and the wells were washed 4 x 250 µl water. Cysteine residues were carboxylmethylated by the addition of 50 µl of a 0.1 M iodoacetic acid (IAA) solution which was freshly prepared in 1 M NaOH and diluted to 0.1 M with RCM buffer. Carboxymethylation was accomplished by incubation for 30 min in the dark at ambient temperature. Vacuum was applied to the plate to remove the IAA solution and the wells were washed with 4 x 250 µl purified water. The PVDF membranes were blocked by the addition of 100 µl of 1% PVP360 (polyvinylpyrrolidine 360,000 MW) (Sigma) solution and incubation for 1 hr at ambient temperature. The PVP-360 solution was removed by gentle vacuum and the wells were washed 4 x 250 µl water. The PNGase F (New England Biolabs, Beverly, MA) digest solution, 25 µl of a 25 Unit/ml solution in 10 mM Tris acetate, pH 8.4, was added to each well and the digest proceeded for 3 hr at 37°C. After digestion, the samples were transferred to 500 µl Eppendorf tubes and 2.5 µlL of a 1.5 M acetic acid solution was added to each sample: The acidified samples were incubated for 3 hr at ambient temperature to convert the oligosaccharides from glycosylamines to the hydroxyl form. Prior to MALDI-TOF mass spectral analysis, the released oligosaccharides were desalted using a 0.7-ml bed of cation exchange resin (AG50W-X8 resin in the hydrogen form) (Bio-Rad, Hercules, CA) slurried packed into compact reaction tubes (US Biochemical, Cleveland, OH).

For MALDI-TOF mass spectral analysis of the samples in the positive mode, the desalted oligosaccharides (0.5 µl aliquots) were applied to the stainless target with 0.5 µl of the 2,5 dihydroxybenzoic acid matrix (sDHB) that was prepared by dissolving 2 mg 2,5 dihydroxybenzoic acid with 0.1 mg of 5-methoxyslicylic acid in 1 ml of ethanol/10 mM sodium chloride 1:1 (v/v). The sample/matrix mixture was dried by vacuum. For analysis in the negative mode, the desalted N-linked oligosaccharides (0.5 µl aliquots) were applied to the stainless target along with 0.5 µl 2',4',6'-trihydroxyacetophenone matrix (THAP) prepared in 1:3 (v/v) acetonitrile/13.3 mM ammonium citrate buffer. The sample/matrix mixture was vacuum dried and then allowed to absorb atmospheric moisture prior to analysis. Released oligosaccharides were analyzed by MALDI-TOF on a PerSeptive BioSystems Voyager-DE mass spectrometer. The mass spectrometer was operated at 20 kV either in the positive or negative mode with the linear configuration and utilizing delayed extraction. Data was acquired using a laser power of 1300 and in the data summation mode (240 scans) to improve the signal to noise. The instrument was calibrated with a mixture of standard oligosaccharides and the data was smoothed using a 19 point Savitsky-Golay algorithm before the masses were assigned. Integration of the mass spectral data was achieved using Caesar 7.0 data analysis software package (SciBridge Software). The results are summarized in the following table.

| Table 2. Binding of Antibodies to Human FcγR | | | | | | |
|---|---|---|---|---|---|---|
| | Mean(S.D.) | N | %-Fuc^{c} | %Gal0 | %Gal1 | %Gal2 |
| FcγRI^{a,b} | | | | | | |
| CHO-S | 1.00 | 5 | 3 | 53 | 42 | 6 |
| CHO-P | 0.97(0.07) | 5 | 2 | 73 | 25 | 3 |
| Lec13(A) | 1.04(0.07) | 4 | 92 | 50 | 43 | 7 |
| Lec13(B) | 1.04(0.10) | 5 | 91 | 55 | 40 | 5 |
| | | | | | | |
| FcγRIIA(R131)^{c} | | | | | | |
| CHO-S | 1.00 | 3 | 3 | 53 | 42 | 6 |
| CHO-P | 0.87(0.14) | 2 | 2 | 73 | 25 | 3 |
| Lec13(A) | 1.70(0.04) | 3 | 92 | 50 | 43 | 7 |
| Lec13(B) | 1.49(0.16) | 3 | 91 | 55 | 40 | 5 |
| Lec13(C) | 1.77(0.38) | 3 | 93 | 51 | 43 | 7 |
| Lec13(D) | 1.71 (0.40) | 3 | 88 | 51 | 43 | 7 |
| Lec13-Avg | 1.62(0.32) | 12 | 91(2) | 52(2) | 42(2) | 7(1) |
| | | | | | | |
| FcγRIIA(H131)^{d} | | | | | | |
| CHO-S | 1.00 | 3 | 3 | 53 | 42 | 6 |
| CHO-P | 0.87(0.07) | 2 | 2 | 73 | 25 | 3 |
| Lec13(A) | 0.93(0.08) | 3 | 92 | 50 | 43 | 7 |
| Lec13(B) | 0.75(0.07) | 3 | 91 | 55 | 40 | 5 |
| Lec13(C) | 0.94(0.15) | 3 | 93 | 51 | 43 | 7 |
| Lec13(D) | 0.91(0.07) | 3 | 88 | 51 | 43 | 7 |
| Lec13-Avg | 0.88(0.12) | 12 | 91(2) | 52(2) | 42(2) | 7(1) |
| | | | | | | |
| FcγRIIB^{c} | | | | | | |
| CHO-S | 1.00 | 3 | 3 | 53 | 42 | 6 |
| CHO-P | 0.81(0.11) | 2 | 2 | 73 | 25 | 3 |
| Lec13(A) | 2.27(0.35) | 3 | 92 | 50 | 43 | 7 |
| Lec13(B) | 1.51(0.22) | 3 | 91 | 55 | 40 | 5 |
| Lec13(C) | 2.07(0.33) | 2 | 93 | 51 | 43 | 7 |
| Lec13(D) | 1.60(0.45) | 3 | 88 | 51 | 43 | 7 |
| Lec13-Avg | 1.81(0.49) | 12 | 91(2) | 52(2) | 42(2) | 7(1) |
| | | | | | | |
| FcγRIIIA (F158)^{e} | | | | | | |
| CHO-S | 1.00 | 3 | 3 | 53 | 42 | 6 |
| CHO-P | 0.94(0.01) | 2 | 2 | 73 | 25 | 3 |
| Lec13(A) | 27.0(2.1) | 3 | 92 | 50 | 43 | 7 |
| Lec13(B) | 22.8(2.3) | 3 | 91 | 55 | 40 | 5 |
| Lec13(C) | 25.1(2.4) | 3 | 93 | 51 | 43 | 7 |
| Lec13(D) | 22.3(1.0) | 3 | 88 | 51 | 43 | 7 |
| Lec13-Avg | 24.3(2.6) | 12 | 91(2) | 52(2) | 42(2) | 7(1) |
| HEK293-AAA | 20.8(0.9) | 2 | | | | |
| Lec13-AAA(A) | 32.8 | 1 | 95 | 75 | 22 | 2 |
| Lecl3-AAA(B) | 32.9(2.9) | 3 | 92 | 75 | 22 | 3 |
| Lecl3-AAA(C) | 34.8(3.0) | 2 | | | | |
| Lecl3-AAA-Avg | 33.5(2.1) | 6 | | | | |
| | | | | | | |
| FcγRIIIA(F158)^{f} | | | | | | |
| HEK293 | 1.00 | 2 | | | | |
| DP12 | 0.35(0.01) | 2 | | | | |
| Lec13 | 7.63(0.20) | 2 | | | | |
| | | | | | | |
| FcγRIIIA(F1.58)^{g} | | | | | | |
| HEK293 | 1.00 | 3 | | | | |
| CHO-P | 0.65(0.24) | 3 | | | | |
| Lecl3 | 1.92(0.39) | 3 | | | | |
| HEK293-AAA | 1.87(0.24) | 3 | | | | |
| | | | | | | |
| FcγRIIIA(F158)-transfected CHO cells^{h} | | | | | | |
| CHO-S | 1.00 | | | 4 | | |
| Lecl3-D | 15.7 | 2.4 | | 4 | | |
| Lecl3-E | 17.0 | 3.1 | | 3 | | |
| Lecl3-F | 15.8 | 3.2 | | 3 | | |
| Lecl3-Avg | 16.1 | 2.5 | | 10 | | |
| HEK293-AAA | 10.7 | 1.4 | | 3 | | |
| Lecl3-AAA-B | 26.8 | 6.6 | | 3 | | |
| Lecl3-AAA-C | 25.9 | 5.9 | | 3 | | |
| Lecl3-AAA-Avg | 26.4 | 5.6 | | 6 | | |
| | | | | | | |
| FcγRIIIA(V158)^{e} | | | | | | |
| CHO | 1.00 | 3 | 3 | 53 | 42 | 6 |
| DP12 | 0.61(0.01) | 2 | 2 | 73 | 25 | 3 |
| Lecl3(A) | 14.9(2.9) | 3 | 92 | 50 | 43 | 7 |
| Lec13(B) | 12.5(1.3) | 3 | 91 | 55 | 40 | 5 |
| Lecl3(C) | 12.6(3.3) | 3 | 93 | 51 | 43 | 7 |
| Lecl3(D) | 14.5(1.9) | 3 | 88 | 51 | 43 | 7 |
| Lecl3-Avg | 13.6(2.4) | 12 | 91(2) | 52(2) | 42(2) | 7(1) |
| HEK293-AAA | 9.3 | 1 | | | | |
| Lecl3-AAA(A) | 25.4 | 1 | 95 | 75 | 22 | 2 |
| Lec13-AAA(B) | 23.8(1.1) | 3 | 92 | 75 | 22 | 3 |
| Lec13-AAA(C) | 22.5(0.2) | 2 | | | | |
| Lec13-AAA-Avg | 23.1(1.4) | 6 | | | | |
| | | | | | | |
| FcγRIIIA(V158)^{f} | | | | | | |
| HEK293 | 1.00 | 2 | | | | |
| CHO-P | 0.32(0.01) | 2 | | | | |
| Lec13 | 6.44(0.19) | 2 | | | | |
| | | | | | | |
| FcγRIIIA(V158)^{g} | | | | | | |
| HEK293 | 1.00 | 3 | | | | |
| CHO-P | 1.00(0.13) | 3 | | | | |
| Lecl3 | 1.18(0.10) | 3 | | | | |
| HEK293-AAA | 1.15(0.05) | 3 | | | | |

| | | | | | | |
|---|---|---|---|---|---|---|
| a All values are the ratio of A(variant)/A(standard) measured at A₄₉₀ₙₘ. CHO-S represents IgG expressed by CHO cells in spinner flasks, CHO-P represents IgG expressed by CHO cells on 15 cm plates, Lecl3 represents IgG expressed in Lec 13 cells on plates, HEK293 represents IgG expressed by human embryonic kidney 293 cells, AAA represents Ser298Ala/Glu333Ala/Lys334Ala IgG1 variant, Lecl3-S represents IgG expressed in Lecl3 cells in a spinner flask (instead of plates). Letters in parentheses indicate independently expressed lots of IgG. b Hu4D5 dimers at [mAb]=0.12 µg/ml. c %-Fuc is percent of total oligosaccharide without fucose, %Gal0, %Gal1, %Gal2 are percent of total oligosaccharide with none (agalactosyl), one (monogalactosyl), or two (digalactosyl) galactose residues covalently linked to the terminal mannose residues. Values in parentheses are deviations from mean for the four independently expressed lots of Lecl3-Hu4D5. d Hu4D5 dimers at [mAb]=3.33 µg/ml e Hu4D5 dimers at [mAb]=1.11 µg/ml f Hu4D5 dimers at [mAb] =0.12 µg/ml g E27 dimers at [mAb]=0.12 µg/ml h E27 hexamers at [mAb]=0.12 µg/ml | | | | | | |

Assays for measuring binding of IgG1 to FcγR and FcRn (ELISA-format and cell-based) have been described previously (Shields et al. J. Biol. Chem. 276:6591-6604 (2001) and WO00/42072 (Presta).

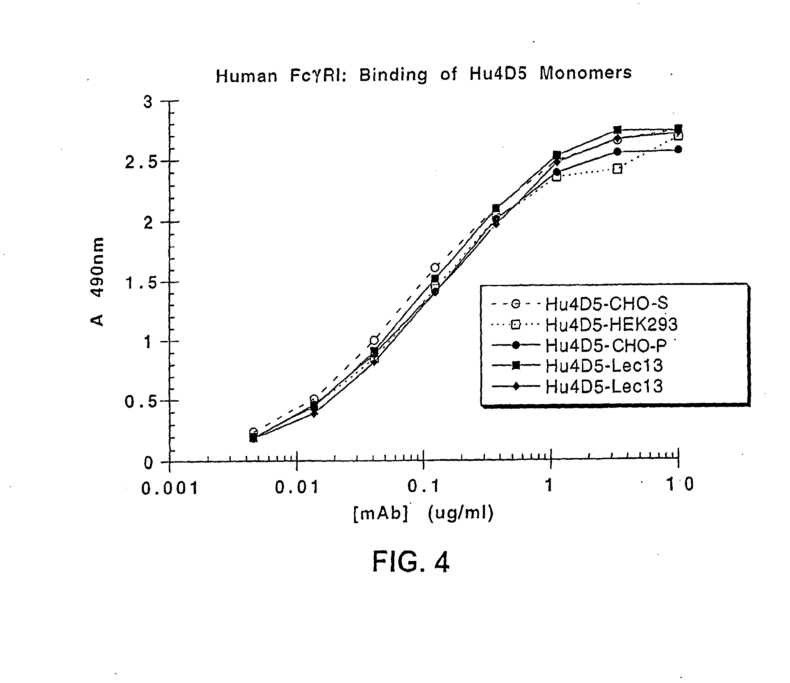

Monomeric Lec13-Hu4D5 IgGI bound to human FcγRI equivalent to binding of Hu4D5-CHO-S and CHO-P (Fig. 4; Table 2). Though the presence of carbohydrate is necessary for binding to FcγRI (Walker et al. Biochem. J. 259: 347-353 (1989)), the equivalent binding of IgGI regardless of differences in fucose content (Lec13 versus CHO) or galactose content (CHO-P versus CHO-S) shows that human FcγRI is not sensitive to the presence of these moieties on the carbohydrate. The effect of galactosylation on binding of IgG to human FcγRI has been previously studied (Wright et al. J. Immunol. 160: 3393-3402 (1998); Kumpel et al. Human Antibod. Hybridomas 5: 143-151 (1994); and Tsuchiya et al. J. Rheumatol. 16: 285-290 (1989)) and review of the data suggests that if galactosylation effects binding to FcγRI, it is subtle and may be isotype-dependent (Wright et al. J. Immunol. 160: 3393-3402 (1998)).

In contrast to the monomeric binding of IgGI to human FcγRI, the binding assays for the low-affinity human FcγR (FcγRII, FcγRIII) required formation of dimers (Hu4D5, HuE27) or hexamers (HuE27) to elicit detection of binding. Human FcγRIIA has two known, naturally occuring allotypes which are determined by the amino acid at position 131 (Clark et al. J. Immunol. 143: 1731-1734 (1989)). Human FcγRIIIA has naturally occurring allotypes at position 48 (Leu, His or Arg) and at position 158 (Val or Phe); the FcγRIIIA(Val158) allotype interacts with human IgG better than the FcγRIIIA(Phe158) allotype (Shields et al. J. Biol. Chem. 276: 6591-6604 (2001); Koene et al. Blood 90:1109-1114 (1997); and Wu et al. J. Clin Invest. 100: 1059-1070 (1997)).

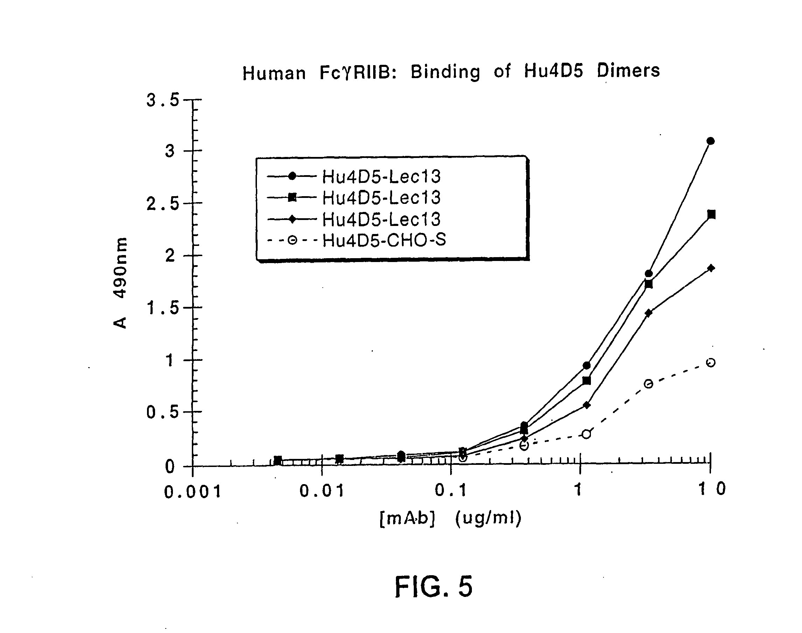

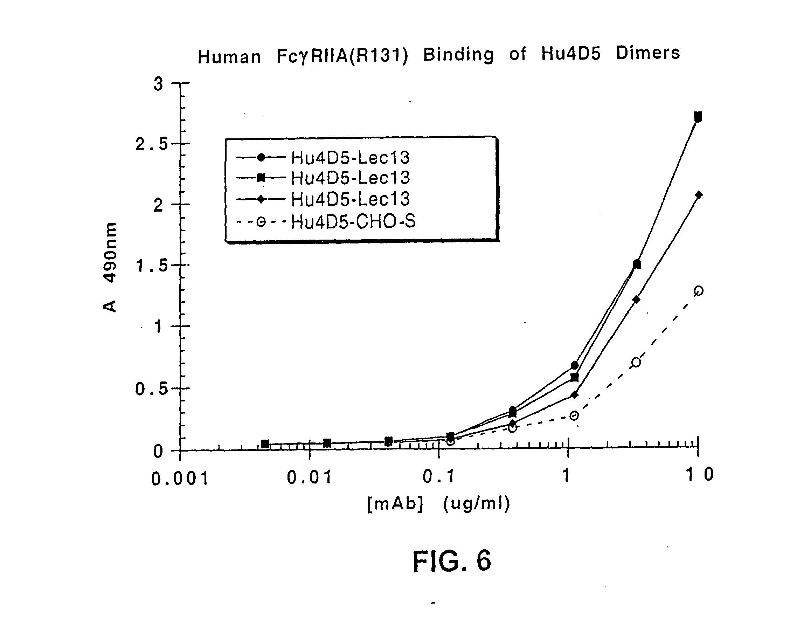

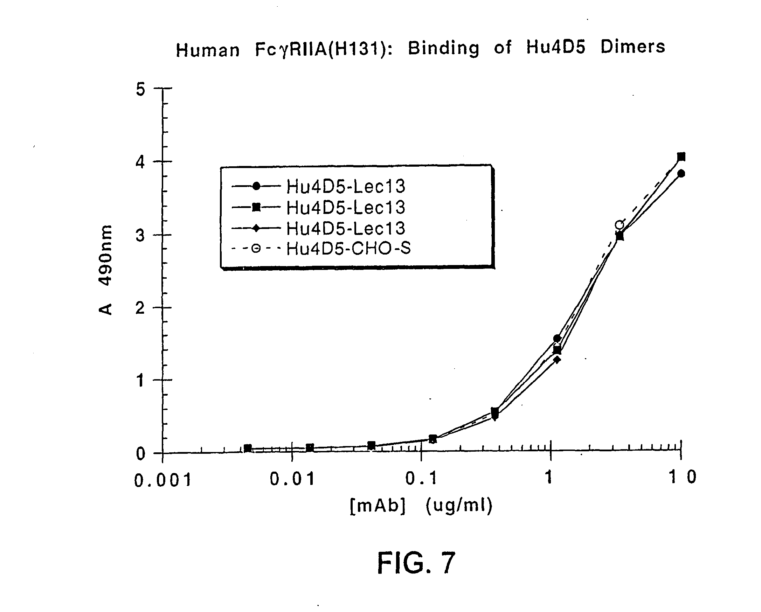

Binding of Lecl3-Hu4D5 dimers to human FcγRIIB and the human R131-polymorphic form of FcγRIIA showed 1.8-fold and 1.6-fold improvement in binding, respectively, compared to CHO-Hu4D5 (Fig. 5, 6; Table 2). In contrast, lack of fucose did not affect binding to the H131-polymorphic form of human FcγRIIA (Fig. 7; Table 2). The similar improvement in binding of IgG1 without fucose to both human FcγRIIA(R131) and FcγRIIB, each having arginine at position 131, versus no effect on FcγRIIA(H131) suggests that the fucose may either directly interact with the FcγR residue at position 131 or alter the IgG1 conformation so as to effect a subtle, negative influence on binding when arginine is present at FcγR position 131.

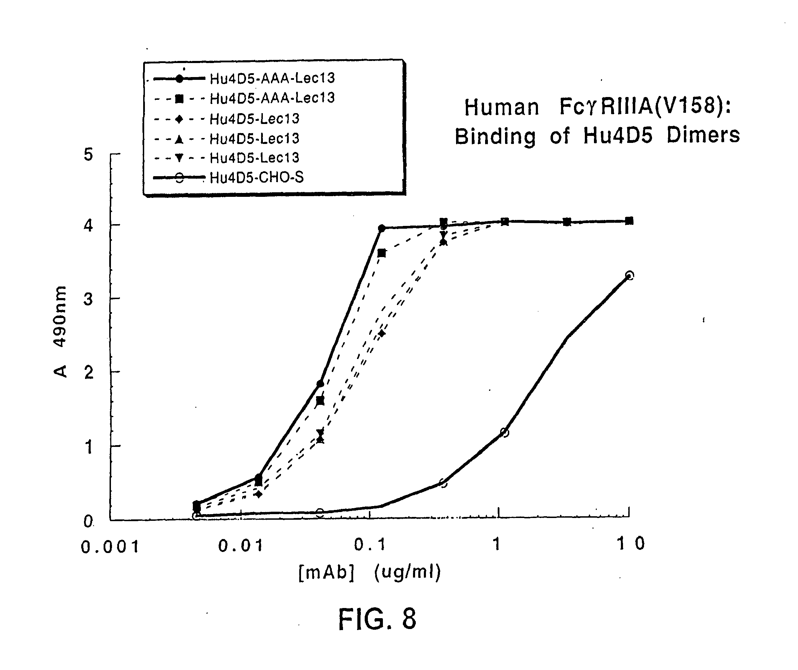

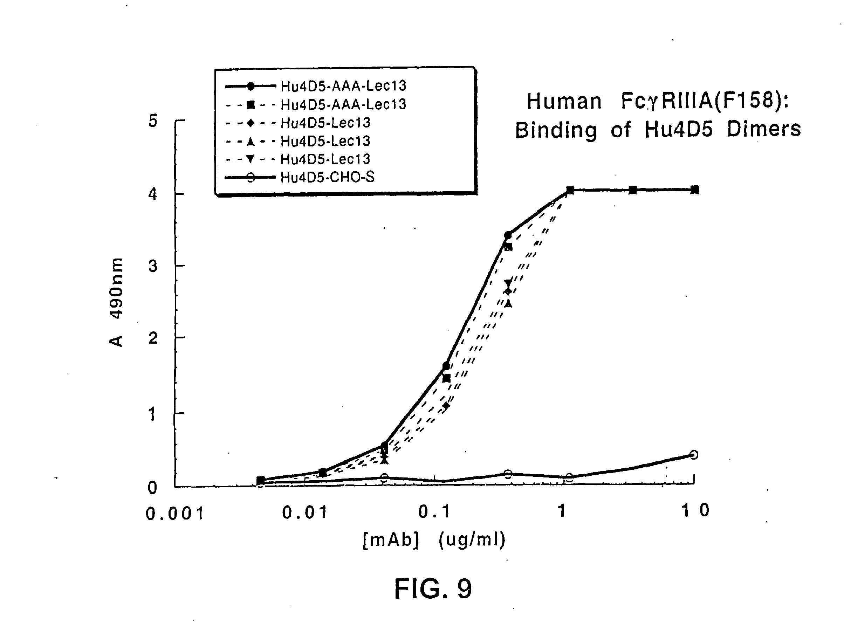

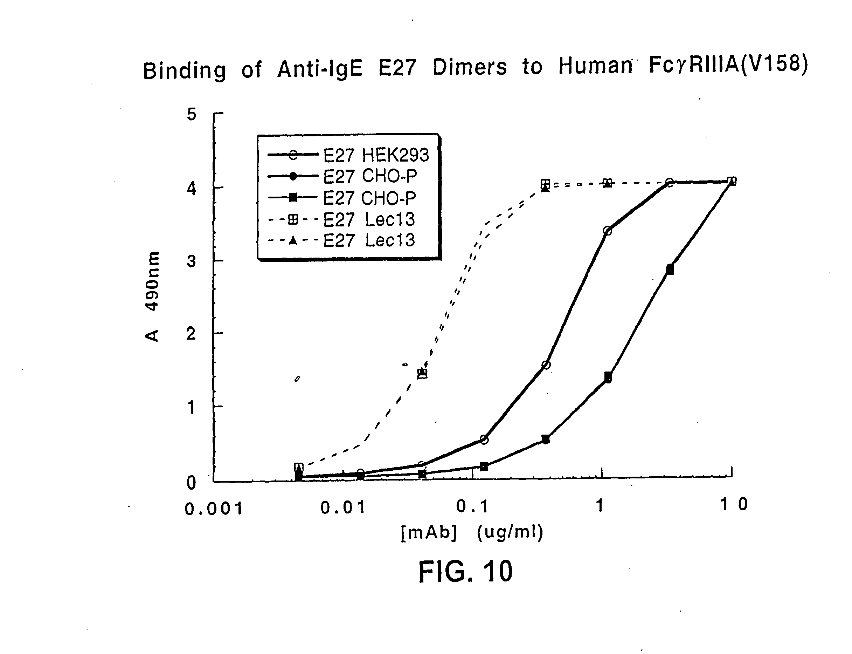

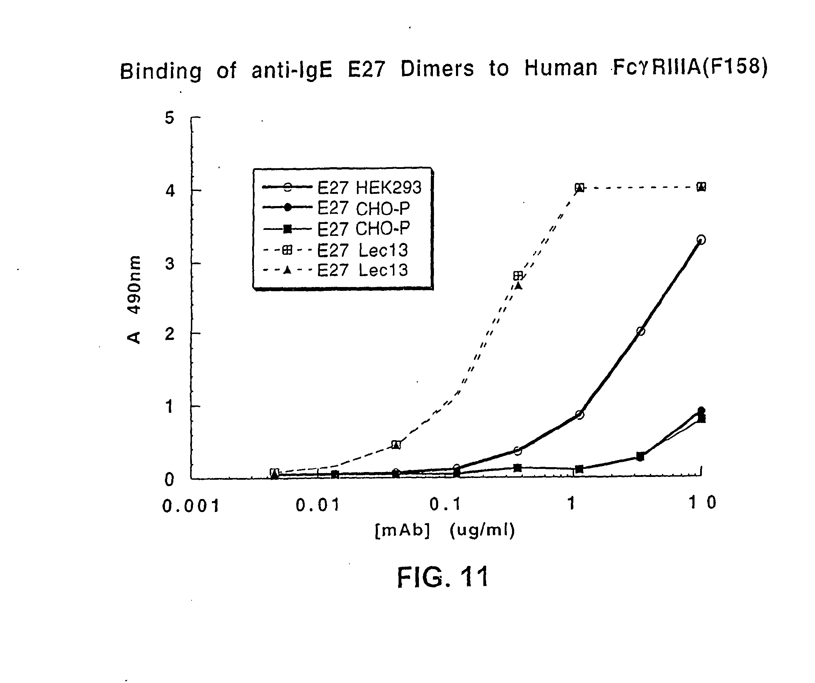

Both polymorphic forms of FcγRIIIA exhibited significantly improved binding to IgG1 which lacked fucose. Binding of dimeric Lecl3-Hu4D5 to FcγRIIIA(V158) showed a 14-fold improvement over CHO-Hu4D5 (Fig. 8; Table 2) and binding to FcγRIIIA(F158) showed at least a 100-fold improvement (Fig. 9). Lec13-HuE27 dimers also exhibited improved binding to both polymorphic forms of FcγRIIIA (Fig. 10, 11; Table 2).

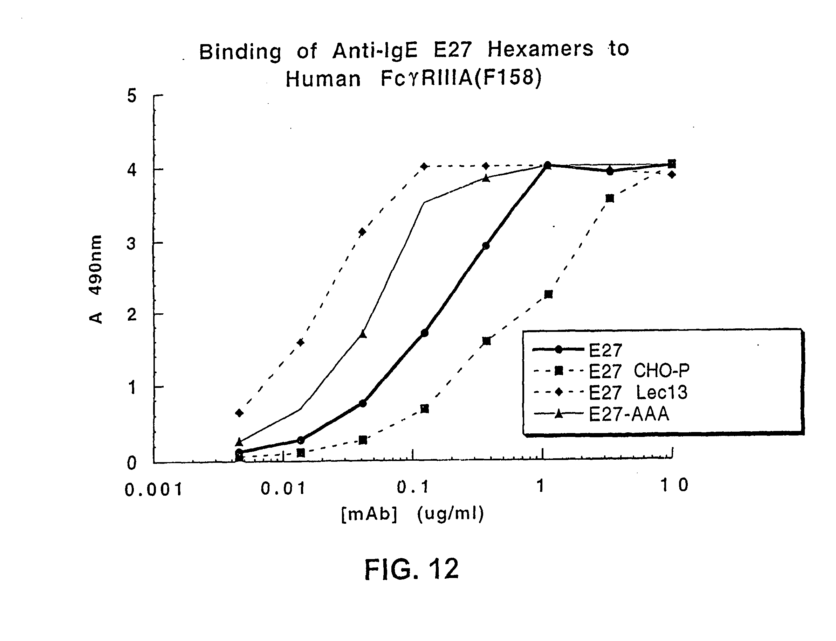

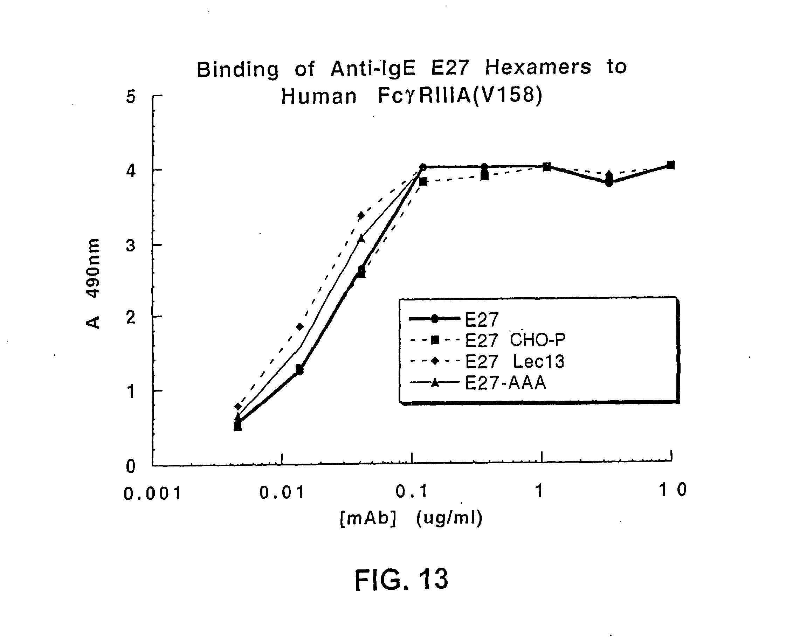

In a previous study of the effect of protein-sequence variants of human IgG1 on binding to human FcγR, hexameric complexes consisting of three anti-IgE E27 and three IgE were used (Shields et al. J. Biol. Chem. 276: 6591-6604 (2001)); hence, these complexes are trimeric in anti-IgE E27. In that study, the improvement in binding of an S298A/E333A/K334A-IgG1 variant to FcγRIIIA(F158) and FcγRIIIA(V158) was 1.5- to 2-fold and 1.1-fold, respectively; while the improvement might seem minimal, the effect on ADCC was significant (Shields et al. J. Biol. Chem. 276: 6591-6604 (2001)). In the current study, the hexameric complex of S298A/E333A/K334A-IgG1 showed improved binding to both FcyRIIIA polymorphic forms in line with the values from the previous study (Fig. 12, 13; Table 2); likewise the hexameric complex of Lec13-HuE27 (native IgG1) exhibited improved binding of approximately 2-fold to FcγRIIIA(F158) (Table 2). When assayed as dimers, the S298A/E333A/K334A-IgG1 variant with fucose exhibited 9-fold and 20-fold improvement in binding to FcγRIIIA(V158) and FcγRIIIA(F158), respectively; the same variant without fucose showed even more improvement in binding to the polymorphic forms of 21-fold and 33-fold, respectively (Table 2). Hence, the absence of fucose not only increases binding of native IgG1 to FcγRIIIA but also can augment binding of IgG1 Fc variants. Thus, protein and carbohydrate alterations are synergistic.

For all forms of HuE27 (native, S298A/E333A/K334A-IgG1, Lecl3-derived), the improvement in binding of the larger complex (trimeric in HuE27) was much smaller than that observed for the same mAb as a dimeric complex. For example, binding of Lecl3-HuE27 as dimer showed an approximately 20-fold improvement, but only a 2-fold improvement for the larger complex (Table 2). This suggests that as the size of the immune complex is increased, the effect of avidity may begin to dominate the binding.

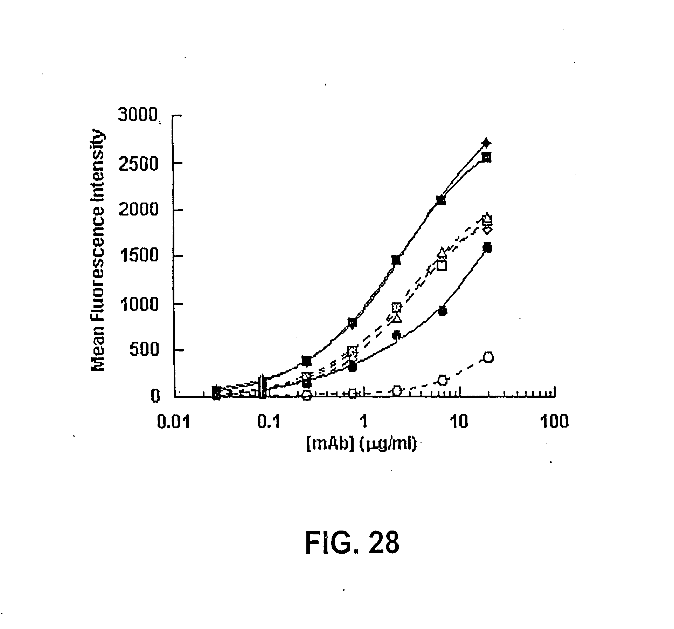

The improved binding of fucose-deficient IgG1 to FcγRIIIA was confirmed with FcγRIIIA(Phe 158) full-length α-chain co-expressed with γ-chain on CHO cells (Fig. 28; Table 2). As for the α-chain fusion protein alone in ELISA-format, fucose deficiency was synergistic with the S29SA/E333A/K334A-IgG1 variant.

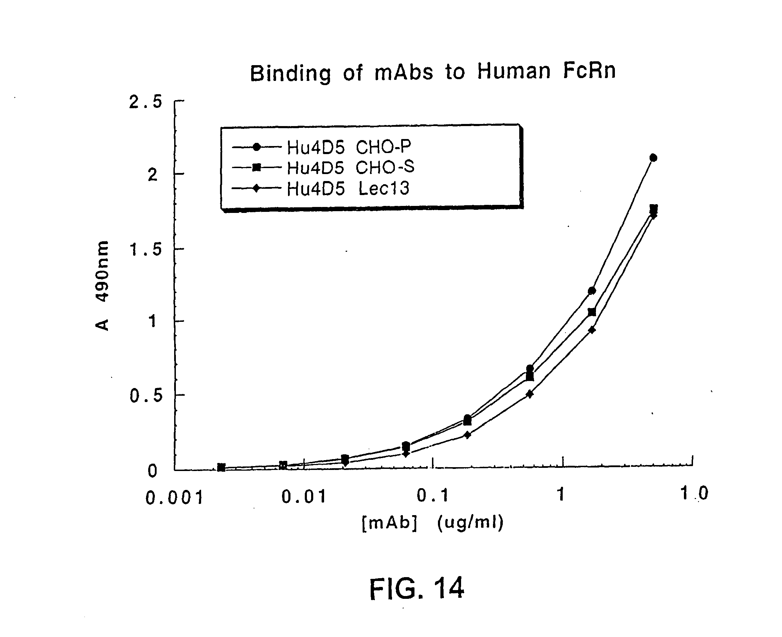

Binding of the native and fucose-minus IgG1 to murine FcγRII and FcγRIII was also evaluated. Human IgG1, even as dimers, binds poorly to these receptors and no improvement in binding was seen with the IgG1 without fucose. Another receptor for IgG, the neonatal Fc receptor (FcRn), is structurally unrelated to the FcγR (Burmeister et al. Nature 372: 379-383 (1994); and Raghavan et al. Annu. Rev. Cell Dev. Biol. 12: 181-220 (1996)) and has been proposed to be involved in a number of biological processes including clearance rate of IgG (Ghetie et al. Annu. Rev. Immunol. 18: 739-766 (2000)). Binding of fucosylated and non-fucosylated IgG1 to FcRn was equivalent (Fig. 14). This is not surprising since aglycosylated IgG1 binds this receptor similar to glycosylated IgG1 (aglyco Ab binds FcRn).

Lack of fucose on the Asn297-linked carbohydrate resulted in significantly improved binding to human FcγRIIIA (both the F158 and V158 polymorphic forms) in an ELISA-format assay. The augmented binding to FcγRIIIA was further substantiated by the ability of the fucose-minus IgG to boost cytotoxicity in ADCC assays utilizing purified human PBMCs. The improved cytotoxicity was especially apparent at lower concentrations of antibody, suggesting that therapeutic antibodies which utilize ADCC could conceivably be given at lower dose to effect an equivalent cell kill as higher dose fucosylated IgG.

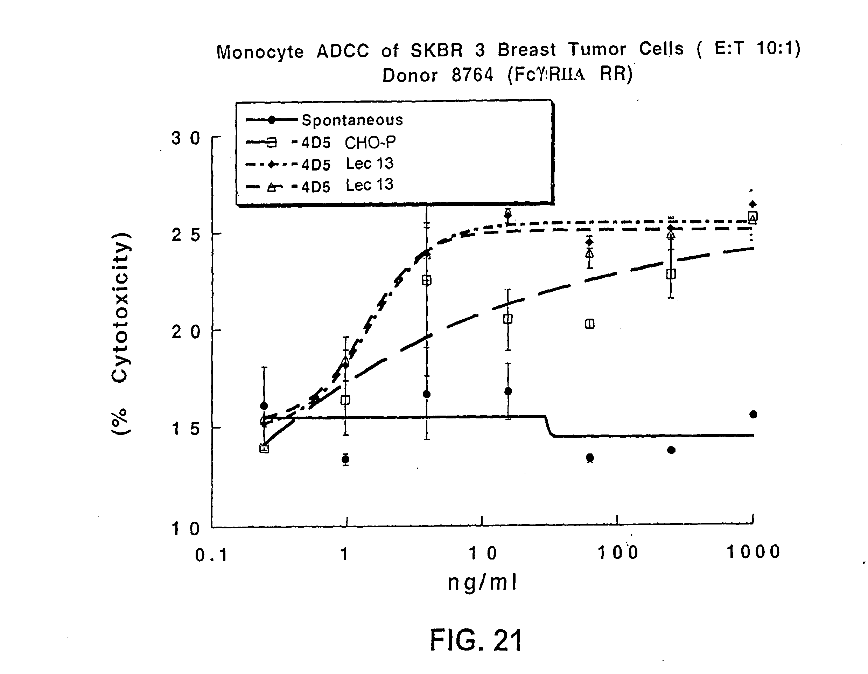

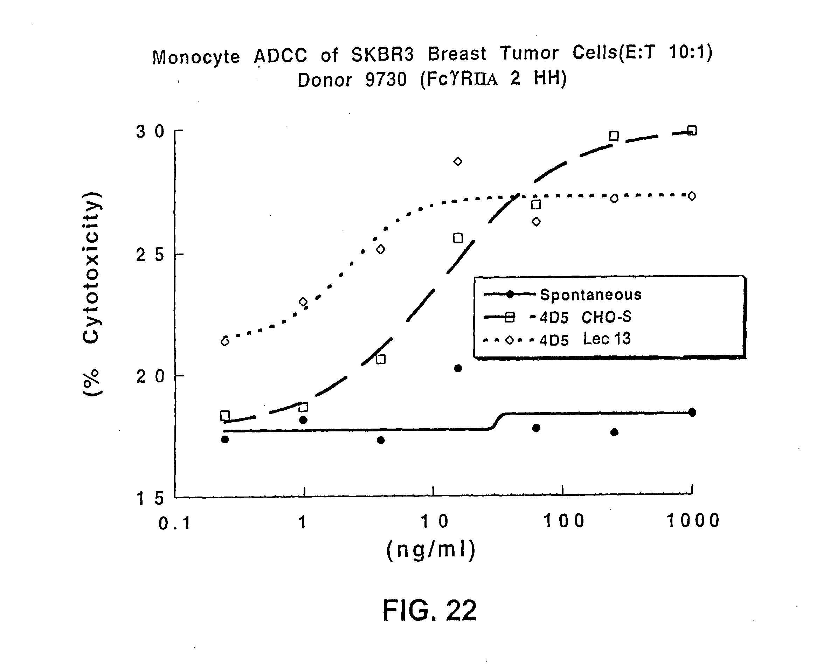

A smaller improvement in binding of fucose-minus IgG was found for human FcγRIIA(R131) and FcγRIIB; no difference was seen for human FcγRIIA(H131). The two former receptors have arginine at position 131, implying without being limited to this theory, that in fucosylated IgG the fucose residue may either interact directly (and evidently, negatively) with FcγRII residue 131 or may subtly influence IgG conformation, which results in a negative interaction. Though the improvement in binding of fucose-minus IgG to FcγRIIA(R131) and FcγRIIB in the ELISA-format assays was small (∼ 2-fold), ADCC assays using monocytes also showed some augmented cytotoxicity at lower concentrations of antibody. Monocytes express FcγRI, FcγRIIA, FcγRIIIB and only a subpopulation of monocytes expresses FcγRIIIA. Since the binding to human FcγRI was equivalent for both fucosyl IgG and fucose-minus IgG, the improvement in ADCC is not likely due to differential interaction with FcγRI. Both FcγRIIA(R131/R131) and FcγRIIA(H131/H131) donor monocytes showed some improvement in ADCC (Figs. 21, 22) suggesting without being limited to any one theory, that (1) FcγRIIA may not be expressed at a high enough level on the monocytes to show a difference between the two polymorphic forms, (2) FcγRIIB may be the predominate binding FcγR (thereby effecting both R131/R131 and H131/H131 monocytes equivalently), or (3) the subpopulation of monocytes expressing FcγRIIA is responsible for the improved ADCC.

Comparison of the carbohydrate found on native IgG from the Lecl3-produced and CHO-produced IgG showed no appreciable differences in the extent of galactosylation and hence the results can be attributed solely to the presence/absence of fucose. However, for the S298A/E333A/K334A IgG1 variant the Lecl3-produced, HEK293-produced, and DP12-produced IgG showed variation in galactosylation. However, the combination of protein-sequence variation and lack of fucose did appear to be additive.

A previous study of protein-sequence variants of human IgG found that alanine (and other) substitutions at some Fc positions could reduce or improve binding to FcγR as well as show improved ADCC (Shields et al. J. Biol. Chem. 276(9):6591-6604 (2001). Interestingly, some of these were not near the interaction interface found in a crystal structure of human IgG Fc-human FcγRIIIA complex (Sondermann et al. Nature 406:267-273 (2000)). For example, of the three alanine substitutions S298A/E333A/K334A used in this study, only S298 is at the interface of the Fc-FcγRIIIA in the crystal structure. Likewise, in the co-crystal structure neither of the fucose residues on the two Fc heavy chains interact with the FcγRIIIA. Inspection of crystal structures of human and rodent Fc or IgG shows that the fucose can adopt varying conformation and exhibits high B-factors, suggesting a high degree of mobility.

Normal CHO and HEK293 cells add fucose to IgG oligosaccharide to a high degree (97-98%). IgG from sera are also highly fucosylated.

### EXAMPLE 2

### C1q and FeRn binding

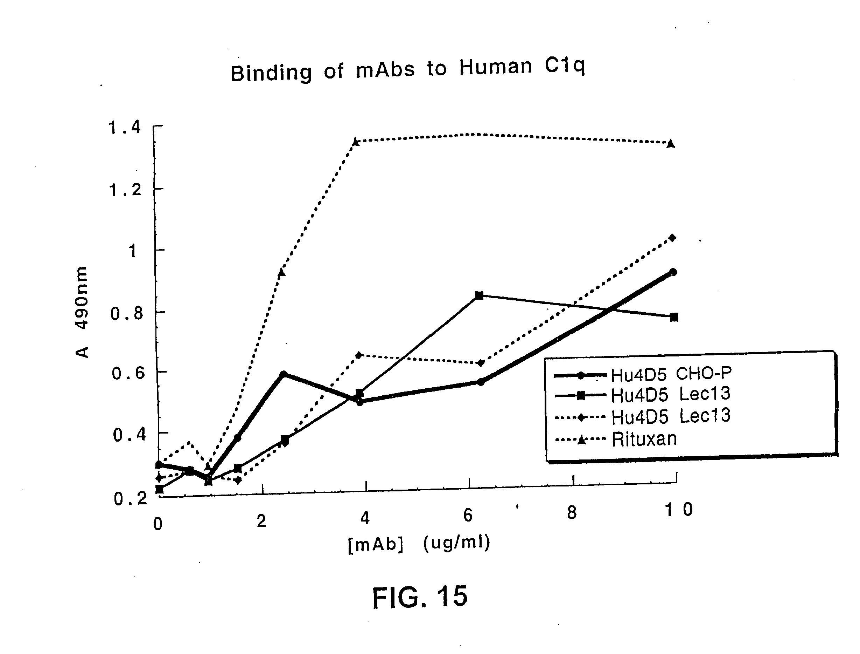

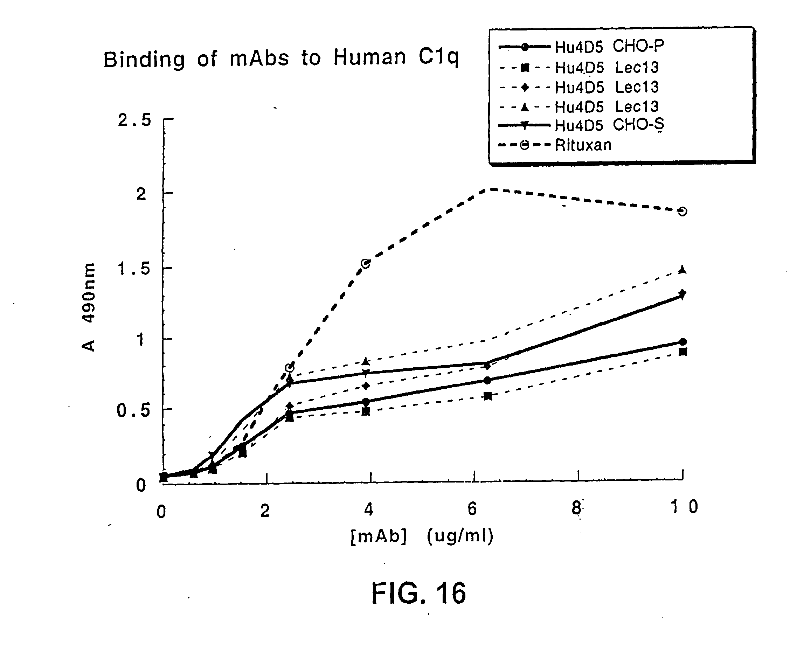

Binding of C1q to antibodies is the first step in the classical pathway of complement activation (Makrides, S. C. Pharmacol. Rev. 50: 59-87 (1998)). The nature of the carbohydrate on the IgG influences the interaction with C1q (Wright et al. J. Immunol. 160: 3393-3402 (1998); Boyd et al. Molec. Immunol. 32: 1311-1318 (1995); and Tsuchiya et al. J. Rheumatol. 16: 285-290 (1989)). Hu4D5 binds human C1q less well than does RITUXAN®, an anti-CD20 mouse/human chimeric IgG1 (Fig. 15, 16) (Idusogie et al. J. Immunol. 164: 4178-4184 (2000)), and the lack of fucose did not affect the ability of Hu4D5 to interact with human C1q (Fig. 15, 16). Likewise, the presence or absence of fucose did not appear to affect IgG1 binding to FcRn.

### EXAMPLE 3

### Antibody Dependent Cellular Cytotoxicity (ADCC)

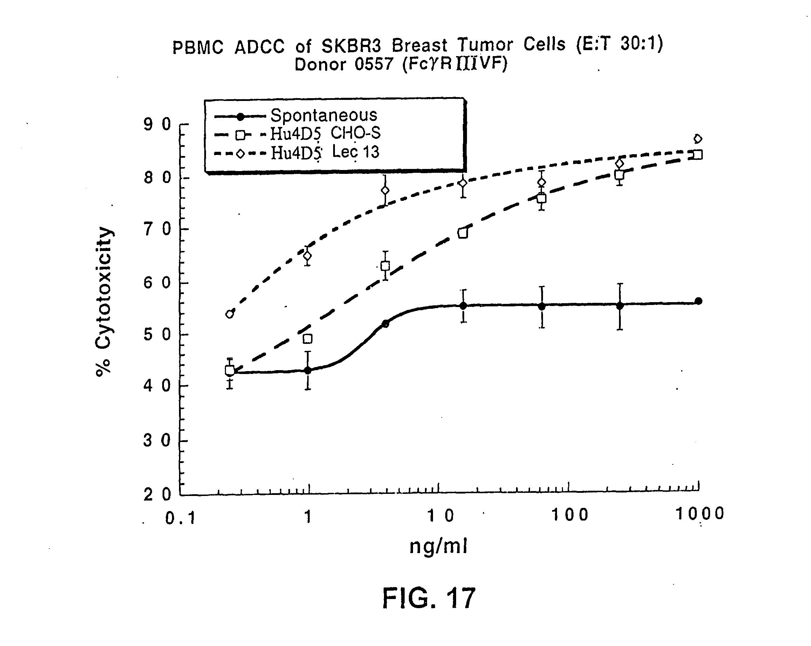

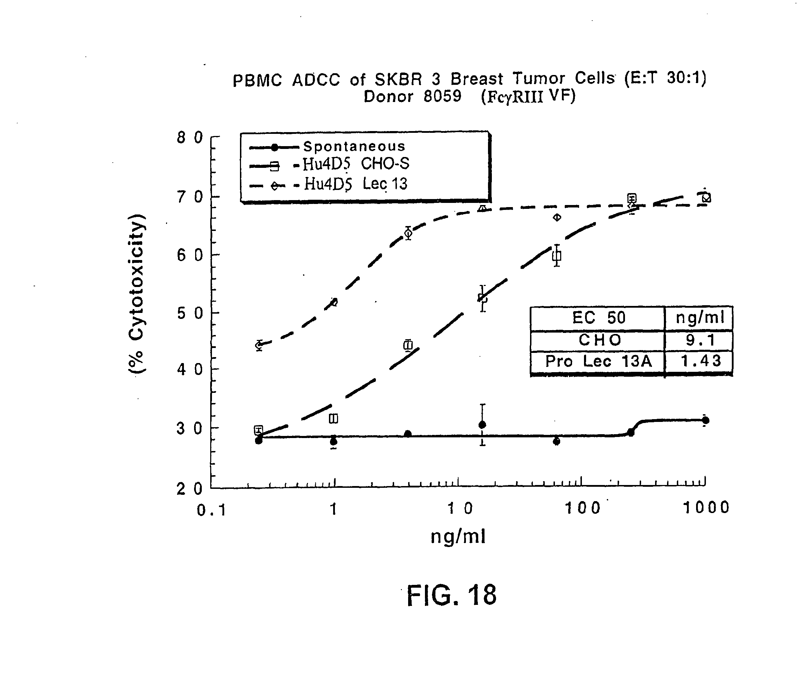

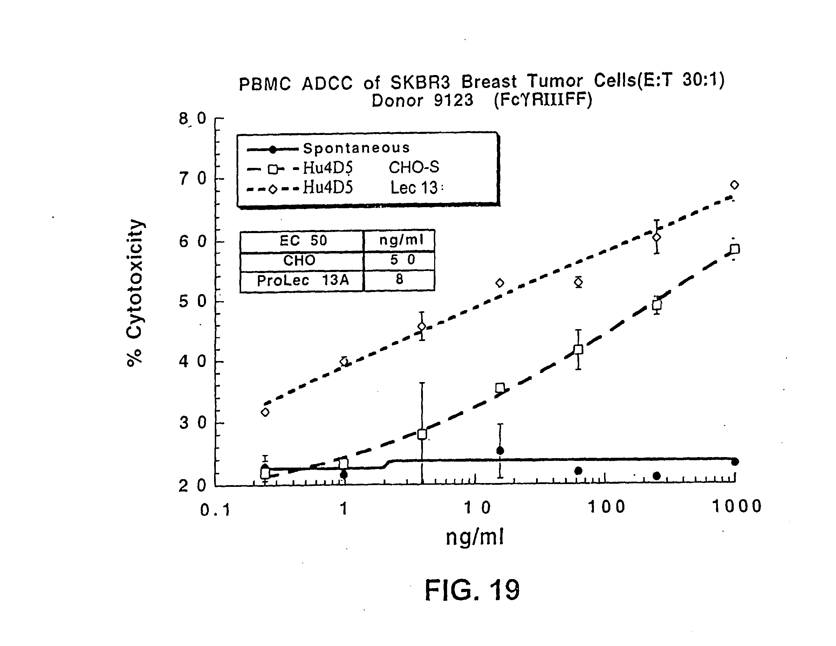

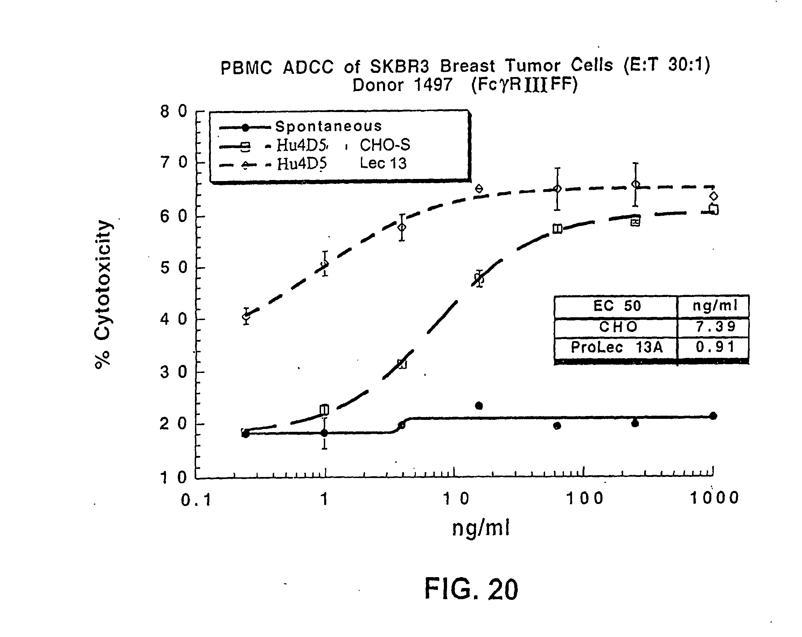

The affect of lack of fucose on ADCC was evaluated using Lecl3-Hu4D5 IgG1 on the human breast cancer cell line SK-BR-3 (Hudziak et al. Mol. Cell Biol. 9: 1165-1172 (1989)). PBMCs from two FcγRIIIA(V158/F158) donors and two FcγRIIIA(F158/F158) donors were used as effector cells in a 30:1 effector:target ratio. For all donors the IgG1 without fucose exhibited significant improved in ADCC compared to IgG1 with fucose (Figs. 17-20). Notably, for all donors the improvement in cytotoxicity was more apparent as the concentration of antibody was reduced. This may reflect the larger improvement in binding seen for the dimers compared to that for the hexamers, i.e. the fucose-minus variant may require fewer mAbs on the surface of the target cell in order to effect binding/activation of an effector cell.

Human monocytes express FcyRI, FcγRIIA, FcγRIIB and only a subpopulation expresses FcγREIIA. Since the lack of fucose did not affect binding to FcγRI but did have a small effect on binding to FcγRIIA(R131) and FcγRIIB, ADCC assays were run using purified human monocytes as effector cells at effector:target ratios of 20:1, 10:1, and 5:1. Purification of monocytes is more difficult than purification of PBMCs and the ADCC assay is consequently more difficult. As with the PBMC ADCC, monocyte ADCC showed improved cytotoxicity for the IgG1 lacking fucose though the effect appears less pronounced and the ability of monocytes to kill target cells is reduced compared to PBMCs (Figs. 21-22).

### EXAMPLE 4

### ADCC Activity of Fc Variant Antibodies

The following experiments compared: (1) Hu4D5 expressed in CHO cells (Hu4D5 CHO-S), (2) Hu4D5 fucose deficient variant expressed in Lecl3 cells (hu4D5 Lecl3), (3) Hu4D5 triple alanine Fc domain substitution variant expressed in 293 HEK cells (Hu4D5 HEK293-AAA) and (4) Hu4D5 fucose deficient triple alanine Fc domain substitution variant expressed in Lecl3 cells (Hu4D5-Lecl3-AAA).

*ADCC Methods:* Natural killer (NK) cells were purified from peripheral blood of 2 donors by negative selection using magnetic beads (Miltenyi Biotech, Auburn, CA). Donors were selected to be homozygous for the allele expressing the F158 form of FcγR3 (CD16)(F/F158) (Shields et al., J. Biol. Chem. 276:6591-6604 (2001)) which expresses a lower affinity binding phenotype for IgG. HER2-overexpressing SKBR-3 breast carcinoma cells were oposonized with 1ng/ml of each antibody for 45 minutes at 25°C in assay media (50:50 Hams F12: DMEM containing 1 % heat inactivated fetal bovine serum and 10mM Hepes buffer) and then treated with varying concentrations of NK cells at effector to target ratios (E:T) ranging from 10:1 to 0.156 for 5 hours at 37°C in a humidified CO₂ incubator. Cytotoxicity was measured by the release of lactate dehydrogenase (LDH) using a commercial kit (Roche Diagnostics, Indianapolis, IN).

*Indirect Immunofluorescence Staining of NK cells Methods:* Purified NK cells were incubated with 2 µg/ml of each Hu4D5 variant for 30 min. at 4°C in staining buffer (phosphate buffered saline, 0.1 % bovine serum albumin, 0.01 % sodium azide). Cells were washed 3 times and incubated with phycoeyrithrin conjugated mouse mab anti-human CD56 (Pharmingen, San Diego, CA) and FITC - F(ab')₂ goat anti-human IgG (F(ab')₂ specific (Jackson Immunoresearch, West Grove, PA) for an additional 30 min. at 4°C. Cells were analyzed for 2 color immunofluorescence staining on a FACScan^{™} flow cytometer (B.D. Biosciences, San Jose, CA)

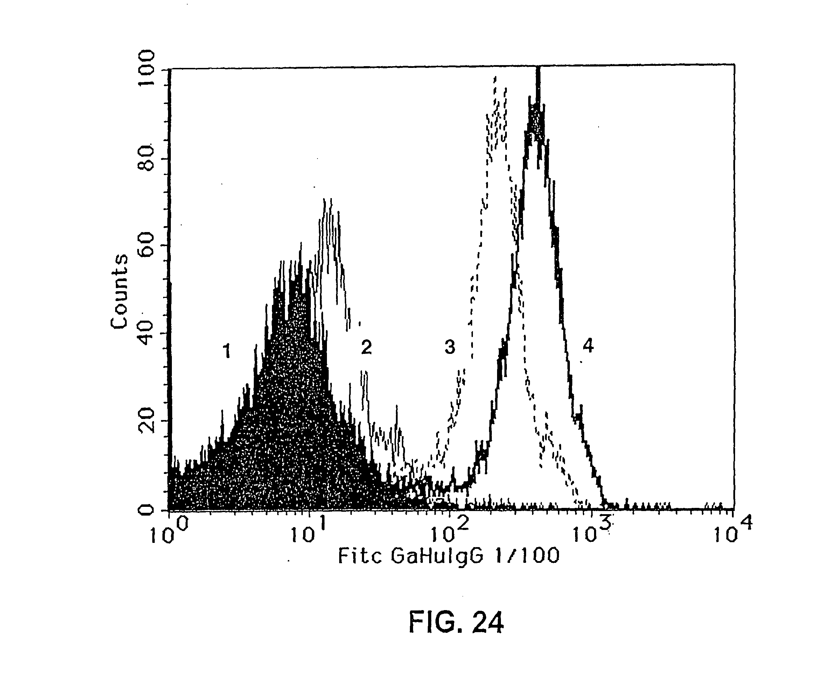

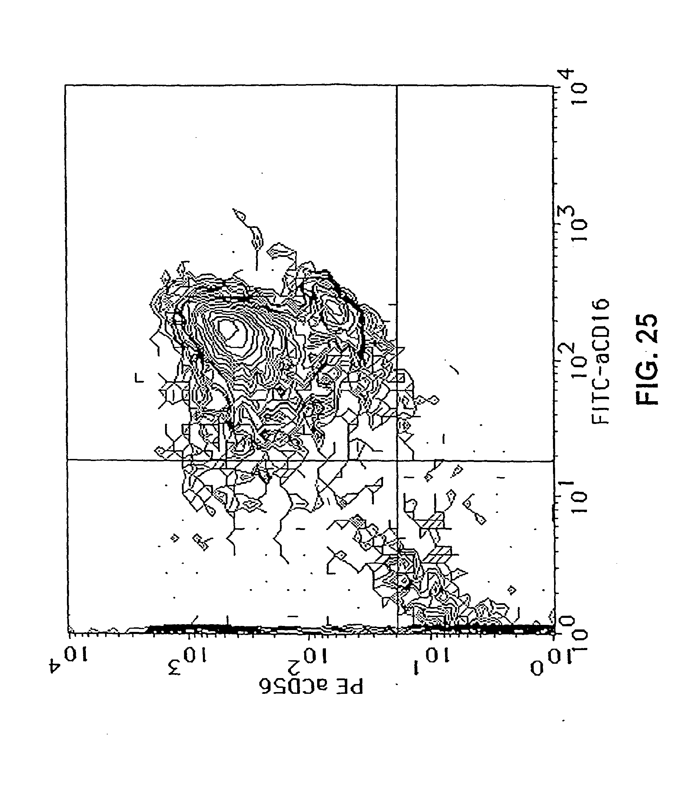

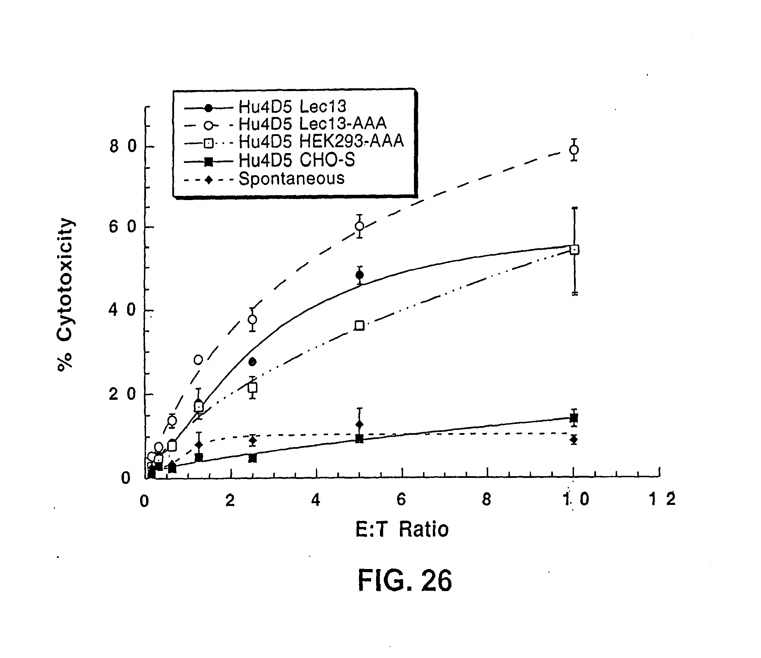

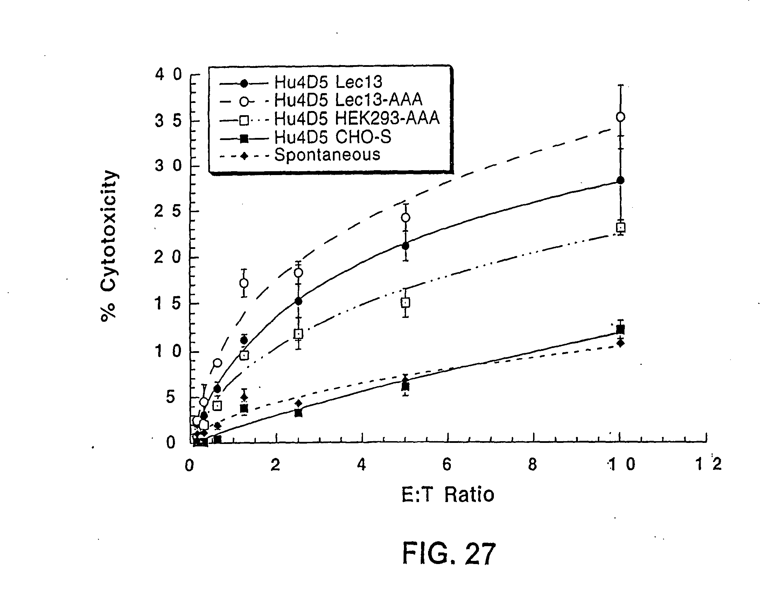

*Conclusion:* In both donors (5365 and 7580), there was enhanced ADCC activity at E/T ratios greater than 2 (see Figures 26 and 27) for the Hu4D5-Lecl3-AAA form of Hu4D5 relative to the Hu4D5-Lecl3 form which suggests a synergistic enhancement of FcγRIII binding/ADCC in the fucose deficient triple alanine Fc variant. This increased in binding was confirmed by indirect immunofluorescence staining of NK cells in donor 5365 (see Figures 24 and 25) in CD56/CD16 expressing NK cells.

## Claims

1. A composition comprising a glycoprotein having a human IgG Fc region, wherein about 51-100% of the glycoprotein in the composition comprises a mature core carbohydrate structure which lacks fucose, attached to the Fc region of the glycoprotein, wherein the Fc region comprises an amino acid sequence that differs from a native sequence human IgG Fc region, and wherein the glycoprotein binds FcγRIII with better affinity, or mediates antibody-dependent cell-mediated cytotoxicity (ADCC) more effectively, than the glycoprotein with a mature core carbohydrate structure including fucose attached to the Fc region of the glycoprotein.

2. The composition of claim 1, wherein the Fc region comprises an amino acid substitution at any one or more of amino acid positions 256, 290, 298, 312, 326, 330, 333, 334, 360, 378 or 430, utilizing EU numbering for the Fc region residues.

3. The composition of claim 2, wherein the Fc region comprises amino acid substitutions at any two or three of the residues at positions 298, 333 and 334.

4. The composition of claim 3, wherein the Fc region comprises amino acid substitutions at positions 298, 333 and 334.

5. The composition of claim 4, wherein the replacement residues at postitions 298, 333 and 334 are alanine.

6. A composition according to any of claims 1 to 5, wherein about 80-100% of the glycoprotein in the composition comprises a mature core carbohydrate structure which lacks fucose, attached to the Fc region of the glycoprotein.

7. A composition according to any of claims 1 to 6, wherein the glycoprotein comprises an antibody.

8. A composition according to any of the preceding claims, wherein the human IgG Fc region comprises a human IgG1 IgG2, IgG3 or IgG4 Fc region.

9. A composition according to claim 7 or claim 8, wherein the antibody is a chimeric, humanized or human antibody.

10. The composition of any of claims 7 to 9, wherein the antibody binds an antigen selected from the group consisting of a B-cell surface marker, an ErbB receptor, a tumor-associated antigen and an angiogenic factor.

11. The composition of claim 10 wherein the antibody binds CD20, HER2, vascular endothelial growth factor (VEGF), CD40, or prostate stem cell antigen (PSCA).

12. The composition of claim 11 wherein the antibody comprises a humanzed anti-HER2 antibody, a chimeric anti-CD20 antibody and a humanized anti-VEGF antibody.

13. A composition according to any of the preceding claims, wherein about 90-99% of the glycoprotein in the composition comprises a mature core carbohydrate structure which lacks fucose, attached to the Fc region of the glycoprotein.

14. A composition according to any of the preceding claims, wherein the glycoprotein has been produced by a chinese hamster ovary (CHO) cell.

15. A composition according to claim 14, wherein the CHO cell is a Lecl3 cell.

16. A composition according to any of claims 1 to 14, wherein the glycoprotein is essentially free of bisecting N-acetylglucosamine (GlcNAc) attached to the mature core carbohydrate structure.

17. A composition according to any of claims 1 to 14, wherein the glycoprotein has bisecting N-acetylglucosamine (GlcNAc) attached to the mature core carbohydrate structure.

18. A composition according o any of claims 1 to 14, 16 or 17, wherein the glycoprotein has one or more galactose residues attached to the mature core carbohydrate structure.

19. A composition according to any of claims 1 to 14, 16 or 17, wherein the glycoprotein is essentially free of one or more galactose residues attached to the mature core carbohydrate structure.

20. A composition according to any of claims 1 to 14 or 16 to 19, wherein the glycoprotein has one or more sialic acid residues attached to the mature core carbohydrate structure.

21. A composition according to any of claims 1 to 14 or 16 to 19, wherein the glycoprotein is essentially free of one or more sialic acid residues attached to the mature core carbohydrate structure.

22. A composition according to any of the preceding claims, which is a pharmaceutical preparation.

23. The pharmaceutical preparation of claim 22 further comprising a pharmaceutically acceptable carrier.

24. A composition according to claim 22 or claim 23, which is sterile.

25. A composition according to any of claims 22 to 24, which is lyophilized.

26. A composition according to any of claims 1 to 6, wherein the glycoprotein is an immunoadhesin.

27. A composition according to any of claims 1 to 21 for use in a method of treatment of a mammal by administration of the composition to treat a disease or disorder in the mammal that would benefit from such treatment.

28. A composition according to claim 27 wherein the mammal is human.

29. A composition according to claim 28 wherein the human expresses FcγRIII(F158).

30. A composition according to any of claims 27 to 29, wherein the disease or disorder is selected from the group consisting of cancer, an autoimmune disease, an inflammatory disorder, infection, or another condition where removal of cells or tissue is desired.

31. A host cell comprising nucleic acid encoding a glycoprotein which comprises an Fc region as defined in any of claims 1 to 5, 7 to 12, or 16 to 21, wherein about 80-100% of the glycoprotein produced by the host cell comprises a mature core carbohydrate structure which lacks fucose, attached to the Fc region of the glycoprotein.

32. The host cell of claim 31 which is a chinese hamster ovary (CHO) cell.

33. A host cell according to claim 32, which is a Lecl3 cell.

34. A method for producing a glycoprotein comprising culturing a host cell according to any of claims 31 to 33 so that the nucleic acid is expressed.

35. The method of claim 34 further comprising recovering the glycoprotein from the host cell culture.

36. The method of claim 35 further comprising conjugating the glycoprotein to a heterologous molecule.

37. The method of claim 36 wherein the heterologous molecule is a cytotoxic agent or an enzyme.

## Patentansprüche

1. Zusammensetzung, umfassend ein Glykoprotein mit einer menschlichen IgG-Fc-Region, worin etwa 51-100 % des Glykoproteins in der Zusammensetzung eine reife Kern-Kohlenhydratstruktur, der Fucose fehlt, gebunden an die Fc-Region des Glykoproteins umfassen, worin die Fc-Region eine Aminosäuresequenz umfasst, die sich von einer menschlichen Nativsequenz-IgG-Fc-Region unterscheidet, und worin das Glykoprotein FcγRIII mit besserer Affinität bindet oder antikörperabhängige zellvermittelte Zytotoxizität (ADCC) wirksamer vermittelt als das Glykoprotein mit einer reifen Kern-Kohlenhydratstruktur, umfassend Fucose, die an die Fc-Region des Glykoproteins gebunden ist.

2. Zusammensetzung nach Anspruch 1, worin die Fc-Region eine Aminosäure-Substitution an einer oder mehreren beliebigen der Aminosäure-Positionen 256, 290, 298, 312, 326, 330, 333, 334, 360, 378 oder 430, unter Verwendung der EU-Nummerierung für die Fc-Regionsreste, umfasst.

3. Zusammensetzung nach Anspruch 2, worin die Fc-Region Aminosäure-Substitutionen an beliebigen zwei oder drei der Reste an den Positionen 298, 333 und 334 umfasst.

4. Zusammensetzung nach Anspruch 3, worin die Fc-Region Aminosäure-Substitutionen an den Positionen 298, 333 und 334 umfasst.

5. Zusammensetzung nach Anspruch 4, worin die Ersatz-Reste an den Positionen 298, 333 und 334 Alanin sind.

6. Zusammensetzung nach einem der Ansprüche 1 bis 5, worin etwa 80-100 % des Glykoproteins in der Zusammensetzung eine reife Kern-Kohlenhydratstruktur, der Fucose fehlt, gebunden an die Fc-Region des Glykoproteins umfassen.

7. Zusammensetzung nach einem der Ansprüche 1 bis 6, worin das Glykoprotein einen Antikörper umfasst.

8. Zusammensetzung nach einem der vorangegangenen Ansprüche, worin die menschliche IgG-Fc-Region eine menschliche IgG1-, IgG2-, IgG3- oder IgG4-Fc-Region umfasst.

9. Zusammensetzung nach Anspruch 7 oder Anspruch 8, worin der Antikörper ein chimärer, humanisierter oder menschlicher Antikörper ist.

10. Zusammensetzung nach einem der Ansprüche 7 bis 9, worin der Antikörper ein Antigen bindet, das aus der aus einem B-Zellen-Oberflächen-Marker, einem ErbB-Rezeptor, einem tumorassoziierten Antigen und einem angiogenen Faktor bestehenden Gruppe ausgewählt ist.

11. Zusammensetzung nach Anspruch 10, worin der Antikörper CD20, HER2, den Gefäßendothel-Wachstumsfaktor (VEGF), CD40 oder das Prostata-Stammzeilen-Antigen (PSCA) bindet.

12. Zusammensetzung nach Anspruch 11, worin der Antikörper einen humanisierten Anti-HER2-Antikörper, einen chimären Anti-CD20-Antikörper und einen humanisierten Anti-VEGF-Antikörper umfasst.

13. Zusammensetzung nach einem der vorangegangenen Ansprüche, worin etwa 90-99 % des Glykoproteins in der Zusammensetzung eine reife Kern-Kohlenhydratstruktur, der Fucose fehlt, gebunden an die Fc-Region des Glykoproteins umfassen.

14. Zusammensetzung nach einem der vorangegangenen Ansprüche, worin das Glykoprotein von einer Chinahamster-Eierstock- (CHO-) Zelle produziert wurde.

15. Zusammensetzung nach Anspruch 14, worin die CHO-Zelle eine Lec13-Zelle ist.

16. Zusammensetzung nach einem der Ansprüche 1 bis 14, worin das Glykoprotein im Wesentlichen frei von halbierendem N-Acetylglucosamin (GlcNAc), gebunden an die reife Kern-Kohlenhydratstruktur, ist.

17. Zusammensetzung nach einem der Ansprüche 1 bis 14, worin das Glykoprotein halbierendes N-Acetylglucosamin (GlcNAc), gebunden an die reife Kern-Kohlenhydratstruktur, aufweist.

18. Zusammensetzung nach einem der Ansprüche 1 bis 14, 16 oder 17, worin das Glykoprotein einen oder mehrere Galactose-Reste aufweist, die an die reife Kern-Kohlenhydratstruktur gebunden sind.

19. Zusammensetzung nach einem der Ansprüche 1 bis 14, 16 oder 17, worin das Glykoprotein im Wesentlichen frei von einem oder mehreren Galactose-Resten, gebunden an die reife Kern-Kohlenhydratstruktur, ist.

20. Zusammensetzung nach einem der Ansprüche 1 bis 14 oder 16 bis 19, worin das Glykoprotein einen oder mehrere Sialinsäure-Reste aufweist, die an die reife Kern-Kohlenhydratstruktur gebunden sind.

21. Zusammensetzung nach einem der Ansprüche 1 bis 14 oder 16 bis 19, worin das Glykoprotein im Wesentlichen frei von einem oder mehreren Sialinsäure-Resten, gebunden an die reife Kern-Kohlenhydratstruktur, ist.

22. Zusammensetzung nach einem der vorangegangenen Ansprüche, die ein pharmazeutisches Präparat ist.

23. Pharmazeutisches Präparat nach Anspruch 22, weiters umfassend einen pharmazeutisch annehmbaren Träger.

24. Zusammensetzung nach Anspruch 22 oder Anspruch 23, die steril ist.

25. Zusammensetzung nach einem der Ansprüche 22 bis 24, die lyophilisiert ist.

26. Zusammensetzung nach einem der Ansprüche 1 bis 6, worin das Glykoprotein ein Immunadhäsin ist.

27. Zusammensetzung nach einem der Ansprüche 1 bis 21 zur Verwendung in einem Behandlungsverfahren eines Säugetiers durch Verabreichung der Zusammensetzung zur Behandlung einer Erkrankung oder einer Störung bei dem Säugetier, das von einer solchen Behandlung profitieren würde.

28. Zusammensetzung nach Anspruch 27, worin das Säugetier ein Mensch ist.

29. Zusammensetzung nach Anspruch 28, worin der Mensch FcγRIII (F158) exprimiert.

30. Zusammensetzung nach einem der Ansprüche 27 bis 29, worin die Erkrankung oder Störung aus der aus Krebs, einer Autoimmun-Erkrankung, einer Entzündungserkrankung, einer Infektion oder einer anderen Erkrankung, bei der die Entfernung von Zellen oder Gewebe gewünscht wird, bestehenden Gruppe ausgewählt ist.

31. Wirtszelle, umfassend Nucleinsäure, die für ein Glykoprotein kodiert, das eine Fc-Region umfasst, wie in einem der Ansprüche 1 bis 5, 7 bis 12 oder 16 bis 21 definiert, worin etwa 80-100 % des von der Wirtszelle produzierten Glykoproteins eine reife Kern-Kohlenhydratstruktur, der Fucose fehlt, gebunden an die Fc-Region des Glykoproteins umfassen.

32. Wirtszelle nach Anspruch 31, die eine Chinahamster-Eierstock- (CHO-) Zelle ist.

33. Wirtszelle nach Anspruch 32, die eine Lec13-Zelle ist.

34. Verfahren zur Produktion eines Glykoproteins, umfassend das Züchten einer Wirtszelle nach einem der Ansprüche 31 bis 33, so dass die Nucleinsäure exprimiert wird.

35. Verfahren nach Anspruch 34, weiters umfassend das Gewinnen des Glykoproteins aus der Wirtszellen-Kultur.

36. Verfahren nach Anspruch 35, weiters umfassend das Konjugieren des Glykoproteins an ein heterologes Molekül.

37. Verfahren nach Anspruch 36, worin das heterologe Molekül ein zytotoxisches Mittel oder ein Enzym ist.

## Revendications

1. Composition comprenant une glycoprotéine ayant une région Fc de IgG humaine, dans laquelle une proportion d'environ 51 à 100 % de la glycoprotéine présente dans la composition comprend une structure glucidique centrale mature qui est dépourvue de fucose, fixée à la région Fc de la glycoprotéine, dans laquelle la région Fc comprend une séquence d'aminoacides qui diffère d'une région Fc de IgG humaine sous forme de la séquence naturelle, et dans laquelle la glycoprotéine se lie à FcγRIII avec une meilleure affinité, ou assure la médiation de la cytotoxicité à médiation cellulaire dépendant d'anticorps (ADCC) plus efficacement, que la glycoprotéine avec une structure glucidique centrale mature comprenant du fucose fixée à la région Fc de la glycoprotéine.

2. Composition suivant la revendication 1, dans laquelle la région Fc comprend une substitution d'aminoacide au niveau d'une ou plusieurs quelconques des positions d'aminoacides 256, 290, 298, 312, 326, 330, 333, 334, 360, 378 ou 430, en utilisant la numérotation EU pour les résidus de la région Fc.

3. Composition suivant la revendication 2, dans laquelle la région Fc comprend des substitutions d'aminoacides au niveau de deux ou trois quelconques des résidus aux positions 298, 333 et 334.

4. Composition suivant la revendication 3, dans laquelle la région Fc comprend des substitutions d'aminoacides aux positions 298, 333 et 334.

5. Composition suivant la revendication 4, dans laquelle les résidus de remplacement aux positions 298, 333 et 334 sont des résidus alanine.

6. Composition suivant l'une quelconque des revendications 1 à 5, dans laquelle une proportion d'environ 80 à 100 % de la glycoprotéine présente dans la composition comprend une structure glucidique centrale mature qui est dépourvue de fucose, fixée à la région Fc de la glycoprotéine.

7. Composition suivant l'une quelconque des revendications 1 à 6, dans laquelle la glycoprotéine comprend un anticorps.

8. Composition suivant l'une quelconque des revendications précédentes, dans laquelle la région Fc de IgG humaine comprend une région Fc de IgG1, IgG2, IgG3 ou IgG4 humaine.

9. Composition suivant la revendication 7 ou la revendication 8, dans laquelle l'anticorps est un anticorps chimère, humanisé ou humain.

10. Composition suivant l'une quelconque des revendications 7 à 9, dans laquelle l'anticorps se lie à un antigène choisi dans le groupe consistant en un marqueur de surface de lymphocyte B, un récepteur ErbB, un antigène associé à une tumeur et un facteur angiogénique.

11. Composition suivant la revendication 10, dans laquelle l'anticorps se lie à CD20, à HER2, au facteur de croissance endothélial vasculaire (VEGF), à CD40 ou à l'antigène de cellule souche de la prostate (PSCA).

12. Composition suivant la revendication 11, dans laquelle l'anticorps comprend un anticorps humanisé anti-HER2, un anticorps chimère anti-CD20 et un anticorps humanisé anti-VEGF.

13. Composition suivant l'une quelconque des revendications précédentes, dans laquelle une proportion d'environ 90 à 99 % de la glycoprotéine présente dans la composition comprend une structure glucidique centrale mature qui est dépourvue de fucose, fixée à la région Fc de la glycoprotéine.

14. Composition suivant l'une quelconque des revendications précédentes, dans laquelle la glycoprotéine a été produite par une cellule d'ovaire d'hamster chinois (CHO).

15. Composition suivant la revendication 14, dans laquelle la cellule CHO est une cellule Lecl3.

16. Composition suivant l'une quelconque des revendications 1 à 14, dans laquelle la glycoprotéine est pratiquement dépourvue de N-acétylglucosamine (GlcNAc) bissectrice fixée à la structure glucidique centrale mature.

17. Composition suivant l'une quelconque des revendications 1 à 14, dans laquelle la glycoprotéine possède de la N-acétylglucosamine (GlcNAc) bissectrice fixée à la structure glucidique centrale mature.

18. Composition suivant l'une quelconque des revendications 1 à 14, 16 ou 17, dans laquelle la glycoprotéine possède un ou plusieurs résidus galactose fixés à la structure glucidique centrale mature.

19. Composition suivant l'une quelconque des revendications 1 à 14, 16 ou 17, dans laquelle la glycoprotéine est pratiquement dépourvue d'un ou plusieurs résidus galactose fixés à la structure glucidique centrale mature.

20. Composition suivant l'une quelconque des revendications 1 à 14 ou 16 à 19, dans laquelle la glycoprotéine possède un ou plusieurs résidus acide sialique fixés à la structure glucidique centrale mature.

21. Composition suivant l'une quelconque des revendications 1 à 14 ou 16 à 19, dans laquelle la glycoprotéine est pratiquement dépourvue d'un ou plusieurs résidus acide sialique fixés à la structure glucidique centrale mature.

22. Composition suivant l'une quelconque des revendications précédentes, qui est une préparation pharmaceutique.

23. Préparation pharmaceutique suivant la revendication 22, comprenant en outre un support pharmaceutiquement acceptable.

24. Composition suivant la revendication 22 ou la revendication 23, qui est stérile.

25. Composition suivant l'une quelconque des revendications 22 à 24, qui est lyophilisée.

26. Composition suivant l'une quelconque des revendications 1 à 6, dans laquelle la glycoprotéine est une immunoadhésine.

27. Composition suivant l'une quelconque des revendications 1 à 21, destinée à être utilisée dans une méthode de traitement d'un mammifère par administration de la composition pour traiter chez le mammifère une maladie ou un trouble qui bénéficierait d'un tel traitement.

28. Composition suivant la revendication 27, dans laquelle le mammifère est un être humain.

29. Composition suivant la revendication 28, dans laquelle l'être humain exprime FcγRIII (F158).

30. Composition suivant l'une quelconque des revendications 27 à 29, dans laquelle la maladie ou le trouble est choisi dans le groupe consistant en le cancer, une maladie auto-immune, un trouble inflammatoire, une infection, ou une autre affection pour laquelle l'élimination de cellules ou d'un tissu est désirée.

31. Cellule hôte comprenant un acide nucléique codant pour une glycoprotéine qui comprend une région Fc telle que définie dans l'une quelconque des revendications 1 à 5, 7 à 12 ou 16 à 21, dans laquelle une proportion d'environ 80 à 100 % de la glycoprotéine produite par la cellule hôte comprend une structure glucidique centrale mature qui est dépourvue de fucose, fixée à la région Fc de la glycoprotéine.

32. Cellule hôte suivant la revendication 31, qui est une cellule d'ovaire d'hamster chinois (CHO).

33. Cellule hôte suivant la revendication 32, qui est une cellule Lecl3.

34. Procédé pour la production d'une glycoprotéine, comprenant la culture d'une cellule hôte suivant l'une quelconque des revendications 31 à 33 de telle sorte que l'acide nucléique soit exprimé.

35. Procédé suivant la revendication 34, comprenant en outre l'étape consistant à recueillir la glycoprotéine à partir de la culture de cellules hôtes.

36. Procédé suivant la revendication 35, comprenant en outre la conjugaison de la glycoprotéine à une molécule hétérologue.

37. Procédé suivant la revendication 36, dans lequel la molécule hétérologue est un agent cytotoxique ou une enzyme.
